# EUROPEAN PATENT APPLICATION

(11) **EP 4 635 436 A1**
(43) Date of publication of application: **22.10.2025**
(21) Application number: 23845364.1
(22) Date of filing: 17.07.2023
(51) Int. Cl.: A61B 18/12

(54) **ABLATION SYSTEM**

(30) Priority: 28.07.2022 CN 202210900583
(71) Applicant: Hangzhou Dinova EP Technology Co., Ltd., Hangzhou, Zhejiang 311103 (CN)
(72) Inventor: LI, Jianmin, Hangzhou, Zhejiang 311103 (CN); WANG, Kun, Hangzhou, Zhejiang 311103 (CN); YOU, Yan, Hangzhou, Zhejiang 311103 (CN)
(74) Representative: Metida
(86) International application number: PCT/CN2023/107785
(87) International publication number: WO 2024/022152

(57) **Abstract**

The application provides an ablation system, which comprises a support body and an ablation part, at least part of the ablation part is arranged on the support body, the ablation part comprises a first conductive part and a second conductive part, the first conductive part and the second conductive part are used to form a loop to transmit pulse ablation energy, their polarities are opposite, and the average current densities of the first conductive part and the second conductive part are not equal.

## Description

This application claims the priority of the Chinese patent application with the application number 202210900583.8 and the title "An Ablation System" filed with the China Patent Office on July 28, 2022, the entire content of which is incorporated into this application by reference.

### TECHNICAL FIELD

This application belongs to the technical field of medical devices, and more specifically relates to an ablation system.

### BACKGROUND

Atrial fibrillation (AF) is the most common persistent arrhythmia. With the increase of age, the incidence of atrial fibrillation continues to increase, reaching 10% in people over 75 years old. During atrial fibrillation, the frequency of atrial excitation is 300-600 times per minute, and the heart rate is often fast and irregular, sometimes reaching 100-160 times per minute. It is not only much faster than the normal heart rate, but also completely irregular, and the atria lose their effective contractile function. Atrial fibrillation usually increases the risk of many potentially fatal complications, including thromboembolic stroke, dilated cardiomyopathy and congestive heart failure. Common symptoms of atrial fibrillation such as palpitations, chest pain, dyspnea, fatigue and dizziness can also affect the quality of life. Compared with normal people, people with atrial fibrillation have a five-fold increase in average incidence and a twofold increase in mortality.

Tissue ablation is usually used to treat various arrhythmias, including atrial fibrillation. In order to treat arrhythmia, ablation catheters can be used to ablate the tissue at the lesion to denature and necrosis, thereby blocking the conduction of abnormal electrical signals and achieving the goal of curing atrial fibrillation. Most of the existing ablation catheters are based on thermal ablation, such as radiofrequency ablation, laser ablation, microwave ablation, thermal substance ablation, etc.

However, the ablation device based on the principle of thermal ablation ablates the tissue by heating. During the ablation process, the ablation electrode in the ablation device has high requirements on the wall apposition. In the case of poor wall apposition of the ablation electrode, the ablation success rate is low.

### SUMMARY

The purpose of the embodiments of this application is to provide an ablation system, including a support body and an ablation element. At least part of the ablation element is arranged on the support body. The ablation element includes a first conductive part and a second conductive part. The first conductive part and the second conductive part are used to form a loop to transmit pulse ablation energy, and their polarities are opposite. The average current densities of the first conductive part and the second conductive part are not equal.

In the existing ablation system, the average current densities on the surfaces of the two conductive parts that form a loop for transmitting pulse energy are close. When it is necessary to increase the average current density of one of the conductive parts to increase the ablation range and depth of the conductive part, the method of increasing the overall ablation energy of the two conductive parts (such as increasing the pulse voltage) is generally used to increase the average current density of one of the conductive parts.

However, increasing the overall ablation energy of the two conductive parts will inevitably cause the total current of the two conductive parts to increase, and the average current density on the surface will increase synchronously, which stimulates the patient's body greatly.

The beneficial effect of the ablation system provided by this application is that compared with the prior art, the ablation system of this application has unequal average current densities of the first conductive part and the second conductive part. Through the cooperation of the first conductive part and the second conductive part, pulse ablation energy with different polarities can be transmitted to the target tissue to realize pulse ablation of the target tissue.

The ablation system of this application adopts the pulse ablation method, which uses the pulse electric field to cause irreversible electrical breakdown (irreversible electroporation) of the cell membrane, leading to cell apoptosis, thereby realizing non-thermal effect ablation of cells. Therefore, it is not affected by the heat sink effect. The treatment time of pulse ablation is short. The treatment time for applying a set of pulse sequences is less than 1 minute, and the total ablation time is generally no more than 5 minutes. In addition, due to the difference in the response thresholds of different tissues to the pulse electric field, it is possible to ablate the myocardium without interfering with other adjacent tissues, thereby avoiding accidental injury to the tissues adjacent to the pulmonary veins. In addition, compared with other energies, pulse ablation does not require heat conduction to ablate deep tissues. All target tissue cells distributed above a certain electric field intensity will undergo electroporation, which reduces the requirement for the apposition pressure of the ablation element against the target tissue wall during ablation. Therefore, even if the ablation device does not completely fit the target tissue wall during the ablation process, the ablation effect is not affected.

In the ablation system provided by this application, the average current densities of the first conductive part and the second conductive part are not equal, that is, the average current densities on the surfaces of at least two different conductive parts forming the loop are different. For the first conductive part and the second conductive part, the one with the expected larger ablation depth and range has a larger average current density. Furthermore, according to the differences of different target tissues of the ablation system, the specific positions of different conductive parts, and the expected ablation depth and range, the average current density of one of the two conductive parts forming the loop can be set to be relatively larger, and the average current density of the other conductive part can be set to be relatively smaller. This makes the conductive part with a larger average current density obtain a relatively larger ablation range and deeper ablation depth, thereby improving the ablation effect and realizing complete electrical isolation. It avoids the situation where a pair of conductive parts forming a loop for transmitting pulse ablation energy have the same average current density on the surface, similar ablation range and depth. In the case of different wall apposition conditions of different conductive parts and different thicknesses of different positions in the target tissue, some target tissues can achieve transmural ablation, while others have great difficulty in transmural ablation. It also avoids after adopting the ablation strategy of increasing the overall ablation energy to ensure that all target tissues can achieve transmural ablation, the patient's body is greatly stimulated in this case.

### BRIEF DESCRIPTION OF DRAWINGS

In order to explain the technical solutions in the embodiments of this application more clearly, the following will briefly introduce the drawings needed in the description of the embodiments or the prior art. Obviously, the drawings in the following description are only some embodiments of this application. For those of ordinary skill in the art, other drawings can be obtained according to these drawings without creative work.
Figure 1 is a structural schematic diagram of the left atrial appendage occlusion and ablation device provided by the first embodiment of this application;
Figure 2 is a structural schematic diagram of the left atrial appendage occlusion and ablation device provided by the second embodiment of this application;
Figure 3 is a structural schematic diagram of the left atrial appendage occlusion and ablation device provided by the third embodiment of this application;
Figure 4 is a structural schematic diagram of the left atrial appendage occlusion and ablation device provided by the fourth embodiment of this application;
Figure 5 is a structural schematic diagram of the left atrial appendage occlusion and ablation device provided by the fifth embodiment of this application;
Figure 6A is a structural schematic diagram of the left atrial appendage occlusion and ablation device provided by the sixth embodiment of this application;
Figure 6B is a structural schematic diagram of the left atrial appendage occlusion and ablation device provided by the sixth embodiment of this application with a coating;
Figure 7 is a structural schematic diagram of the left atrial appendage occlusion and ablation device provided by the seventh embodiment of this application;
Figure 8 is a structural schematic diagram of the left atrial appendage occlusion and ablation device provided by the eighth embodiment of this application;
Figure 9 is a structural schematic diagram of the left atrial appendage occlusion and ablation device provided by the ninth embodiment of this application;
Figure 10 is a structural schematic diagram of the left atrial appendage occlusion and ablation device provided by the tenth embodiment of this application;
Figure 11 is a structural schematic diagram of the left atrial appendage occlusion and ablation device provided by the eleventh embodiment of this application;
Figure 12 is a structural schematic diagram of the left atrial appendage occlusion and ablation device provided by the twelfth embodiment of this application;
Figure 13 is a structural schematic diagram of the left atrial appendage occlusion and ablation device provided by the thirteenth embodiment of this application;
Figure 14 is a structural schematic diagram of the left atrial appendage occlusion and ablation device provided by the fourteenth embodiment of this application;
Figure 15A is a structural schematic diagram of the left atrial appendage occlusion and ablation device provided by the fifteenth embodiment of this application;
Figure 15B is a structural schematic diagram of the left atrial appendage occlusion and ablation device in Figure 15A without showing the coating;
Figure 15C is a structural schematic diagram of the left atrial appendage occlusion and ablation device provided by another modified embodiment based on the fifteenth embodiment of this application;
Figure 15D is a structural schematic diagram of the left atrial appendage occlusion and ablation device in Figure 15C without showing the coating;
Figure 16 is a schematic diagram of the ablation area interval distribution of the ablation element provided by the fifteenth embodiment;
Figure 17 is a schematic diagram of the partial overlapping distribution of the ablation areas of the ablation element provided by the fifteenth embodiment;
Figure 18 is a structural schematic diagram of the left atrial appendage occlusion and ablation device provided by the sixteenth embodiment of this application;
Figure 19 is a structural schematic diagram of the left atrial appendage occlusion and ablation device provided by the seventeenth embodiment of this application;
Figure 20 is a structural schematic diagram of the left atrial appendage occlusion and ablation device provided by the eighteenth embodiment of this application;
Figure 21 is a structural schematic diagram of the left atrial appendage occlusion and ablation device provided by the nineteenth embodiment of this application;
Figure 22 is a structural schematic diagram of the left atrial appendage occlusion and ablation device provided by the twentieth embodiment of this application;
Figure 23A is a structural schematic diagram of the left atrial appendage occlusion and ablation device provided by the twenty-first embodiment of this application;
Figure 23B is a structural schematic diagram of the left atrial appendage occlusion and ablation device in Figure 23A without showing the coating;
Figure 24 is a structural schematic diagram of the left atrial appendage occlusion and ablation device provided by the twenty-second embodiment of this application;
Figure 25 is a structural schematic diagram of the left atrial appendage occlusion and ablation device provided by the twenty-third embodiment of this application;
Figure 26 is a structural schematic diagram of the left atrial appendage occlusion and ablation device provided by the twenty-fourth embodiment of this application;
Figure 27 is a structural schematic diagram of the left atrial appendage occlusion and ablation device provided by the twenty-fifth embodiment of this application;
Figure 28 is a structural schematic diagram of the left atrial appendage occlusion and ablation device provided by the twenty-sixth embodiment of this application;
Figure 29A is a structural schematic diagram of the left atrial appendage occlusion and ablation device provided by the twenty-seventh embodiment of this application;
Figure 29B is a structural schematic diagram of the left atrial appendage occlusion and ablation device provided by a modified embodiment based on the twenty-seventh embodiment of this application;
Figure 29C is a structural schematic diagram of the left atrial appendage occlusion and ablation device in Figure 29B without showing the coating;
Figure 30 is a structural schematic diagram of the left atrial appendage occlusion and ablation device provided by the twenty-eighth embodiment of this application;
Figure 31 is a structural schematic diagram of the left atrial appendage occlusion and ablation device provided by the twenty-ninth embodiment of this application;
Figure 32 is a structural schematic diagram of the left atrial appendage occlusion and ablation device provided by the thirtieth embodiment of this application;
Figure 33 is a structural schematic diagram of the left atrial appendage occlusion and ablation device provided by the thirty-first embodiment of this application;
Figure 34 is a structural schematic diagram of the left atrial appendage occlusion and ablation device provided by the thirty-second embodiment of this application;
Figure 35 is a structural schematic diagram of the ablation system provided by the thirty-third embodiment of this application;
Figure 36 is a schematic diagram of the principle structure of the ablation system shown in Figure 35;
Figure 37 is a structural schematic diagram of the ablation system provided by the thirty-fourth embodiment of this application;
Figure 38 is a structural schematic diagram of the ablation system provided by the thirty-fifth embodiment of this application;
Figure 39 is a structural schematic diagram of the ablation system provided by the thirty-sixth embodiment of this application;
Figure 40 is a structural schematic diagram of the ablation system provided by the thirty-seventh embodiment of this application;
Figure 41 is a structural schematic diagram of the ablation system provided by the thirty-eighth embodiment of this application;
Figure 42 is a structural schematic diagram of the ablation system provided by the thirty-ninth embodiment of this application;
Figure 43 is a structural schematic diagram of the ablation system provided by the fortieth embodiment of this application;
Figure 44 is a structural schematic diagram of the ablation system provided by the forty-first embodiment of this application;
Figure 45 is a structural schematic diagram of the ablation system provided by the forty-second embodiment of this application;
Figure 46 is a schematic diagram of the principle structure of the ablation system shown in Figure 45;
Figure 47 is a structural schematic diagram of the ablation system provided by the forty-third embodiment of this application;
Figure 48 is a structural schematic diagram of the ablation system provided by the forty-fourth embodiment of this application;
Figure 49A is a structural schematic diagram of the ablation system provided by the forty-fifth embodiment of this application;
Figure 49B is a structural schematic diagram of the anchoring part in Figure 49A without showing the coating;
Figure 49C is a structural schematic diagram of the ablation system provided by a modified embodiment based on the forty-fifth embodiment;
Figure 49D is a structural schematic diagram of the ablation system provided by a modified embodiment based on Figure 49C;
Figure 49E is a structural schematic diagram of the anchoring part in Figure 49D without showing the coating;
Figure 50 is a structural schematic diagram of the ablation system provided by the forty-sixth embodiment of this application;
Figure 51 is a structural schematic diagram of the ablation system provided by the forty-seventh embodiment of this application;
Figure 52 is a structural schematic diagram of the ablation system provided by the forty-eighth embodiment of this application;
Figure 53 is a structural schematic diagram of the ablation system provided by the forty-ninth embodiment of this application;
Figure 54 is a structural schematic diagram of the ablation system provided by the fiftieth embodiment of this application;
Figure 55 is a structural schematic diagram of the ablation system provided by the fifty-first embodiment of this application;
Figure 56 is a structural schematic diagram of the ablation system provided by the fifty-second embodiment of this application;
Figure 57 is a structural schematic diagram of the ablation system provided by the fifty-third embodiment of this application;
Figure 58 is a structural schematic diagram of the ablation system provided by the fifty-fourth embodiment of this application;
Figure 59 is a structural schematic diagram of the ablation system provided by the fifty-fifth embodiment of this application.

### DESCRIPTION OF EMBODIMENTS

In order to make the technical problems, technical solutions and beneficial effects to be solved by this application clearer, the following will further describe this application in detail with reference to the accompanying drawings and embodiments. It should be understood that the specific embodiments described here are only used to explain this application, and are not used to limit this application.

It should be noted that when an element is referred to as being "fixed on" or "arranged on" another element, it can be directly on the other element or indirectly on the other element. When an element is said to be "connected to" another element, it can be directly connected to the other element or indirectly connected to the other element.

It should be understood that the terms "center", "longitudinal", "transverse", "length", "width", "thickness", "upper", "lower", "front", "rear", "left", "right", "vertical", "horizontal", "top", "bottom", "inner", "outer", etc. indicate the orientation or positional relationship based on the orientation or positional relationship shown in the drawings, which is only for the convenience of describing this application and simplifying the description, rather than indicating or implying that the device or element referred to must have a specific orientation, be constructed and operated in a specific orientation, and therefore cannot be understood as a limitation to this application. The term "and/or" as used herein includes any and all combinations of one or more related listed items.

In addition, the terms "first" and "second" are only used for descriptive purposes, and cannot be understood as indicating or implying relative importance or implicitly indicating the number of indicated technical features. Therefore, the features defined with "first" and "second" may explicitly or implicitly include one or more of these features. In the description of this application, "multiple" means two or more, unless otherwise clearly and specifically defined.

### Definition Explanation:

Left atrial appendage orifice: The position entering the left atrial appendage from the left atrium.

Proximal end and distal end: In the field of medical device technology, the end of the medical device implanted in the human or animal body that is closer to the operator is generally called the proximal end, and the end farther from the operator is called the distal end. Based on this principle, the proximal and distal ends of any part of the medical device are defined. Without violating the technical principle of this application, the specific technical solutions in the following embodiments can be applied to each other.

Proximal side and distal side: Along the channel for delivering medical devices in the organism, the side of the medical device or its components implanted in the human or animal body that faces the operator is called the proximal side, and the side that is far away from the operator is called the distal side. Without violating the technical principle of this application, the specific technical solutions in the following embodiments can be applied to each other.

Axial direction and radial direction: "Axial direction" generally refers to the length direction of the medical device when it is delivered, and "radial direction" generally refers to the direction perpendicular to the "axial direction" of the medical device. Based on this principle, the "axial direction" and "radial direction" of any part of the medical device are defined.

Insulation treatment: Forming an insulating layer on the surface of a component to insulate that part of the component. Specifically, the insulation treatment methods include: setting an insulating coating material at the position that needs insulation treatment, the coating material includes but is not limited to parylene coating, PTFE (Poly-tetra-fluoroethylene) coating, PI (Polyimide) coating; or covering a membrane at the position that needs insulation treatment, the membrane material includes but is not limited to FEP (Fluorinated-ethylenepropylene), PU (polyurethane), ETFE (ethylene-tetra-fluoro-ethylene), PFA (Polyfluoroalkoxy), PTFE, PEEK (poly-ether-ether-ketone), silica gel. Or, put an insulating sleeve on the position that needs insulation treatment. The material of the insulating sleeve includes but is not limited to FEP, PU, ETFE, PFA, PTFE, PEEK, silica gel. In some embodiments, at least one of the above insulation treatment schemes can be used for the part that needs insulation treatment, or a combination of two or more of the same or different insulation treatment methods can be used.

### Embodiment 1

The ablation system provided by the embodiment of this application adopts the percutaneous puncture method, and delivers the ablation device to the target tissue through a delivery device, including but not limited to delivering to a specific position of the heart to ablate the pulmonary vein, left atrial appendage, or trigger foci (such as the superior vena cava, coronary sinus ostium, etc.) with typical atrial flutter and non-pulmonary vein origin, so as to achieve the effect of electrical isolation. It can be understood that the ablation tissue area is not limited to the heart, but can also be located in other body tissues, which is not limited here.

The ablation system provided by the embodiment of this application can be a left atrial appendage occlusion and ablation system, other instruments for occluding and ablating tissues, or an ablation catheter that does not need to be implanted in the body, which is only used to ablate target tissues, that is, it can be an ablation system only used for tissue ablation. The ablation system provided by the embodiment of this application includes a support body, a delivery device and an ablation element. The distal end of the delivery device is connected to the support body for delivering the support body to the target tissue.

At least part of the ablation element is arranged on the support body. The ablation element includes a first conductive part and a second conductive part. In some embodiments, both the first conductive part and the second conductive part are arranged on the support body. In some embodiments, one of the first conductive part and the second conductive part is arranged on the support body. Correspondingly, the conductive part not arranged on the support body can be arranged on the delivery device, or independently arranged relative to the support body and the delivery device, that is, the conductive part not arranged on the support body is not arranged on the delivery device, such as an external conductive part arranged on the patient's body surface, such as a metal plate arranged outside the body.

Both the first conductive part and the second conductive part are electrically connected to an external pulse ablation source. The first conductive part and the second conductive part are used to transmit pulse ablation energy with different polarities to the target tissue to realize tissue ablation.

The support body includes a radially contractible and expandable framework or a balloon. The framework can be made by weaving or cutting processes and forms a plurality of mesh holes. The balloon is used to fill with medium to adjust the radial size. The following description takes the framework structure as an example. The support body can be provided with a membrane on the surface of the framework to block thrombus, insulate, maintain the mechanical stability of the support body and other functions.

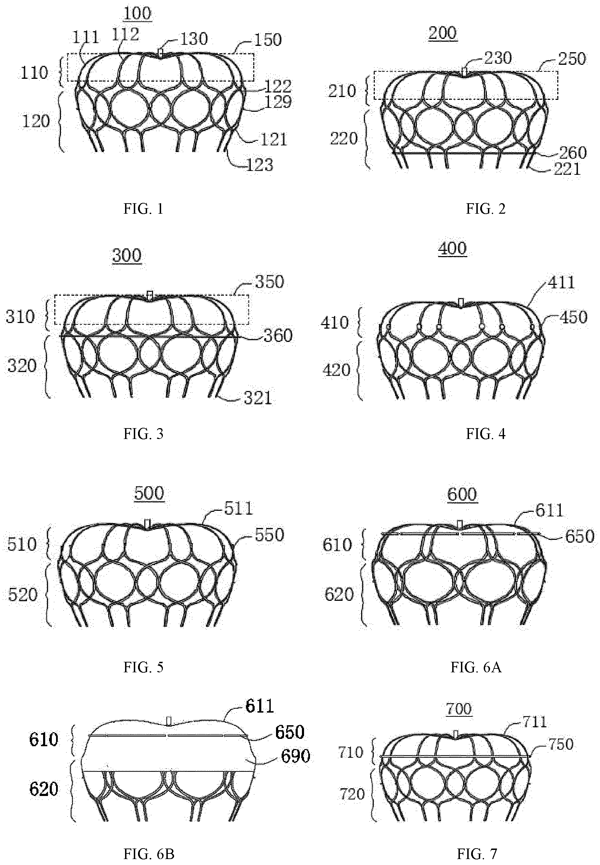

Please refer to Figure 1. The ablation system provided in this embodiment includes a left atrial appendage occlusion and ablation device 100 and a delivery device. The left atrial appendage occlusion and ablation device 100 is used for occluding the left atrial appendage and performing electrical ablation. The left atrial appendage occlusion and ablation device 100 includes a support body and a first conductive part arranged on the support body. In this embodiment, the support body can be made by weaving or cutting processes and forms a framework with a plurality of mesh holes. The second conductive part is arranged on the support body of the left atrial appendage occlusion and ablation device 100, or on the delivery device, or the second conductive part is independently arranged from the support body and the delivery device, that is, the second conductive part is arranged outside the support body and the delivery device, for example, the second conductive part is an external conductive part attached to the patient's body surface during ablation. In this embodiment, the first conductive part is electrically connected to an external pulse ablation instrument through a delivery device. The target tissue is the left atrial appendage in the myocardial tissue.

The support body includes a sealing part 110 and an anchoring part 120 connected to each other. The sealing part 110 is used to block the left atrial appendage orifice, and the anchoring part 120 is used to fix on the tissue wall of the left atrial appendage. Both the sealing part 110 and the anchoring part 120 are radially expandable structures. Any part of the sealing part 110 and the anchoring part 120 can be manufactured by weaving or cutting processes. In this embodiment, the sealing part 110 is arranged on the proximal side of the anchoring part 120. In some embodiments, the anchoring part is arranged on the circumferential edge of the sealing part.

Specifically, the sealing part 110 includes a first framework 111, the anchoring part includes a second framework 121, and the first framework 111 and the second framework 121 are connected to each other; the sealing part 110 is provided with an ablation element for transmitting ablation energy, specifically the first conductive part 150 in the ablation element.

The left atrial appendage occlusion and ablation device 100 in this embodiment has a single-disk structure, that is, the sealing part 110 and the anchoring part 120 in the left atrial appendage occlusion and ablation device 100 are located in the same disk surface. The framework in the circumferential edge area of the sealing part 110 and the anchoring part 120 is used to fit the left atrial appendage tissue.

The sealing part 110 is arranged on the proximal side of the anchoring part 120. The proximal end surface of the sealing part 110 is used to be released at the orifice of the left atrial appendage. The circumferential edge area of the first framework 111 is used to fit the orifice of the left atrial appendage and the inner cavity tissue wall of the left atrial appendage, especially the part with the largest radial size in the first framework 111 is used to fit the inner cavity tissue wall of the left atrial appendage. Optionally, at least one layer of membrane for blocking thrombus outflow is arranged on the inner or outer side of the left atrial appendage occlusion and ablation device 100, that is, the support body includes at least one layer of membrane arranged on the outer surface/or inner surface of the framework to block the thrombus in the left atrial appendage from flowing out to the left atrium. The anchoring part 120 is used to be released in the inner cavity of the left atrial appendage. The circumferential side wall of the second framework 121 in the anchoring part 120 is used to generate radial supporting force on the inner wall of the left atrial appendage and fix in the inner cavity of the left atrial appendage. Optionally, as shown in Figure 1, the second framework includes a number of anchor thorns 129 arranged on its surface. The anchor thorns 129 extend radially outward from the framework, so that after the anchoring part 120 is released, the anchor thorns 129 can be combined in the inner cavity tissue of the left atrial appendage, improving the anchoring ability of the anchoring part 120.

As shown in Figure 1, the anchoring part 120 is a distal opening structure, or the left atrial appendage occlusion and ablation device 100 is a cup-shaped structure.

The sealing part 110 is provided with the first conductive part 150 for transmitting ablation energy. In some embodiments, at least part of the first conductive part 150 can be used to collect tissue physiological signals.

In this embodiment, at least part of the first framework 111 serves as the first conductive part 150. More specifically, all of the first framework 111 serves as the first conductive part 150. The first conductive part 150 is used to transmit ablation energy. In some embodiments, the first conductive part 150 can also be used to collect tissue physiological signals, such as cardiac electrophysiological signals, so as to realize signal mapping before and after ablation.

Compared with the tissue in the inner cavity of the left atrial appendage, the tissue at the orifice of the left atrial appendage has a smooth surface and regular shape, which is convenient for the sealing part 110 to appose. All the first frameworks in the sealing part 110 serve as the first conductive part to ablate the tissue at the orifice of the left atrial appendage, which is beneficial to improve the wall apposition performance of the first conductive part 150 and facilitate the ablation of the tissue at the orifice of the left atrial appendage.

In some embodiments, part of the first framework 111 serves as the first conductive part 150. Specifically, the first framework 111 includes an edge area and a central area. The central area is closer to the central axis of the sealing part 110 than the edge area. The central axis passes through the central area. The part with the largest radial size in the sealing part 110 is located in the circumferential edge area. The first conductive part 150 is arranged in the circumferential edge area of the sealing part 110, so that the first conductive part 150 can appose or be close to the left atrial appendage tissue. Further, in some embodiments, the first conductive part 150 is arranged in the area with the largest radial size on the first framework 111.

Arranging the first conductive part 150 in the edge area of the first framework 111, or further arranging the first conductive part 150 in the area with the largest radial size on the first framework 111, and the first conductive part 150 is not arranged in the central area of the first framework 111, thereby reducing the area where ablation energy is released, concentrating the ablation energy at the position of the first conductive part 150 with a smaller discharge area, which is beneficial to increase the ablation depth.

Specifically, the sealing part 110 is made of a conductive material, and can be made of a superelastic metal material with good biocompatibility, such as stainless steel,nickel-titanium alloy, cobalt-chromium alloy and other materials. Since the left atrial appendage occlusion and ablation device 100 in this embodiment is an integrated structure. The left atrial appendage occlusion and ablation device 100 in Figure 1 is made of nickel-titanium alloy tube by laser cutting. It can be understood that the left atrial appendage occlusion and ablation device 100 can also be made of other metal materials with good biocompatibility, or made by other processes besides cutting, such as weaving.

In this embodiment, the surface of the second framework 121 in the anchoring part 120 is insulated. For the specific method of insulation treatment, please refer to the previous description. In this embodiment, the sealing part 110 is provided with the first conductive part 150 for ablating tissue, that is, the left atrial appendage occlusion and ablation device 100 can use the first conductive part 150 to ablate tissue.

As shown in Figure 1, the left atrial appendage occlusion and ablation device 100 includes a first conductive connector 130 arranged on the first framework 111. The first conductive connector 130 is used to be electrically connected between the first conductive part 150 and an external ablation energy source. The external ablation signal source can be a pulse ablation instrument, which is used to output pulse ablation electric energy for the left atrial appendage occlusion and ablation device 100 to perform ablation operations.

The pulse ablation method uses the pulse electric field to cause irreversible electrical breakdown (irreversible electroporation) of the cell membrane, leading to cell apoptosis, thus realizing non - thermal effect ablation of cells, so it is not affected by the heat sink effect. The treatment time of pulse ablation is short. The treatment time for applying a set of pulse sequences is less than 1 minute, and the total ablation time is generally no more than 5 minutes. In addition, due to the difference in the response thresholds of different tissues to the pulse electric field, it is possible to ablate the myocardium without interfering with other adjacent tissues, thus avoiding accidental injury to the tissues adjacent to the pulmonary veins. In addition, compared with other energies, pulse ablation does not require heat conduction to ablate deep tissues. All target tissue cells distributed above a certain electric field intensity will undergo electroporation, which reduces the requirements for the pressure of the ablation part against the target tissue wall during ablation. Therefore, even if the ablation device does not completely fit the inner wall of the target tissue during the ablation process, the ablation effect is not affected.

The first conductive connector 130 is arranged at the proximal end of the sealing part 110, and is preferably made of a conductive material to transmit electric energy, such as stainless steel, nickel-titanium alloy, cobalt-chromium alloy and other materials. Preferably, the proximal end of the first framework 111 is converged on the first conductive connector 130. The first conductive connector 130 is also used to connect the delivery device. The distal end of the delivery device is mechanically connected to the first conductive connector 130 and realizes electrical connection with the first conductive connector 130. The proximal end of the delivery device is used to maintain electrical connection with an external ablation energy source.

In some embodiments, a second conductive part for cooperating with the first conductive part 150 can be arranged outside the left atrial appendage occlusion and ablation device 100. The second conductive part is used to transmit ablation energy. In some embodiments, at least part of the second conductive part can be used to collect tissue physiological signals. The first conductive part and the second conductive part are electrically isolated. The first conductive part and the second conductive part can be used to transmit pulse ablation energy with different polarities to the left atrial appendage tissue to realize tissue ablation. The two conductive parts have different polarities. During ablation, the conductive part with one polarity is the first conductive part, and the conductive part with the other polarity is the second conductive part. The first conductive part is arranged on the support body.

The second conductive part can be arranged on the delivery device, such as on the catheter in the delivery device for connecting the first conductive connector 130, or on a movable part (such as an ablation catheter) in the delivery device. The movable part can move relative to the left atrial appendage occlusion and ablation device 100. The movable part is used to be released from the distal end of the delivery device. An ablation electric field for ablating the left atrial appendage tissue is formed between the first conductive part 150 and the second conductive part. The second conductive part can be released on the proximal side, inner cavity or distal side of the left atrial appendage occlusion and ablation device 100. In some embodiments, the second conductive part is independently arranged from the left atrial appendage occlusion and ablation device 100 and the delivery device. The first conductive part 150 in the left atrial appendage occlusion and ablation device 100 is used in cooperation with the second conductive part arranged outside the left atrial appendage occlusion and ablation device 100 and the delivery device, for example, the second conductive part is a second conductive part attached to the patient's body surface during ablation. The second conductive part can be connected to an external pulse ablation instrument through a delivery device or other cables.

In some embodiments, on the basis that the first framework 111 is arranged in the sealing part 110 as the first conductive part 150, the left atrial appendage occlusion and ablation device 100 is provided with a second conductive part in the anchoring part 120. The second conductive part can be part or all of the second framework of the anchoring part 120. The first conductive part 150 and the second conductive part are insulated.

More specifically, in the single-disk left atrial appendage occlusion and ablation device 100 shown in Figure 1, at least part of the first framework 111 serves as the first conductive part 150, and at least part of the second framework 121 serves as the second conductive part. The first framework 111 and the second framework 121 are insulated, for example, the connection between the first framework 111 and the second framework 121 is connected by an insulating material, such as insulating glue, an insulating connector, or the distal end of the first framework 111 and/or the proximal end of the second framework 121 are made of an insulating material.

In the embodiment where the second conductive part is arranged in the anchoring part 120, the left atrial appendage occlusion and ablation device 100 is also provided with a second conductive connector, which is used to be electrically connected to the second conductive part. The first conductive connector 130 and the second conductive connector are electrically isolated to transmit ablation electric energy with different polarities. Specifically, the first conductive connector 130 and the second conductive connector are insulated, for example, an insulating material is arranged between them. The second conductive connector and the second conductive part can be electrically connected by a second wire.

In the embodiment where the second conductive part is arranged in the anchoring part 120 but the left atrial appendage occlusion and ablation device 100 is not provided with a second conductive connector, the second conductive part is electrically connected to the delivery device through a second wire. After the ablation is completed, the second wire is disconnected from the delivery device, or the second wire is disconnected from the second conductive part, or the second wire is cut off. It can be understood that the connection mode between the second conductive part and the delivery device can be applied to the connection mode between the first conductive part 150 and the delivery device, which will not be repeated here.

Compared with the tissue in the inner cavity of the left atrial appendage, the tissue at the orifice of the left atrial appendage has a smooth surface and regular shape, which is convenient for the sealing part 110 to appose. Part or all of the first framework 111 in the sealing part 110 serves as the first conductive part 150 to ablate the tissue at the orifice of the left atrial appendage, which is beneficial to improve the wall apposition performance of the first conductive part 150 and facilitate the ablation of the tissue at the orifice of the left atrial appendage.

Further, please refer to Figure 1 again. The first framework 111 includes a plurality of sealing rods 112. Each sealing rod 112 extends between the proximal end and the distal end. The proximal ends of the plurality of sealing rods 112 are combined with the first conductive connector 130. It can be understood that the proximal ends of the plurality of sealing rods 112 are not limited to being combined with the first conductive connector 130, but can also be combined with other components. The plurality of sealing rods 112 extend in different directions around the first conductive connector 130 as the center. The distal ends of the plurality of sealing rods 112 are spaced from each other, so that the first framework 111 forms a hollow structure with mesh holes.

The second framework 121 extends from the ends of the plurality of spaced sealing rods 112 of the first framework 111. The second framework 121 includes a first anchoring rod 122 and a second anchoring rod 123. The first anchoring rod 122 is connected between the sealing rod 112 and the second anchoring rod 123. The distal end of each sealing rod 112 is connected to two different first anchoring rods 122. The two first anchoring rods 122 connected to the same sealing rod 112 extend in different directions. Among the 4 first anchoring rods 122 connected to two adjacent sealing rods 112, the middle sections of two adjacent anchoring rods 122 connected to different sealing rods 112 are close to each other and connected, and an anchor thorn 129 is arranged at the mutually connected position. The distal ends of the two first anchoring rods 122 connected to the same sealing rod 112 are connected to each other and connected to the proximal end of a second anchoring rod 123. The distal end of the second anchoring rod 123 extends to the distal end. The adjacent second anchoring rods 123 are arranged at intervals, that is, the distal ends of the second anchoring rods 123 are not connected or close to each other, thus forming an open structure at the distal end of the anchoring part 120, that is, the distal end of the second framework 121 and the distal end of the left atrial appendage occlusion and ablation device 100 are open structures.

The plurality of first anchoring rods 122 form a plurality of mesh holes in the anchoring part 120, which is convenient for improving the radial deformation ability of the anchoring part 120 and the friction force with the tissue.

The anchor thorn 129 extends from the proximal end to the distal end. The fixed end of the anchor thorn 129 is connected to the first anchoring rod 122. The free end of the anchor thorn 129 tilts protrudes radially outward in all directions relative to the first anchoring rod 122, which is convenient for combining with the internal tissue of the left atrial appendage.

### Embodiment 2

Please refer to Figure 2. The ablation system of this embodiment includes a left atrial appendage occlusion and ablation device 200 for occluding and ablating the left atrial appendage. The left atrial appendage occlusion and ablation device 200 includes a sealing part 210 arranged at the proximal end and an anchoring part 220 arranged at the distal end. The sealing part 210 and the anchoring part 220 are connected to each other.

The main difference between the left atrial appendage occlusion and ablation device 200 and the left atrial appendage occlusion and ablation device 100 is that the second conductive part 260 is arranged on the framework of the left atrial appendage occlusion and ablation device 200, and the second conductive part 260 is used to transmit ablation energy; The second conductive part 260 is arranged on the anchoring part 220; The first conductive part 250 and the second conductive part 260 form electrical isolation, and the first conductive part 250 and the second conductive part 260 are used to transmit pulse ablation energy with different polarities to the left atrial appendage. At least part of the first conductive part 250 and/or the second conductive part 260 can be used to collect tissue physiological signals.

Specifically, the second conductive part 260 is at least one electrode piece additionally arranged on the surface of the anchoring part 220. The second conductive part 260 is made of a conductive material with good biocompatibility, such as platinum, iridium, gold, silver and other medical metals that can be used for interventional therapy.

In this embodiment, the number of electrode pieces in the second conductive part 260 is one, and the specific electrode piece is a ring electrode.

The ring electrode is a strip or filament electrode, such as an electrode wire or an electrode sheet, extending along the circumferential direction of the anchoring part 220 and surrounding the anchoring part 220 for at least one circle. The ring electrode is annular, such as circular, elliptical, or irregular annular, so as to form an annular ablation zone.

As shown in Figure 2, the ring electrode is arranged in a plane perpendicular to the central axis of the left atrial appendage occlusion and ablation device 200, that is, each part of the ring electrode is located at the same axial position on the left atrial appendage occlusion and ablation device 200. In other embodiments, the ring electrode is arranged in a plane inclined relative to the central axis of the left atrial appendage occlusion and ablation device 200.

As shown in Figure 2, the first conductive part 250 is arranged in the circumferential edge area of the proximal part of the support body, and the second conductive part 260 is arranged in the circumferential edge area of the distal part of the support body. The second conductive part 260 and the first conductive part 250 work together to ablate the tissue.

In some embodiments, the entire surface of the second framework 221 needs to undergo electrical insulation treatment. In some embodiments, the part of the second framework 221 that is not in contact with the second conductive part 260 needs to undergo electrical insulation treatment, and the part that is in contact with the second conductive part 260 does not need to be insulated. In some embodiments, the part of the second framework 221 that is in contact with the second conductive part 260 needs to undergo electrical insulation treatment, and the part that is not in contact with the second conductive part 260 does not need to be insulated, or is selectively insulated.

When the two conductive parts are used to transmit pulse ablation energy with different polarities, an ablation electric field for ablating the target tissue is generated between the two conductive parts. The ablation area of each conductive part is the area around the conductive part where the electric field intensity is greater than the threshold intensity of the target tissue, and the target tissue in the ablation area can be ablated. Specifically, the area around the first conductive part 250 where the electric field intensity is greater than the threshold field intensity of the target tissue is the first ablation area, and the area around the second conductive part 260 where the electric field intensity is greater than the threshold field intensity of the target tissue is the second ablation area.

The left atrial appendage occlusion and ablation device 200 is used to ablate the left atrial appendage in myocardial tissue. When the electric field intensity used for pulse ablation of the left atrial appendage is lower than the threshold field intensity of myocardial tissue, the ablation electric field cannot ablate the left atrial appendage, and the left atrial appendage has no physiological effect. When the electric field intensity used for pulse ablation of the left atrial appendage is higher than the threshold ablation field intensity of myocardial tissue, the pulse breaks down the cell membrane to form nanoscale irreversible electroporation, which destroys the intracellular environment homeostasis, leading to necrosis or apoptosis of left atrial appendage cells.

For complete electrical isolation of the left atrial appendage, tissue ablation needs to be completely transmural, that is, both the inner and outer walls of the tissue undergo irreversible electroporation. At this electric field intensity, the pulse ablation energy acts on the cell membrane of the target tissue, and the cells form pores of irreversible electroporation, thus destroying the homeostasis of the intracellular and extracellular environment, achieving the effect of electrical isolation, and ensuring that the left atrial appendage and left atrium on both sides of the target tissue cannot transmit electrophysiological signals, so as to achieve the purpose of treating atrial fibrillation.

High transmurality requires that the electric field intensity covering the inner and outer walls of the left atrial appendage must be greater than the myocardial threshold field intensity. Generally, the wall thickness of the left atrial appendage is about 3mm. In some embodiments, during ablation, the electric field intensity generated by the ablation element at a position 3mm away from its surface is greater than the myocardial threshold field intensity. In some embodiments, the generated electric field intensity is set to be greater than 400V/cm, so as to ensure transmural ablation for the left atrial appendage tissue of different patients.

The closer to the ablation element, the denser the electric field lines, the greater the electric field intensity, and the easier the tissue is to be ablated. When the electric field intensity covering the outer wall of the left atrial appendage is above 400 V/cm, the electric field intensity formed on the surface of the ablation element is greater than that on the outer wall of the left atrial appendage, which is greater than the myocardial threshold of 400V/cm.

The electric field intensity acting on myocardial tissue cells is related to the pulse voltage range, the discharge area of the ablation element and the distance between the conductive parts.

In the embodiment where the first ablation part is arranged on the support body and the second ablation part is arranged on the support body or the delivery device, the ablation element (the first conductive part and the second conductive part) adopts a pulse voltage range of 500V~4000V, the discharge area of the first conductive part 250 is 15mm²~4700mm², and the discharge area of the second conductive part 260 is 15mm²~4700mm². When the conductive part has multiple electrode pieces, the value range of the discharge area refers to the sum of the discharge areas of all electrode pieces of the conductive part. The distance between the nearest two points on the surfaces of the first conductive part 250 and the second conductive part 260 ranges from 1mm to 45mm.

Both the first conductive part 250 and the second conductive part 260 are arranged on the framework of the left atrial appendage occlusion and ablation device 200. The two conductive parts transmit pulse ablation electric energy with different polarities. The first conductive part 250 and the second conductive part 260 are arranged at different positions on the framework of the left atrial appendage occlusion and ablation device 200 at intervals in the axial direction, with a voltage range of 500V~4000V. If the voltage is too low, the electric field intensity generated between the two conductive parts is too low, the ablation area is too small, and transmural ablation cannot be achieved, so the ablation effect cannot be achieved. If the voltage is too high, the insulation performance of the left atrial appendage occlusion and ablation device 200 and the delivery device is highly required, and it is difficult for existing insulation processes and materials to meet the withstand voltage requirements. Moreover, if the voltage is too high, especially when the insulation performance of the left atrial appendage occlusion and ablation device 200 and the delivery device cannot meet the requirements, it is easy to cause electrical coupling between the ablation element and other components, resulting in electric sparks, tissue eschar, and even heart perforation leading to pericardial effusion and other harmful results to the human body.

The two conductive parts can be in various forms such as dot, rod, line or sheet. Regardless of the form, the key factors are the distance between the two conductive parts and the discharge area of each conductive part.

With the voltage difference between the two conductive parts unchanged, the distance between the two conductive parts directly affects the electric field intensity formed between the two electrodes. The discharge area of the conductive part can change the current density around the conductive part, further affecting the electric field effect.

When the current is constant, if the discharge area of the ablation element is too large, the current density is too small, the electric field intensity formed on the surface of the conductive part is too low, the depth of the ablation area is shallow, the electric field intensity at the outer wall of the left atrial appendage is difficult to reach the myocardial threshold intensity, it is difficult to form transmural ablation, and the ablation success rate is low. If the discharge area is too small, the current density is too high, the conductive part is easy to be electrically coupled with other components, generating electric sparks, the components in the left atrial appendage occlusion and ablation device 200 are easy to be melted or burned due to overheating, the insulating material is damaged, and the wall apposition of the conductive part cannot be guaranteed.

When the pulse voltage is in the range of 500V~4000V, the discharge area of the ablation element is in the range of 15mm²~4700mm², and the distance between the two conductive parts is in the range of 1mm~45mm, it is beneficial to form an electric field intensity greater than the myocardial threshold field intensity at the outer wall of the left atrial appendage. For example, during ablation, the electric field intensity generated by the ablation element at a position 3mm away from its surface is greater than 400V/cm, which is beneficial for the ablation area to cover the outer wall of the left atrial appendage, thus forming transmural ablation in the left atrial appendage tissue.

The above parameters are applicable to various forms of conductive parts, such as at least part of the framework, or various forms of electrodes.

The first conductive part 250 and the second conductive part 260 are used to form a loop to transmit pulse ablation energy, and their polarities are opposite. Preferably, the average current densities of the first conductive part 250 and the second conductive part 260 are not equal. For example, the average current density of the first conductive part 250 is greater than that of the second conductive part 260, or the average current density of the first conductive part 250 is less than that of the second conductive part 260, so that the one of the first conductive part 250 and the second conductive part 260 with the expected larger ablation depth and range has a larger average current density.

In the ablation system, the average current densities of the first conductive part 250 and the second conductive part 260 are not equal, that is, the average current densities on the surfaces of at least two different conductive parts forming the loop are different. For the first conductive part and the second conductive part, the one with the expected larger ablation depth and range has a larger average current density. Furthermore, according to the differences of different target tissues of the ablation system, the specific positions of different conductive parts, and the expected ablation depth and range, the average current density of one of the two conductive parts forming the loop can be set to be relatively larger, and the average current density of the other conductive part can be set to be relatively smaller. This makes the conductive part with a larger average current density obtain a relatively larger ablation range and deeper ablation depth, thereby improving the ablation effect and realizing complete electrical isolation. It avoids the situation where a pair of conductive parts forming a loop for transmitting pulse ablation energy have the same average current density on the surface, similar ablation range and depth. In the case of different wall apposition conditions of different conductive parts and different depths of different positions in the target tissue, some target tissues can achieve transmural ablation, while others have great difficulty in transmural ablation. After adopting the ablation strategy of increasing the ablation energy to ensure that all target tissues can achieve transmural ablation, the patient's body is greatly stimulated.

In this embodiment, the first conductive part 250 and the second conductive part 260 are arranged at intervals in the axial direction, and the first conductive part 250 is arranged on the proximal side of the second conductive part 260. In other embodiments, the first conductive part 250 and the second conductive part 260 can be arranged at intervals in the circumferential direction. In the left atrial appendage occlusion and ablation system 200, in addition to the first conductive part 250 and the second conductive part 260, other conductive parts can also be arranged, that is, the specific number of conductive parts in the left atrial appendage occlusion and ablation system 200 is not limited.

During ablation, the average current density on the surface of the second conductive part is greater than that on the surface of the first conductive part.

Setting the current density of the second conductive part arranged on the distal side of the two conductive parts forming the loop to be larger, forming a larger ablation range and deeper ablation depth, is particularly suitable for ablation of heart tissue, such as ablation of intracardiac tissue, such as ablation at orifices within the heart (ablation of pulmonary veins, left atrial appendage and other tissues), or ablation of focal lesions within the heart.

In the left atrial appendage occlusion and ablation system 200, both the first conductive part 250 and the second conductive part 260 are used to ablate the target tissue, that is, form an ablation area in the target tissue. The wall apposition condition of the second conductive part 260 is worse than that of the first conductive part 250.

Different conductive parts in the ablation system are used to be close to different positions of the target tissue during ablation, but the wall apposition conditions of different conductive parts are different. Some conductive parts have good wall apposition conditions, such as being able to be close to the target tissue, so they are more likely to form a larger ablation range and deeper ablation depth; Some conductive parts have poor wall apposition conditions. For example, due to the position of the conductive part, the position of the target tissue, and the differences in the structure of different patients, the conductive part is suspended during ablation, far from the target tissue, unable to ablate the target tissue, or has a small ablation range and depth, resulting in poor ablation effect.

The expected ablation depth of the conductive part with relatively poor wall apposition conditions is larger, so as to reduce or avoid the impact of poor wall apposition conditions on its ablation depth. Setting the average current density of the conductive part with relatively poor wall apposition conditions to be relatively larger enables it to form a larger ablation range and deeper ablation depth, which is conducive to achieving complete electrical isolation. Setting the average current density of the conductive part with good wall apposition conditions to be relatively smaller can also ensure a certain ablation depth under good wall apposition conditions, which is conducive to ensuring the ablation depth and good ablation effect of the two conductive parts.

The left atrial appendage occlusion and ablation device 200 is used to be released and occluded at the orifice of the left atrial appendage. The sealing part 210 is used to be released and sealed at the orifice of the left atrial appendage. The anchoring part 220 is connected to the distal side of the sealing part 210 and used to be released and fixed on the inner side of the orifice of the left atrial appendage. The second conductive part 260 is used to ablate the tissue on the inner side of the orifice of the left atrial appendage.

Studies have found that the tissue at the orifice of the left atrial appendage has a smooth surface and regular shape compared with the tissue inside the orifice of the left atrial appendage. The cardiac pectinate muscles migrating into the sinus cavity of the left atrial appendage cause the complexity of the internal structure of the left atrial appendage. The muscle bundles inside the left atrial appendage are thick and mostly distributed in a feathery palm leaf shape, which is particularly obvious on the upper and lower surfaces of the left atrial appendage, while the remaining structure of the cavity wall between the muscle bundles of the left atrial appendage is extremely thin.

The anchoring part 220 is fixed on the surface of at least part of the pectinate muscles in contact with its periphery. The second conductive part 260 is arranged on the periphery of the anchoring part 220 to ablate the tissue inside the orifice of the left atrial appendage. It can be released and close to the pectinate muscles, but it is difficult to approach the entire peripheral surface of the pectinate muscles and the tissue between the muscle bundles. By increasing the average current density on the surface of the second conductive part 260, it is beneficial to increase the ablation depth and range of the second conductive part 260, and facilitate the formation of transmural ablation in the thick muscle bundles around the second conductive part 260 and the thin tissue between the muscle bundles.

The discharge area of the second conductive part 260 is smaller than that of the first conductive part 250, so as to increase the average current density on the surface of the second conductive part 260 without changing the overall ablation energy of the two conductive parts.

Preferably, the discharge area of the first conductive part 250 is 15mm²~4700mm², and the discharge area of the second conductive part 260 is 15mm²~500mm².

The surface area of the conductive part of the first conductive part 250 is relatively large, and the surface area of the conductive part of the second conductive part 260 is relatively small. When the area of the first conductive part 250 is within the above range, it can ensure that the first conductive part 250 can choose various implementation modes, such as multiple discrete electrode pieces, arc electrodes, dot electrodes, rod electrodes, electrodes extending around the outer wall of the support body for at least one circle, and other electrode forms, or the first conductive part 250 is a scheme of part of the metal framework of the support body, and the support body can be made by weaving or cutting processes. In addition, within this range, it can ensure that the area of the first conductive part 250 can facilitate the ablation of the target tissue, avoid the first conductive part 250 having too small an area, resulting in too large current density, excessive energy concentration here, forming electric sparks, and in addition, the ablation range is too small, the ablation area is reduced, and it is difficult to form a complete ablation zone when the number of conductive parts is limited; It also avoids the situation that the area of the first conductive part is too large, resulting in too low average current density of the first conductive part 250, too small ablation range and too low ablation depth.

When the second conductive part 260 is within the above range, it avoids too small area, too large current density, electric sparks, and excessive energy concentration here, leading to too small energy distribution of the first conductive part 250, inability to penetrate the wall, and reduction of the ablation area. The second conductive part 260 adopting the above range can significantly improve the ablation depth of the second conductive part and ensure the ablation depth and range of the two conductive parts. In some embodiments, the discharge area of the first conductive part 250 is 15mm²~4700mm², and the discharge area of the second conductive part 260 is 15mm²~300mm². Preferably, the distance between the first conductive part 250 and the second conductive part 260 can be further limited to 3mm~22mm, that is, the nearest distance between the first conductive part 250 and the second conductive part 260 is 3mm~22mm, that is, the distance between the closest points of the first conductive part 250 and the second conductive part 260 is 3mm~22mm. Within this range, it is convenient for the ablation areas of the two conductive parts to partially overlap, that is, the first ablation area and the second ablation area partially overlap. At least part of the two ablation areas overlap, so that the size of the ablation area formed by the superposition of the first ablation area and the second ablation area is larger. When the ablation areas partially overlap, the ablation energy of the two conductive parts is concentrated, which can ablate the tissue in a relatively large section, facilitate the formation of transmural ablation in the uneven thickness of the left atrial appendage tissue, and reduce the impact of the deviation of the framework release position on the ablation effect.

In this embodiment, the first conductive part 250 and the second conductive part 260 are arranged at intervals in the axial direction of the framework, and the first ablation area and the second ablation area are formed at different positions in the axial direction of the ablation system. After the left atrial appendage occlusion and ablation device 200 is released to the orifice of the left atrial appendage, there may be positions where the circumferential circle of the sealing part 210 and the anchoring part 220 are not closely attached to the tissue. If a conductive part is arranged at this position, and the first ablation area and the second ablation area do not overlap or completely overlap, the area range of the ablation area at the left atrial appendage position is small, the possibility that the ablation area corresponding to the conductive part covers the outer wall of the left atrial appendage tissue will be reduced, and ablation gaps are easy to form in the circumferential direction, that is, the probability that the ablation area cannot form a complete annular ablation zone in the circumferential direction is greatly increased; When the first ablation area and the second ablation area at least partially overlap, the first ablation area and the second ablation area partially overlap to form an ablation area with a large coverage area and a long axial length, that is, the two ablation areas overlap and are continuous. Then, the ablation area with a large coverage area after partial overlap can cover the outer wall of the left atrial appendage tissue, reduce the probability that the ablation area cannot form a complete annular ablation zone in the circumferential direction, increase the axial length of the annular transmural ablation zone formed in the left atrial appendage, facilitate the concentration of ablation energy for transmural ablation, and improve the ablation success rate. When the two ablation electric fields partially overlap, the ablation area is convex in the circumferential direction, so as to facilitate transmural ablation at the overlapping position of the ablation areas. It can be understood that when the first conductive part 250 and the second conductive part 260 are arranged at intervals in the circumferential direction of the framework, or the first conductive part 250 and the second conductive part 260 are arranged at intervals in any positional relationship (for example, the second conductive part is arranged on the support body, the second conductive part is arranged on the delivery device, or the second conductive part is independently arranged from the support body and the delivery device, and the directions of the first conductive part and the second conductive part are parallel or not), if the first ablation area and the second ablation area partially overlap, it is convenient to form an ablation area with a larger coverage area and a transmural annular ablation zone.

Preferably, in view of the uniform thickness and regular shape of the left atrial appendage orifice, the larger ablation area formed by the partially overlapping first ablation area and second ablation area can cover the inner and outer walls of the left atrial appendage orifice.

In some embodiments, when the pulse voltage range, the discharge area of the ablation element and the distance between the conductive parts exceed the above limited ranges, for example, the left atrial appendage occlusion and ablation device 200 can increase the voltage range and reduce the distance between the two conductive parts to make the two ablation areas partially overlap, so as to realize transmural ablation.

The natural state is the state where the left atrial appendage occlusion and ablation device 200 is not subjected to external force, that is, the left atrial appendage occlusion and ablation device 200 is not bound by the relevant components of the delivery device and the tissue.

As shown in Figure 2, in the natural state, the proximal end of the second conductive part 260 is arranged on the distal side of the distal end of the first conductive part 250. That is, the distal end of the first conductive part 250 and the proximal end of the second conductive part 260 are staggered in the axial direction, so as to increase the distance between the two conductive parts, avoid the two conductive parts having similar conditions of apposing the target tissue wall during ablation, and when one of the conductive parts has poor apposition conditions (such as being suspended), the other conductive part also has poor apposition conditions due to the close axial positions of the two conductive parts, thereby improving the apposition conditions of the two conductive parts and ensuring the ablation effect of the ablation element.

Preferably, the distance between the nearest two points on the surfaces of the first conductive part 250 and the second conductive part 260 ranges from 3mm to 18mm. When the shortest distance between the two conductive parts is 3mm~18mm, it can ensure that the interval between the two conductive parts is large, the apposition conditions of the two conductive parts are different during ablation, and the impact on the apposition conditions of the other conductive part is small when one of the apposition conditions is poor. At the same time, it ensures that the distance between the two conductive parts is not too large, and ensures the electric field intensity and ablation effect formed by the ablation element.

It should be noted that the various technical solutions provided in this embodiment can be applied to the first embodiment and other embodiments without contradiction, which will not be repeated here.

### Embodiment 3

Please refer to Figure 3. The ablation system of this embodiment includes a left atrial appendage occlusion and ablation device 300, which is used for occluding and ablating the left atrial appendage. The left atrial appendage occlusion and ablation device 300 includes a sealing part 310 arranged at the proximal end and an anchoring part 320 arranged at the distal end, and the sealing part 310 and the anchoring part 320 are connected to each other.

The main difference between the left atrial appendage occlusion and ablation device 300 provided in this embodiment and the left atrial appendage occlusion and ablation device 200 provided in the second embodiment is that in this embodiment, the second conductive part 360 is arranged on the proximal part of the second framework 321; In the second embodiment, the second conductive part 360 is arranged on the distal part of the second framework 321.

In some embodiments, the entire surface of the second framework 321 needs to be insulated. In some embodiments, the part of the second framework 321 that is not in contact with the second conductive part 360 is insulated, and the part in contact with the second conductive part 360 does not need to be insulated.

### Embodiment 4

Please refer to Figure 4. The ablation system of this embodiment includes a left atrial appendage occlusion and ablation device 400, which is used for occluding and ablating the left atrial appendage. The left atrial appendage occlusion and ablation device 400 includes a sealing part 410 arranged at the proximal end and an anchoring part 420 arranged at the distal end, and the sealing part 410 and the anchoring part 420 are connected to each other.

The main difference between the left atrial appendage occlusion and ablation device 400 provided in this embodiment and the left atrial appendage occlusion and ablation device 100 provided in the first embodiment is that the first conductive part 450 arranged on the left atrial appendage occlusion and ablation device 400 includes a plurality of electrode parts arranged at intervals on the surface of the sealing part 410, and each electrode part is a dot electrode. Each dot electrode is arranged on a different first sealing rod. In other embodiments, the number of dot electrodes on each first sealing rod can be set as required, for example, at least one first sealing rod is not provided with a dot electrode, or one first sealing rod is provided with a plurality of dot electrodes.

Each dot electrode is dot - shaped, such as a granular electrode surrounded by a straight line or a curve. The cross section of each dot electrode is circular, elliptical, annular, rectangular, trapezoidal, other polygons or irregular shapes.

In some embodiments, the plurality of dot electrodes in the first conductive part 450 are used to transmit the same ablation electric energy. In some embodiments, adjacent dot electrodes are used to transmit ablation electric energy with different parameters, such as different polarities. In some embodiments, the ablation electric energy transmitted by each dot electrode is set as required, for example, the plurality of dot electrodes are grouped, and the dot electrodes in the same group are used to transmit ablation electric energy with the same parameters.

In some embodiments, the first conductive part 450 is insulated from the first framework 411. Correspondingly, the surface of the first framework 411 is insulated, or the surface of one side of the plurality of electrode parts of the first conductive part 450 in contact with the first framework 411 is insulated.

### Embodiment 5

Please refer to Figure 5. The ablation system of this embodiment includes a left atrial appendage occlusion and ablation device 500, which is used for occluding and ablating the left atrial appendage. The left atrial appendage occlusion and ablation device 500 includes a sealing part 510 arranged at the proximal end and an anchoring part 520 arranged at the distal end, and the sealing part 510 and the anchoring part 520 are connected to each other.

The main difference between the left atrial appendage occlusion and ablation device 500 provided in this embodiment and the left atrial appendage occlusion and ablation device 400 provided in the fourth embodiment is that the plurality of electrode parts in the first conductive part 550 arranged on the left atrial appendage occlusion and ablation device 500 are all rod - shaped electrodes.

Specifically, each rod - shaped electrode is rod - shaped, or long strip - shaped, and the two ends of the rod - shaped electrode extend between the proximal end and the distal end. In some embodiments, the two ends of the rod - shaped electrode extend in the circumferential direction of the left atrial appendage occlusion and ablation device 500. In some embodiments, the extending direction of the rod - shaped electrode is inclined relative to the axis of the left atrial appendage occlusion and ablation device 500.

In some embodiments, the plurality of rod - shaped electrodes in the first conductive part 550 are used to transmit the same ablation electric energy. In some embodiments, adjacent rod - shaped electrodes are used to transmit ablation electric energy with different parameters, such as different polarities. In some embodiments, the ablation electric energy transmitted by each rod - shaped electrode is set as required, for example, the plurality of rod - shaped electrodes are grouped, and the rod - shaped electrodes in the same group are used to transmit ablation electric energy with the same parameters.

In some embodiments, the first conductive part 550 is insulated from the first framework 511. Correspondingly, the surface of the first framework 511 is insulated, or the surface of one side of the plurality of electrode parts of the first conductive part 550 in contact with the first framework 511 is insulated.

### Embodiment 6

Please refer to Figure 6A. The ablation system of this embodiment includes a left atrial appendage occlusion and ablation device 600, which is used for occluding and ablating the left atrial appendage. The left atrial appendage occlusion and ablation device 600 includes a sealing part 610 arranged at the proximal end and an anchoring part 620 arranged at the distal end, and the sealing part 610 and the anchoring part 620 are connected to each other.

The main difference between the left atrial appendage occlusion and ablation device 600 provided in this embodiment and the left atrial appendage occlusion and ablation device 500 provided in the fifth embodiment is that the plurality of electrode parts in the first conductive part 650 arranged on the left atrial appendage occlusion and ablation device 600 are all arc electrodes.

Specifically, each arc electrode is in the shape of an arc and extends in the circumferential direction of the left atrial appendage occlusion and ablation device 600, and the arc length occupied by the arc electrode is less than 360 degrees. The plurality of arc electrodes are arranged at intervals on the circumferential surface of the first framework 611.

As shown in Figure 6A, the plurality of arc electrodes form a ring shape, which is convenient for forming an annular ablation zone. The number of arc electrodes can be set as required.

The circumferential angles occupied by different arc electrodes in the circumferential direction of the left atrial appendage occlusion and ablation device 600 do not overlap. In some embodiments, the circumferential angles occupied by different arc electrodes in the circumferential direction of the left atrial appendage occlusion and ablation device 600 at least partially overlap.

As shown in Figure 6A, different arc electrodes are located at the same axial position of the left atrial appendage occlusion and ablation device 600. In other embodiments, adjacent arc electrodes are located at different axial positions.

In some embodiments, the plurality of arc electrodes in the first conductive part 650 are used to transmit the same ablation electric energy. In some embodiments, adjacent arc electrodes are used to transmit ablation electric energy with different parameters, such as different polarities. In some embodiments, the ablation electric energy transmitted by each arc electrode is set as required, for example, the plurality of arc electrodes are grouped, and the rod - shaped electrodes in the same group are used to transmit ablation electric energy with the same parameters.

In some embodiments, the first conductive part 650 is insulated from the first framework 611. Correspondingly, the surface of the first framework 611 is insulated, or the surface of one side of the plurality of electrode parts of the first conductive part 650 in contact with the first framework 611 is insulated.

Please refer to Figure 6B. Figure 6B is a schematic structural diagram of the left atrial appendage occlusion and ablation device 600 shown in Figure 6A with a membrane 690 on the surface of the support body. In this embodiment, the support body includes a membrane 690 arranged on the outer surface of the framework, so as to prevent thrombus in the left atrial appendage from flowing into the left atrium through the grid of the support body. The sealing part 610 arranged on the proximal side of the support body is used to block the left atrial appendage, and the membrane 690 covers at least the sealing part 610. It can be understood that the distal end of the membrane 690 can extend to the surface of the anchoring part 620. A membrane 690 is arranged on the surface of the support body to prevent the support body from directly contacting the tissue, increase the contact area between the support body and the tissue, thereby reducing the stimulation of the framework and metal materials of the support body to the tissue, and helping to reduce the incidence of postoperative complications. The membrane 690 is also used to maintain the stability of the framework structure, avoid excessive deformation of the framework, and improve the stability of the anchoring of the support body after implantation. As shown in Figure 6B, the distal end of the membrane 690 does not extend to the position of the anchor thorn, and the anchor thorn can be smoothly inserted into the left atrial appendage wall. In some embodiments, the membrane 690 covers the anchor thorn, and the free end of the anchor thorn can pierce the membrane and be exposed on the outer surface of the membrane 690, thus facilitating the anchoring of the support body.

In a modified embodiment, at least one of the outer surface and the inner surface of the support body is provided with a membrane 690.

The membrane 690 is arranged between the first conductive part 650 and the support body. In this embodiment, both the first conductive part 650 and the support body are made of conductive metal materials. Preferably, the membrane 690 is also an insulating membrane, which is used to realize insulation between the first conductive part 650 and the support body, and avoid short circuit between the first conductive part 650 and the support body during the process of the first conductive part 650 releasing ablation energy, that is, the ablation energy released by the first conductive part 650 is transmitted to the support body. To improve the insulation performance between the first conductive part 650 and the support body, at least one other insulation mode can be added between the first conductive part 650 and the support body, such as plating an insulating coating on the surface of the framework, sleeving an insulating sleeve on the surface of the support body framework, thickening the membrane, making at least part of the support body with an insulating material, etc.

In a modified embodiment, the insulation between the first conductive part 650 and the support body can be realized by other methods mentioned in this application except for setting a membrane. Without violating the spirit of the present invention, the membrane 690 provided in this embodiment can be applied to other embodiments to realize at least one of the functions of blocking flow, insulation, maintaining structural stability, reducing stimulation to tissues, etc., which will not be repeated here.

### Embodiment 7

Please refer to Figure 7. The ablation system of this embodiment includes a left atrial appendage occlusion and ablation device 700, which is used for occluding and ablating the left atrial appendage. The left atrial appendage occlusion and ablation device 700 includes a sealing part 710 arranged at the proximal end and an anchoring part 720 arranged at the distal end, and the sealing part 710 and the anchoring part 720 are connected to each other.

The main difference between the left atrial appendage occlusion and ablation device 700 provided in this embodiment and the left atrial appendage occlusion and ablation device 600 provided in the sixth embodiment is that the number of electrode parts included in the first conductive part 750 of the left atrial appendage occlusion and ablation device 700 is one, and the electrode form is a ring electrode. For the description of the specific ring electrode, please refer to the foregoing embodiment.

A second conductive part used in cooperation with the first conductive part 750 can be arranged in the left atrial appendage occlusion and ablation device 700, or the second conductive part can be arranged on the delivery device, or the second conductive part can be arranged independently of the left atrial appendage occlusion and ablation device 700 and the delivery device, which are allowed. For details, please refer to the first embodiment.

### Embodiment 8

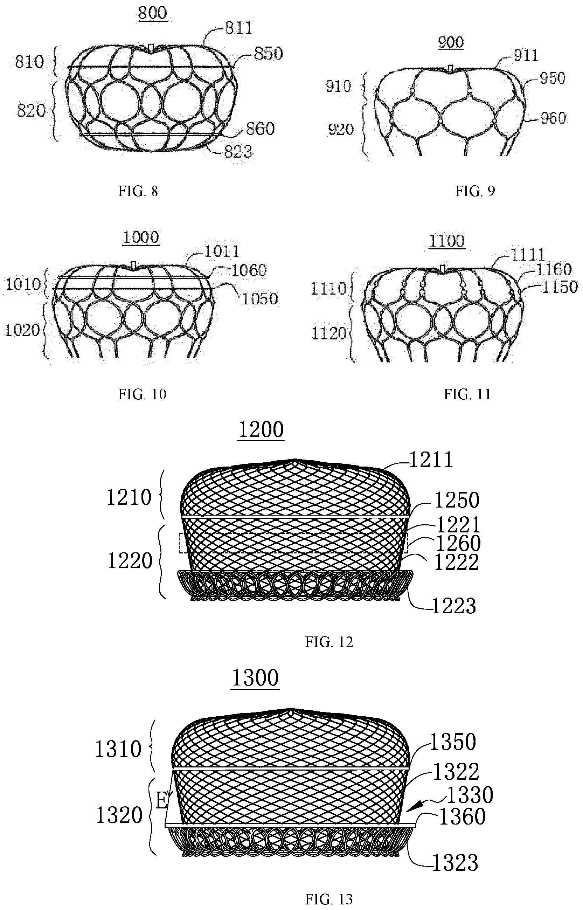

Please refer to Figure 8. The ablation system of this embodiment includes a left atrial appendage occlusion and ablation device 800, which is used for occluding and ablating the left atrial appendage. The left atrial appendage occlusion and ablation device 800 includes a sealing part 810 arranged at the proximal end and an anchoring part 820 arranged at the distal end, and the sealing part 810 and the anchoring part 820 are connected to each other.

The main difference between the left atrial appendage occlusion and ablation device 800 provided in this embodiment and the left atrial appendage occlusion and ablation device 700 provided in the seventh embodiment is that the distal end of the anchoring part 820 of the left atrial appendage occlusion and ablation device 800 is a closed structure, and the second conductive part 860 is arranged on the framework of the left atrial appendage occlusion and ablation device 800, specifically on the anchoring part 820, and the second conductive part 860 is an electrode part arranged on the surface of the anchoring part 820, and the form of the electrode part is a ring electrode.

In this embodiment, the distal end of the anchoring part 820 is closed, that is, the distal ends of the anchoring rods 823 are combined together to close the distal end of the anchoring part 820. In some embodiments, the distal end of the anchoring part 820 is also provided with a connecting piece for converging the ends of the anchoring rods 823.

The specific position of the second conductive part 860 on the anchoring part 820 can be set as required.

The electric field formed between the first conductive part 850 and the second conductive part 860 is used to ablate the tissue at the orifice of the left atrial appendage. For the description of the ring electrodes of the first conductive part 850 and the second conductive part 860, please refer to the foregoing embodiment. This embodiment can adopt the flow blocking and insulation modes in the sixth embodiment, for example, a membrane is used to block flow, and realize insulation between the first conductive part 850 and the sealing part 810, and between the second conductive part 860 and the anchoring part 820, etc.

### Embodiment 9

Please refer to Figure 9. The ablation system of this embodiment includes a left atrial appendage occlusion and ablation device 900, which is used for occluding and ablating the left atrial appendage. The left atrial appendage occlusion and ablation device 900 includes a sealing part 910 arranged at the proximal end and an anchoring part 920 arranged at the distal end, and the sealing part 910 and the anchoring part 920 are connected to each other.

The main difference between the left atrial appendage occlusion and ablation device 900 provided in this embodiment and the left atrial appendage occlusion and ablation device 800 provided in the eighth embodiment is that the first conductive part 950 and the second conductive part 960 arranged on the framework of the left atrial appendage occlusion and ablation device 900 are in the form of dot electrodes. For the description of the specific dot electrodes, please refer to the foregoing embodiment. The second conductive part 960 is arranged closer to the proximal end of the support body than the second conductive part 860.

### Embodiment 10

Please refer to Figure 10. The ablation system of this embodiment includes a left atrial appendage occlusion and ablation device 1000, which is used for occluding and ablating the left atrial appendage. The left atrial appendage occlusion and ablation device 1000 includes a sealing part 1010 arranged at the proximal end and an anchoring part 1020 arranged at the distal end, and the sealing part 1010 and the anchoring part 1020 are connected to each other.

The main difference between the left atrial appendage occlusion and ablation device 1000 provided in this embodiment and the left atrial appendage occlusion and ablation device 800 provided in the eighth embodiment is that both the first conductive part 1050 and the second conductive part 1060 arranged in the left atrial appendage occlusion and ablation device 1000 are arranged on the sealing part 1010.

On the sealing part 1010, the first conductive part 1050 and the second conductive part 1060 are arranged at different axial positions, and the first conductive part 1050 is arranged on the distal side of the second conductive part 1060. Both the first conductive part 1050 and the second conductive part 1060 are in the form of ring electrodes. For the description of the specific ring electrodes, please refer to the foregoing embodiment.

Since the first conductive part 1050 is arranged in the edge area of the sealing part 1010, preferably, the first conductive part 1050 is arranged at the position with the largest radial size of the sealing part 1010, and correspondingly, the second conductive part 1060 can be arranged on the proximal side or the distal side of the first conductive part 1050 as required.

### Embodiment 11

Please refer to Figure 11. The ablation system of this embodiment includes a left atrial appendage occlusion and ablation device 1100, which is used for occluding and ablating the left atrial appendage. The left atrial appendage occlusion and ablation device 1100 includes a sealing part 1110 arranged at the proximal end and an anchoring part 1120 arranged at the distal end, and the sealing part 1110 and the anchoring part 1120 are connected to each other.

The main difference between the left atrial appendage occlusion and ablation device 1100 provided in this embodiment and the left atrial appendage occlusion and ablation device 1000 provided in the tenth embodiment is that both the first conductive part 1150 and the second conductive part 1160 arranged on the framework of the left atrial appendage occlusion and ablation device 1100 are in the form of dot electrodes. For the description of the specific dot electrodes, please refer to the foregoing embodiment.

### Embodiment 12

Please refer to Figure 12. The ablation system of this embodiment includes a left atrial appendage occlusion and ablation device 1200, which is used for occluding and ablating the left atrial appendage. The left atrial appendage occlusion and ablation device 1200 includes a sealing part 1210 arranged at the proximal end and an anchoring part 1220 arranged at the distal end, and the sealing part 1210 and the anchoring part 1220 are connected to each other.

The main difference between the left atrial appendage occlusion and ablation device 1200 provided in this embodiment and the left atrial appendage occlusion and ablation device 700 provided in the seventh embodiment is that the structures of the sealing part 1210 and the anchoring part 1220 in the left atrial appendage occlusion and ablation device 1200 are different from those in the seventh embodiment.

Specifically, the left atrial appendage occlusion and ablation device 1200 is made by weaving. The sealing part 1210 is the proximal part of the left atrial appendage occlusion and ablation device 1200, and the anchoring part 1220 is the distal part of the left atrial appendage occlusion and ablation device 1200. The anchoring part 1220 includes a main body 1222 and a reinforcing ring 1223 which can be arranged at the distal end of the main body 1222, and the reinforcing ring 1223 is arranged along the circumferential direction of the main body.

The reinforcing ring 1223 is fixed on the outer circumferential surface of the main body 1222, and the part of the reinforcing ring 1223 close to the main body 1222 is connected with the main body 1222. The part of the reinforcing ring 1223 radially far away from the main body 1222 protrudes radially in the direction far away from the axis of the left atrial appendage occlusion and ablation device 1200, that is, the reinforcing ring 1223 is convexly arranged on the periphery of the distal end of the main body 1222. The reinforcing ring 1223 is used to be released inside the left atrial appendage, contact and push against the left atrial appendage tissue, and improve the anchoring performance of the left atrial appendage occlusion and ablation device 1200.

The reinforcing ring 1223 is a plurality of annular support rings connected to each other in the circumferential direction, and the plurality of annular support rings are arranged in a circle in the circumferential direction. In some embodiments, each annular support ring forms a complete ring shape. In some embodiments, each annular support ring is a curved structure, not forming a complete ring shape, and the ends of adjacent annular support rings are connected to obtain the reinforcing ring 1223.

The sealing part 1210 and the main body 1222 can be made by integral braiding. In some embodiments, the left atrial appendage occlusion and ablation device 1200 is made by integral braiding.

In this embodiment, the first conductive part 1250 is an electrode part arranged on the surface of the sealing part 1210, and the specific electrode form is a ring electrode. For the description of the ring electrode, please refer to the foregoing embodiment.

As shown in Figure 12, in some embodiments, the second conductive part 1260 is arranged on the left atrial appendage occlusion and ablation device 1200. For example, the second conductive part 1260 is at least part of the second framework 1221. In some embodiments, the second conductive part 1260 is at least part of the framework in the reinforcing ring 123. The first conductive part 1250 and the second conductive part 1260 interact to facilitate the formation of an annular ablation zone at the orifice of the left atrial appendage. For other arrangement modes of the second conductive part 1260, please refer to the seventh and first embodiments.

In some embodiments, the reinforcing ring 1223 is arranged on the sealing part 1210, protruding in a circle in the circumferential direction of the sealing part 1210, and used for blocking the orifice of the left atrial appendage. In this embodiment, the first conductive part 1250 can be at least part of the framework in the reinforcing ring 1223, so as to facilitate the formation of an annular ablation zone at the orifice of the left atrial appendage.

### Embodiment 13

Please refer to Figure 13. The ablation system of this embodiment includes a left atrial appendage occlusion and ablation device 1300, which is used for occluding and ablating the left atrial appendage. The left atrial appendage occlusion and ablation device 1300 includes a sealing part 1310 arranged at the proximal end and an anchoring part 1320 arranged at the distal end, and the sealing part 1310 and the anchoring part 1320 are connected to each other.

The main difference between the left atrial appendage occlusion and ablation device 1300 provided in this embodiment and the left atrial appendage occlusion and ablation device 1200 provided in the twelfth embodiment is that the second conductive part 1360 is an electrode part arranged on the surface of the anchoring part 1320, more specifically, the electrode part is in the form of a ring electrode. For the description of the specific ring electrode, please refer to the foregoing embodiment.

One radial end of the reinforcing ring 1323 is connected to the main body 1322, and the other radial end of the reinforcing ring 1323 is a free end, protruding radially outward relative to the main body. The second conductive part 1360 is arranged at the free end of the reinforcing ring 1323. In some embodiments, the second conductive part 1360 is arranged at the position with the largest radial size of the reinforcing ring, so as to facilitate the second conductive part 1360 to contact the tissue. The middle parts of the plurality of annular support rings of the reinforcing ring 1323 are interconnected to form a channel. In some embodiments, the second conductive part 1360 is inserted into the channel formed by the reinforcing ring 1323.

The left atrial appendage occlusion and ablation device 1300 includes a accommodating part 1330. The accommodating part 1330 is arranged adjacent to the axis of the support body relative to the first conductive part 1350 and/or the second conductive part 1360. In this embodiment, the accommodating part 1330 is arranged adjacent to the axis of the support body relative to both the first conductive part 1350 and the second conductive part 1360, so that a containing space for accommodating left atrial appendage tissue is formed on the outer side of the circumferential outer wall of the accommodating part 1330.

The shortest line between any point on the first conductive part 1350 and any point on the second conductive part 1360 is a characteristic electric field line E. The characteristic electric field line E is linear, starting from one of the first conductive part 1350 and the second conductive part 1360 and ending at the other of the first conductive part 1350 and the second conductive part 1360. The electric field intensity distributed near the characteristic electric field line E is denser than that at other positions in the electric field, and the electric field intensity is greater. The electric field intensity at this position is more likely to be greater than the threshold field intensity of the target tissue. Therefore, the tissue through which the characteristic electric field line E passes is easy to be ablated, and the ablation effect is better.

As shown in Figure 13, the first conductive part 1350 and the second conductive part 1360 are arranged on opposite sides of the containing space. During ablation, at least one characteristic electric field line E passes through the area outside the support body, that is, the area on the side of the framework away from its axis, specifically the containing space formed outside the accommodating part 1330 of the framework. This facilitates the characteristic electric field line E to pass through the tissue located in the containing space.

Specifically, as shown in Figure 13, the radial size of the part of the main body 1322 between the first conductive part 1350 and the second conductive part 1360, which is used to be attached to the left atrial appendage tissue wall, gradually decreases from the proximal end to the distal end (from the sealing part to the anchoring part). The second conductive part 1360 is arranged at the position with the largest radial size of the reinforcing ring 1323, and the radial size of the second conductive part 1360 is larger than that of the first conductive part 1350, or the radial size of the second conductive part 1360 is similar to that of the first conductive part 1350. In this way, the part of the framework of the left atrial appendage occlusion and ablation device 1300 between the first conductive part 1350 and the second conductive part 1360 is the accommodating part 1330, and a containing space for accommodating left atrial appendage tissue is formed on the outer side of the outer wall of the accommodating part 1330. When the left atrial appendage occlusion and ablation device 1300 is implanted into the left atrial appendage, at least part of the outer wall of the accommodating part 1330 is used to be attached to the left atrial appendage tissue.

The left atrial appendage occlusion and ablation device 1300 is made by braiding. The accommodating part 1330 between the first conductive part 1350 and the second conductive part 1360 is arranged around the circumferential direction of the left atrial appendage occlusion and ablation device 1300, and the first conductive part 1350 and the second conductive part 1360 are arranged at intervals in the axial direction, so that the tissue in a circle in the circumferential direction of the left atrial appendage can be accommodated in the accommodating part 1330, and the first conductive part 1350 and the second conductive part 1360 ablate the tissue in the accommodating part 1330, forming a continuous annular ablation zone in the left atrial appendage.

It can be understood that the formation of the accommodating part 1330 is not limited to the framework structure of the left atrial appendage occlusion and ablation device provided in the plurality of embodiments of this application, but can also be other structures recessed towards the axis direction of the left atrial appendage occlusion and ablation device between the carriers of the first conductive part and the second conductive part, so that the part between the first conductive part and the second conductive part forms the above - mentioned accommodating space, that is, the accommodating part for accommodating the target tissue can be arranged on the support body and the delivery device, or formed between the support body and the delivery device.

### Embodiment 14

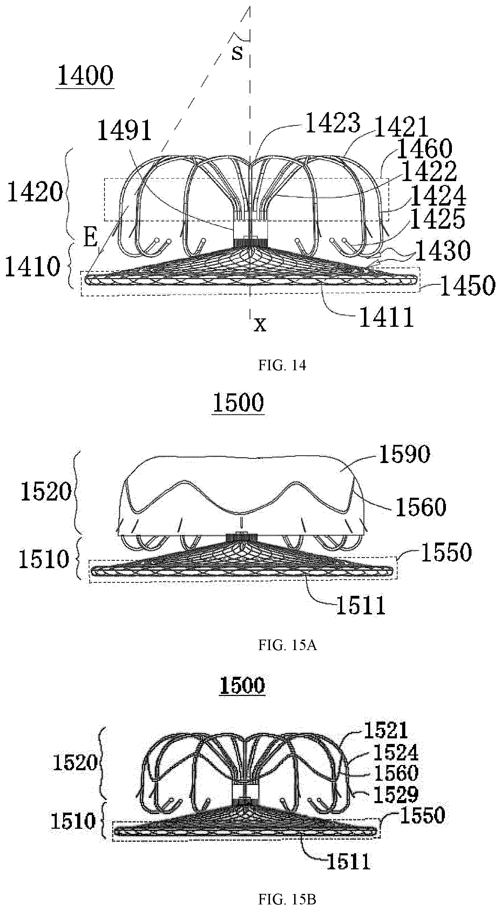

Please refer to Figure 14. The ablation system of this embodiment includes a left atrial appendage occlusion and ablation device 1400, which is used for occluding and ablating the left atrial appendage. The left atrial appendage occlusion and ablation device 1400 includes a sealing part 1410 arranged at the proximal end and an anchoring part 1420 arranged at the distal end, and the sealing part 1410 and the anchoring part 1420 are connected to each other.

The main difference between the left atrial appendage occlusion and ablation device 1400 provided in this embodiment and the left atrial appendage occlusion and ablation device 100 provided in the first embodiment is that the left atrial appendage occlusion and ablation device 1400 has a double - disc structure, the sealing part 1410 is disc - shaped, the anchoring part 1420 is disc - shaped, and the sealing part 1410 and the anchoring part 1420 are connected in an insulated manner through a connecting piece 1491. At least part of the connecting piece 1491 can be made of an insulating material to realize insulated connection between the sealing part 1410 and the anchoring part 1420.

Specifically, the first framework 1411 in the sealing part 1410 is made by braiding. From the perspective of Figure 14, the distal end of the sealing part 1410 is conical. It can be understood that the proximal end of the sealing part 1410 is planar, conical or other shapes.

The second framework 1421 in the anchoring part 1420 is obtained by laser cutting a pipe. The second framework 1421 includes a first anchoring rod 1422, a second anchoring rod 1423, a third anchoring rod 1424 and a fourth anchoring rod 1425 which are connected in sequence.

The proximal end of the first anchoring rod 1422 is connected to the connecting piece 1491, and the first anchoring rod 1422 extends between the proximal end and the distal end. One end of the first anchoring rod 1422 far away from the connecting piece 1491 is connected to the ends of two second anchoring rods 1423. One end of each second anchoring rod 1423 is connected to a corresponding first anchoring rod 1422, and the other ends of two adjacent second anchoring rods 1423 are connected together and connected to a corresponding third anchoring rod 1424. Adjacent second anchoring rods 1423 are connected end to end to form a sawtooth structure. The second anchoring rods 1423 are used to form the distal end surface of the anchoring part 1420, and the third anchoring rods 1424 extend between the proximal end and the distal end, arranged on the periphery of the first anchoring rods 1422, and used to lean against the inner wall of the left atrial appendage. The proximal end of the third anchoring rod 1424 is connected to the fourth anchoring rod 1425, one end of the fourth anchoring rod 1425 is connected to the third anchoring rod 1424, and the other end of the fourth anchoring rod 1425 is a free end, which extends towards the axis and to the distal end.

The first conductive part 1450 is arranged on the sealing part 1410, and the first conductive part 1450 is at least part of the framework of the first framework 1411 in the sealing part 1410, such as all the first framework 1411 or part of the first framework 1411. In Figure 14, the first conductive part 1450 is the circumferential edge area of the sealing part 1410, at least arranged in the area with the largest radial size in the circumferential edge area of the sealing part 1410. In addition, the first conductive part 1450 is at least arranged in the area of the circumferential edge area of the sealing part 1410 facing the anchoring part 1420, which is used to lean against the position of the orifice of the left atrial appendage. In some embodiments, the first conductive part 1450 is arranged in the area of the circumferential edge area of the sealing part 1410 facing the anchoring part 1420, and at least part of the area of the sealing part 1410 opposite to the anchoring part 1420 (at least a portion of the area of the proximal surface).

Optionally, the anchoring part 1420 can use at least part of the second framework 1421 as the second conductive part 1460, and the pulse ablation electric field formed between the first conductive part 1450 and the second conductive part 1460 is used to ablate the tissue.

In the pulse ablation electric field, the electric field lines point from the first conductive part 1450 to the second conductive part 1460, or from the second conductive part 1460 to the first conductive part 1450. Along the direction of the electric field lines, the closer to the first conductive part 1450 or the second conductive part 1460, the denser the electric field lines, the greater the ablation electric field intensity, and the easier it is to form a transmural ablation zone.

Since the tissue at the orifice of the left atrial appendage leans against the first conductive part 1450, and the electric field lines point to the second conductive part 1460 arranged on the anchoring part 1420, or from the second conductive part 1460 to the first conductive part 1450, most of the electric field lines will pass through the tissue at the orifice of the left atrial appendage. The electric field lines at the tissue at the orifice of the left atrial appendage are dense, the electric field intensity is high, and it is easy to form a transmural ablation zone. The electric field greater than the myocardial injury threshold field intensity passes through the tissue at the orifice of the left atrial appendage, and the success rate of ablation is high.

On the other hand, the shortest line between any point on the first conductive part 1450 and any point on the second conductive part 1460 is a characteristic electric field line E of the first conductive part 1450 and the second conductive part 1460. The area near the characteristic electric field line E has a relatively higher electric field intensity than other positions in the electric field, and is an area where the electric field energy is relatively concentrated.

Since Figure 14 is a view of the framework in a natural state of expansion without external force, the radial size of the sealing part 1410 is larger than that of the orifice of the left atrial appendage, so that the edge area with a larger radial size on the surface of the sealing part 1410 facing the anchoring part 1420 can lean against the tissue on the side of the orifice of the left atrial appendage close to or facing the left atrium. The tissue at the orifice of the left atrial appendage is clamped between the sealing part 1410 and the anchoring part 1420.

As shown in Figure 14, the distal end surface of the sealing part 1410 is conical, and the radial size gradually decreases from the proximal end to the distal end. The first conductive part 1450 is at least the edge part of the first framework 1411. The framework of the left atrial appendage occlusion and ablation device 1400 includes an accommodating part 1430 arranged between the first conductive part 1450 and the second conductive part 1460, and the accommodating part 1430 is specifically the part where the first framework and the second framework face and are close to each other. An accommodating space for accommodating the left atrial appendage tissue is formed on the outer side of the outer wall of the accommodating part 1430. After the left atrial appendage occlusion and ablation device 1400 is implanted into the left atrial appendage, at least part of the outer wall of the accommodating part 1430 is used to lean against the left atrial appendage tissue.

Preferably, at least one part of the second conductive part 1460 is closer to the axis of the support body than the first conductive part 1450, so that the distal part of at least one characteristic electric field line E passing through the accommodating space (the part close to the second conductive part 1360) inclines towards the axis of the support body. The conductive parts are all arranged on the periphery of the support body, which can reduce the difficulty of the characteristic electric field line E passing through the tissue in the accommodating space, facilitate the characteristic electric field line E passing through the accommodating space to pass through more tissues to be ablated, and ensure the ablation effect.

In this embodiment, the radial size of the sealing part 1410 is larger than that of the anchoring part 1420, and the first conductive part 1450 is arranged at the position with the largest radial size of the sealing part 1410. Therefore, the radial size of the first conductive part 1450 is larger than that of the second conductive part 1460, which facilitates more distal parts of the characteristic electric field lines E passing through the first conductive part 1450 to incline towards the axis direction of the left atrial appendage occlusion and ablation device 1400, so that more characteristic electric field lines passing through the first conductive part 1450 pass through the storage space and the deep tissue at the orifice of the left atrial appendage, and facilitate the formation of an electric field intensity greater than the myocardial electric field threshold within a certain thickness range of the tissue at the orifice of the left atrial appendage, thus forming transmural ablation at the orifice of the left atrial appendage.

In the natural state, the difference in radial size between the first conductive part 1450 and the second conductive part 1460 is 3 - 15mm. The first conductive part 1450 and the second conductive part 1460 are used to be close to the target tissue. When the difference in radial size between the first conductive part 1450 and the second conductive part 1460 is within the above range, it can ensure that the distal parts of at least some characteristic electric field lines between the first conductive part 1450 and the second conductive part 1460 incline towards the axis, and when the first conductive part 1450 and the second conductive part 1460 act on the open tissue, it is convenient for the characteristic electric field lines to pass through more tissues to be ablated, ensuring the ablation effect, and avoiding the problem that the distance between the two conductive parts is too far and the electric field intensity is reduced too much due to the too large difference in radial size between the two conductive parts.

Define: the first characteristic point is the point with the largest radial size on the periphery of the first conductive part 1450; the second characteristic point is the point with the largest radial size on the periphery of the second conductive part 1460; the characteristic plane is the plane passing through the axis of the support body and the first characteristic point.

In the natural state, the projection of the shortest one of the characteristic electric field lines between the selected first characteristic point and at least one second characteristic point on the characteristic plane forms an angle s of 0 - 70° with the axis x of the support body.

The first characteristic point is a point with the largest radial size on the periphery of the first conductive part 1450. The first characteristic point can be any one of the multiple points with the largest radial size on the periphery of the first conductive part 1450. The selected first characteristic point is a determined point with the largest radial size on the periphery of the first conductive part. In the embodiment where the largest radial size on the periphery of the first conductive part is in the shape of an annular line, the selected first characteristic point can be any point in the annular line. The first characteristic point is located in the characteristic plane.

The second characteristic point is a point with the largest radial size on the periphery of the second conductive part 1460. The second characteristic point can be any one of the multiple points with the largest radial size on the periphery of the second conductive part 1460. In the embodiment where the largest radial size on the periphery of the second conductive part is in the shape of an annular line, the second characteristic point can be any point in the annular line. The second characteristic point can be located in the characteristic plane or outside the characteristic plane.

The number of the second characteristic points is at least one. A characteristic electric field line can be obtained between the selected first characteristic point and each second characteristic point, and a plurality of characteristic electric field lines corresponding to the second characteristic points one by one can be obtained between the selected first characteristic point and the plurality of second characteristic points. Among these characteristic electric field lines, there is the shortest characteristic electric field line passing through the selected first characteristic point, for example, this shortest characteristic electric field line passing through the selected first characteristic point is the characteristic electric field line E shown in Figure 14. The starting point of the characteristic electric field line E shown in Figure 14 is the second characteristic point, and the end point is the selected first characteristic point. The shortest characteristic electric field line E passing through the selected first characteristic point is the characteristic electric field line radially farthest from the axis x of the support body, that is, these characteristic electric field lines E are formed on the periphery of the left atrial appendage occlusion and ablation device 1400, and are more likely to pass through the accommodating space outside the support body than other characteristic electric field lines, representing the ability of the two conductive parts to radiate electric fields outside the support body.

Compared with other characteristic electric field lines passing through the selected first characteristic point, the shortest characteristic electric field line E passing through the selected first characteristic point has relatively denser distribution of surrounding electric field lines and relatively more concentrated ablation energy. It can be considered that the ablation effect of the target tissue area corresponding to the shortest characteristic electric field line passing through the first characteristic point is better and plays a main ablation role.

The projection of the shortest characteristic electric field line E passing through the selected first characteristic point on the characteristic plane has a certain inclined angle s with the axis x of the support body. The characteristic electric field line E is inclined relative to the axis x, which is convenient for the characteristic electric field line E to pass through more tissues to be ablated, and is beneficial to ensuring the success rate of ablation. In this embodiment, the characteristic plane passes through the axis x, the first characteristic point and the second characteristic point. In other embodiments, the second characteristic point may not be in the characteristic plane, and the shortest characteristic electric field line passing through the selected first characteristic point needs to be projected onto the characteristic plane. This inclined angle s represents the degree of radial shrinkage of the second conductive part 1460 relative to the first conductive part 1450 when the distance between the two conductive parts is small. Preferably, the angle s is 0° - 70°, which controls the angle not to be too large, ensures that the distance between the two conductive parts is not too large, and ensures the intensity of the ablation electric field.

In the first embodiment shown in Figure 1, if the second conductive part 1460 is arranged on the distal side of the sealing part 110, such as on the anchoring part 1420, or on the delivery device, or independently of the left atrial appendage occlusion and ablation device 100 and the delivery device, the ablation effect of the left atrial appendage tissue near the first conductive part 1450 and the second conductive part is better, especially the tissue in contact with the first conductive part 1450 and the second conductive part. When the characteristic electric field lines pass through the surface of the left atrial appendage tissue or the deep position of the left atrial appendage tissue, a larger ablation thickness can be ensured, and transmural ablation can be ensured. Since the thickness of the tissue at the orifice of the left atrial appendage is relatively uniform and the shape is regular, it is easy to ablate there, and the difficulty of transmurality is low.

The above technical solutions can be applied to other embodiments, which will not be repeated in other embodiments.

As shown in Figure 14, in some embodiments, an electrode part is additionally arranged on the second framework 1421 as the second conductive part 1460, or a second conductive part is arranged outside the left atrial appendage occlusion and ablation device 1400, such as on the delivery device of the left atrial appendage occlusion and ablation device 1400, or independently of the left atrial appendage occlusion and ablation device 1400 and the delivery device.

As shown in Figure 14, the first conductive part 1450 is arranged on the proximal part of the framework, and the second conductive part 1460 is arranged on the distal side of the first conductive part 1450. The radial sizes of the two conductive parts at their positions on the framework can be set to be different or the same. Preferably, as shown in Figure 14, the radial size of the first conductive part 1450 is larger than that of the second conductive part 1460. In some embodiments, a first conductive part 1450 is arranged in the circumferential edge area with the largest radial size on the first framework 1411, such as a ring electrodethe is arranged in circumferential edge area with the largest radial size on the proximal side of the first framework 1411. Since the radial size of the first conductive part 1450 is set to be larger than that of the second conductive part 1460, it is convenient for the characteristic electric field line E to pass through the orifice tissue.

### Embodiment 15

Please refer to Figure 15A and Figure 15B. The ablation system of this embodiment includes a left atrial appendage occlusion and ablation device 1500, which is used for occluding and ablating the left atrial appendage. The left atrial appendage occlusion and ablation device 1500 includes a sealing part 1510 arranged at the proximal end and an anchoring part 1520 arranged at the distal end, and the sealing part 1510 and the anchoring part 1520 are connected to each other.

The main difference between the left atrial appendage occlusion and ablation device 1500 provided in this embodiment and the left atrial appendage occlusion and ablation device 1400 provided in the fourteenth embodiment is that the second conductive part 1560 is arranged on the left atrial appendage occlusion and ablation device 1500, and the second conductive part 1560 is an electrode part arranged on the surface of the anchoring part 1520. The anchoring part 1520 includes a second framework 1521 and a membrane 1590 arranged on the outer surface of the second framework 1521. The surface of the anchoring part 1520 is provided with the membrane 1590, and the second conductive part 1560 is arranged on the surface of the membrane 1590 facing away from the second framework 1521. The membrane 1590 is used to realize insulation between the anchoring part 1520 and the second conductive part 1560. It can be understood that other insulation modes provided in other embodiments can be adopted between the second framework 1521 and the second conductive part 1560, such as arranging an insulating coating or sleeving an insulating sleeve on at least one of the second framework 1521 and the second conductive part 1560. In some embodiments, at least two same or different insulation modes are adopted between the anchoring part 1520 and the second conductive part 1560, such as arranging the membrane 1590 between the second framework 1521 and the second conductive part 1560, and arranging an insulating coating or an insulating sleeve on the surface of the second framework 1521 of the anchoring part 1520. It can be understood that the membrane 1590 also has the function of blocking flow.

The second conductive part 1560 can be a wave electrode.

Specifically, the wave electrode extends in the circumferential and axial directions to form a preset electrode pattern. For example, as shown in Figure 15A, the wave electrode extends in the circumferential direction of the left atrial appendage occlusion and ablation device 1500 and alternately extends to the proximal end or the distal end in the axial direction, thus extending in a wave - like trajectory on the side wall of the anchoring part 1520 in the circumferential direction, expanding the occupied space of the second conductive part 1560 in the axial direction, increasing the ablation range, and facilitating the loading and release of the left atrial appendage occlusion and ablation device 1500.

In this embodiment, the first conductive part 1550 and the second conductive part 1560 meet the parameters provided in the foregoing embodiment, for example, during ablation, the electric field intensity generated at a position 3mm away from the surfaces of the first conductive part 1550 and the second conductive part 1560 is greater than 400V/cm. In some embodiments, the ablation system is used to ablate cardiac tissues other than the left atrial appendage, such as the pulmonary vein with a thickness of generally 2mm. It is ensured that during ablation, the electric field intensity generated at a position 2mm away from the surface of the ablation part is greater than the myocardial threshold field intensity, such as greater than 400V/cm, so as to facilitate transmural ablation.

In the embodiment where the ablation part is arranged on the support body and/or the delivery device, the pulse voltage range adopted by the ablation part (the first conductive part 1550 and the second conductive part 1560) is 500V ~ 4000V, the discharge area of the first conductive part 1550 is 15mm² ~ 4700mm², and the discharge area of the second conductive part 1560 is 15mm² ~ 4700mm². When the average current density on the surface of the second conductive part 1560 is greater than that of the first conductive part 1550, the discharge area of the second conductive part 1560 can refer to 15mm² ~ 500mm² provided in the foregoing embodiment. When the conductive part has multiple electrode parts, the value range of the discharge area refers to the sum of all discharge areas (electrode parts or frameworks) of the conductive part. The distance between the nearest two points on the surfaces of the first conductive part 1550 and the second conductive part 1560 ranges from 1mm to 45mm. Preferably, the distance between the first conductive part 1550 and the second conductive part 1560 can be further limited to 3mm ~ 22mm. Within this range, it is convenient for the ablation areas of the two conductive parts to partially overlap. To avoid similar wall - apposition conditions between the two conductive parts, that is, to ensure a certain interval distance between the two conductive parts, the distance between the nearest two points on the surfaces of the two conductive parts ranges from 3mm to 18mm.

As shown in Figure 15B, the second conductive part 1560 is a wave - shaped electrode with wave crests and wave troughs, where the wave crest of the second conductive part 1560 is the vertex of the second conductive part 1560 far away from the sealing part 1510, and the wave trough of the second conductive part 1560 is the vertex of the second conductive part 1560 adjacent to the sealing part 1510. In this embodiment, the wave crests and wave troughs of the second conductive part 1560 are arranged corresponding to the third anchoring rods 1524 of the anchoring part 1520. The wave crests and wave troughs of the second conductive part 1560 can be fixedly arranged on the membrane 1590, or pass through the membrane 1590 and be fixed on the third anchoring rods 1524. The third anchoring rods 1524 can be provided with connecting parts (such as through holes) for fixedly connecting the third anchoring rods 1524.

In this embodiment, the anchor thorns 1529 pass through the membrane 1590 and are exposed on the outer side of the membrane 1590. The anchor thorns 1529 are arranged on the third anchoring rods 1524, and specifically, one anchor thorn 1529 is arranged on each third anchoring rod 1524. The anchor thorns 1529 are arranged on the proximal side of the second conductive part 1560, and the radial size of the third anchoring rods 1524 at this position is large, which is beneficial to ensuring the anchoring performance of the anchoring part 1520. It can be understood that in some embodiments, the radial size of the part of the third anchoring rod 1524 where the anchor thorn 1529 is arranged is the same as that of the part of the third anchoring rod 1524 where the second conductive part 1560 is arranged, or the radial size of the part of the third anchoring rod 1524 where the anchor thorn 1529 is arranged is smaller than that of the part of the third anchoring rod 1524 where the second conductive part 1560 is arranged.

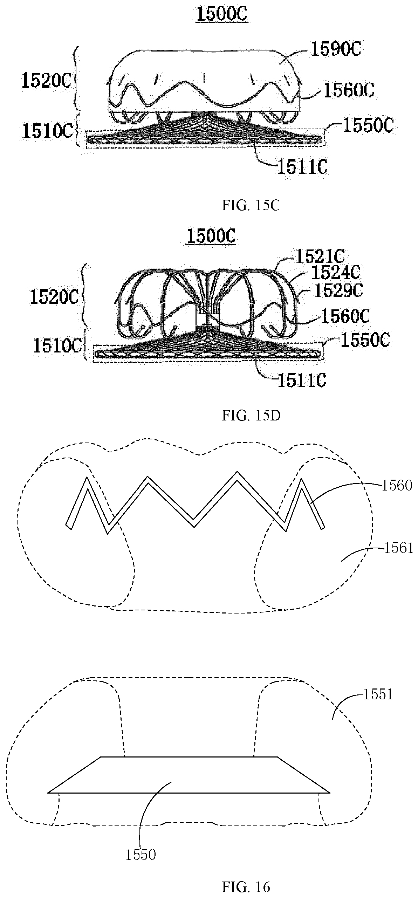

Please refer to Figure 15C and Figure 15D. In a modified embodiment based on the fifteenth embodiment, the main difference between the left atrial appendage occlusion and ablation device 1500C and the left atrial appendage occlusion and ablation device 1500 is that the relative positions of the second conductive part 1560C and the third anchoring rod 1524C are different, and the relative positions of the second conductive part 1560C and the anchor thorn 1529C are different.

Specifically, in this embodiment, the wave crests and wave troughs of the second conductive part 1560C are arranged staggered from the third anchoring rods 1524C, that is, the wave crests of the second conductive part 1560C are arranged between two adjacent anchoring rods 1524C, and the wave troughs of the second conductive part 1560C are also arranged between two adjacent anchoring rods 1524C. The second conductive part 1560C can be fixed on the membrane 1590C through its wave crests, wave troughs, or the parts between the wave crests and wave troughs, or pass through the membrane 1590C and be fixed on the third anchoring rods 1524C. Preferably, the second conductive part 1560C is fixed on the membrane 1590C at the positions of the wave crests and wave troughs, so as to improve the insulation performance between the second conductive part 1560C and the anchoring part 1520C.

As shown in Figure 15C and Figure 15D, in this embodiment, the second conductive part 1560C is arranged on the proximal side of the anchor thorn 1529C, and further, when an ablation electric field is formed between the first conductive part 1550C and the second conductive part 1560C, the electric field intensity at the position of the anchor thorn 1529C is small, avoiding the anchor thorn 1529C discharging to the tissue under the action of the ablation electric field, and improving the safety of the left atrial appendage occlusion and ablation device 1500C.

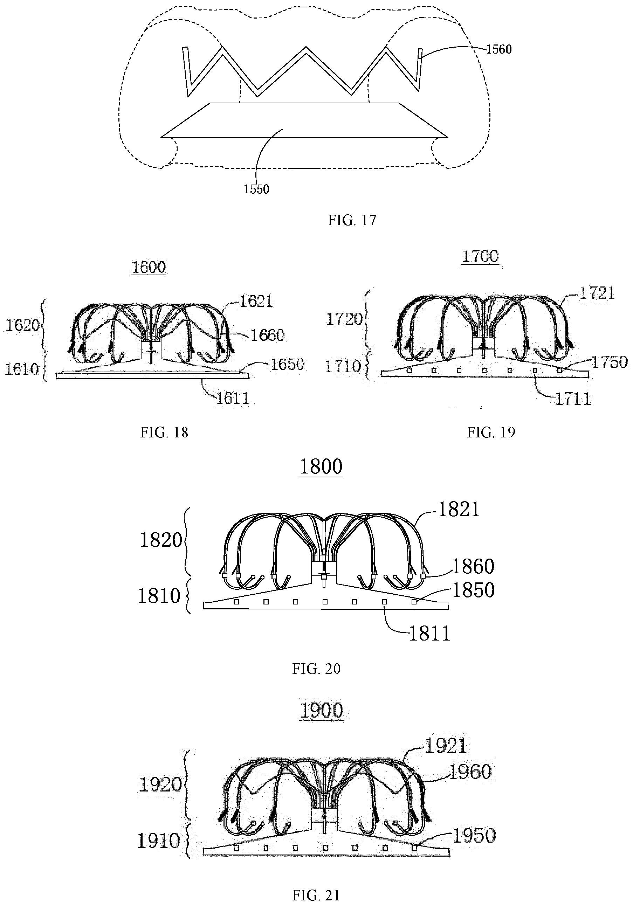

The following takes the ablation part in the fifteenth embodiment as an example to illustrate the distribution of ablation areas corresponding to the ablation part in the preferred embodiment of this application. Figure 16 is a schematic diagram of the electric field of the interval distribution of ablation areas of the ablation part provided in the fifteenth embodiment, and Figure 17 is a schematic diagram of the electric field of the partial overlapping distribution of ablation areas of the ablation part provided in the fifteenth embodiment. In Figure 16, the area around the first conductive part 1550 where the electric field intensity is greater than the myocardial threshold intensity is the first ablation area 1551, and the area around the second conductive part 1560 where the electric field intensity is greater than the myocardial threshold intensity is the second ablation area 1561. Both Figure 16 and Figure 17 are schematic diagrams of the distribution when the ablation area corresponds to an electric field intensity of 400V/cm or more under the condition that the pulse frequency range is 500Hz ~ 3MHz. The ablation part in Figure 16 and Figure 17 adopts the ablation part in the fifteenth embodiment corresponding to Figure 15A. The ablation part includes a first conductive part 1550 and a second conductive part 1560. The first conductive part 1550 is at least part of the framework of the sealing part 1510, and the second conductive part 1560 is a wave - shaped electrode part arranged on the anchoring part 1520. It can be seen from Figure 16 that the ablation areas between the two conductive parts do not intersect and overlap, but each forms an isosurface. When the left atrial appendage occlusion and ablation device corresponding to Figure 16 is occluded at the orifice of the left atrial appendage for ablation, the ablation areas formed by the two conductive parts do not overlap, and the electric field intensity in the area between the two ablation areas is too low, which will lead to incomplete electrical isolation there. In the axial direction, the size of the first ablation area 1551 is smaller than that of the ablation area formed after the partial overlap of the two ablation areas shown in Figure 17 (the area defined by the outer contour of the dotted line in Figure 17). When the left atrial appendage occlusion and ablation device is implanted at the orifice of the left atrial appendage, due to the release angle and position of the sealing part or the anchoring part, the ablation areas formed by the sealing part and the anchoring part may not be transmural. For example, the sealing part may have a certain angle of deflection relative to the orifice, that is, there is a position where the sealing part and the circle of the orifice of the left atrial appendage are not tightly attached. The first ablation area 1551 may cover the tissue at the orifice of the left atrial appendage (between the inner wall and the outer wall), but not completely cover the outer wall of the orifice of the left atrial appendage, making it difficult to achieve transmural ablation at the orifice, thus having no good ablation effect. Or the release angle of the anchoring part inside the left atrial appendage is too deflected, resulting in partial suspension of the second conductive part 1560, failure to completely appose the wall, and poor ablation effect.

By adopting the way of partial overlapping of ablation areas shown in Figure 17, the size of the ablation area in the axial direction is larger than the axial size of any single ablation area, which is convenient to concentrate the energy of the two ablation areas to ablate a part, and improve the success rate of ablation in the area to be ablated. That is, even after the device is implanted into the orifice of the left atrial appendage, even if there are some positions in the circumferential edge area of the sealing part 1510 or the anchoring part 1520 with poor apposition, due to the ablation part having an ablation area with a larger size in the axial direction, compared with two separated ablation areas with smaller axial sizes, it is easier to form transmural ablation around the sealing part 1510.

By adopting the way of partial overlapping of ablation areas, there are proximal and distal parts in the axial direction. Even if the proximal part is difficult to cover the outer wall of the left atrial appendage due to poor apposition, the distal part may cover the outer wall of the left atrial appendage, thus reducing the impact of the apposition condition of the sealing part after release on the ablation effect and improving the success rate of ablation.

In this embodiment, the first conductive part 1550 is arranged on the sealing part 1510, and the ablation area, which are formed by the partial overlap of the first ablation area and the second ablation area, is distributed at the orifice of the left atrial appendage and the inner wall of the left atrial appendage near the orifice, so as to ablate the vicinity of the orifice of the left atrial appendage. Compared with the internal tissue of the left atrial appendage, the tissue at the orifice of the left atrial appendage has a smooth surface and regular shape, the first conductive part 1550 is easier to appose the wall, and the thickness is relatively uniform. The average current density of the second conductive part 1560 is larger than that of the first conductive part 1550, and the ablation depth is larger, so that both the first conductive part 1550 and the second conductive part 1560 can achieve good ablation effect near the orifice, and the ablation area is convenient to form continuous transmural ablation near the orifice.

Since the wall thickness of the left atrial appendage is generally 3mm, the distance between the boundary of the two overlapping ablation areas and the surface of the conductive part is large, exceeding the wall thickness of the left atrial appendage, so transmural ablation can be realized.

### Embodiment 16

Please refer to Figure 18. The ablation system of this embodiment is a left atrial appendage occlusion and ablation device 1600, which is used for occluding and ablating the left atrial appendage. The left atrial appendage occlusion and ablation device 1600 includes a sealing part 1610 arranged at the proximal end and an anchoring part 1620 arranged at the distal end, and the sealing part 1610 and the anchoring part 1620 are connected to each other.

The main difference between the left atrial appendage occlusion and ablation device 1600 provided in this embodiment and the left atrial appendage occlusion and ablation device 1500 provided in the fifteenth embodiment is that the first conductive part 1650 is an electrode part arranged on the surface of the sealing part 1610, and the form of the electrode part is a ring electrode. For the description of the specific ring electrode, please refer to the foregoing embodiment. The sealing part 1610 can be provided with at least one membrane, and the membrane can be arranged on the outer surface or inner cavity of the sealing part 1610. The membrane can be arranged between the first conductive part 1650 and the first framework 1611.

### Embodiment 17

Please refer to Figure 19. The ablation system of this embodiment is a left atrial appendage occlusion and ablation device 1700, which is used for occluding and ablating the left atrial appendage. The left atrial appendage occlusion and ablation device 1700 includes a sealing part 1710 arranged at the proximal end and an anchoring part 1720 arranged at the distal end, and the sealing part 1710 and the anchoring part 1720 are connected to each other.

The main difference between the left atrial appendage occlusion and ablation device 1700 provided in this embodiment and the left atrial appendage occlusion and ablation device 1400 provided in the fourteenth embodiment is that the first conductive part 1750 is an electrode part arranged on the surface of the first framework 1711 in the sealing part 1710. The specific form of the electrode part is a plurality of dot electrodes arranged at intervals. For the description of the specific dot electrodes, please refer to the foregoing embodiment. The second conductive part can be arranged on the second framework 1721 of the anchoring part 1720, or not arranged on the second framework 1721, and the second conductive part can be arranged on components other than the second framework 1721.

### Embodiment 18

Please refer to Figure 20. The ablation system of this embodiment is a left atrial appendage occlusion and ablation device 1800, which is used for occluding and ablating the left atrial appendage. The left atrial appendage occlusion and ablation device 1800 includes a sealing part 1810 arranged at the proximal end and an anchoring part 1820 arranged at the distal end, and the sealing part 1810 and the anchoring part 1820 are connected to each other.

The main difference between the left atrial appendage occlusion and ablation device 1800 provided in this embodiment and the left atrial appendage occlusion and ablation device 1700 provided in the seventeenth embodiment is that the second conductive part 1860 is arranged on the left atrial appendage occlusion and ablation device 1800, specifically on the second framework 1821 of the anchoring part 1820, and the second conductive part 1860 is a plurality of dot electrodes arranged at intervals on the surface of the anchoring part 1820. For the description of the specific dot electrodes, please refer to the foregoing embodiment.

### Embodiment 19

Please refer to Figure 21. The ablation system of this embodiment is a left atrial appendage occlusion and ablation device 1900, which is used for occluding and ablating the left atrial appendage. The left atrial appendage occlusion and ablation device 1900 includes a sealing part 1910 arranged at the proximal end and an anchoring part 1920 arranged at the distal end, and the sealing part 1910 and the anchoring part 1920 are connected to each other.

The main difference between the left atrial appendage occlusion and ablation device 1900 provided in this embodiment and the left atrial appendage occlusion and ablation device 1800 provided in the eighteenth embodiment is that the second conductive part 1960 arranged on the surface of the anchoring part 1920 is a wave electrode. For the structural form of the specific wave - shaped electrode, please refer to the above embodiment.

### Embodiment 20

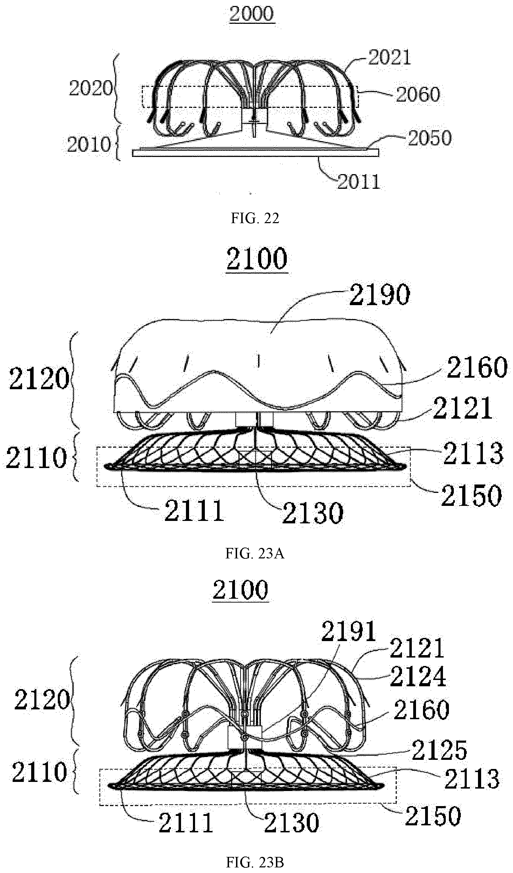

Please refer to Figure 22. The ablation system of this embodiment is a left atrial appendage occlusion and ablation device 2000, which is used for occluding and ablating the left atrial appendage. The left atrial appendage occlusion and ablation device 2000 includes a sealing part 2010 arranged at the proximal end and an anchoring part 2020 arranged at the distal end, and the sealing part 2010 and the anchoring part 2020 are connected to each other.

The main difference between the left atrial appendage occlusion and ablation device 2000 provided in this embodiment and the left atrial appendage occlusion and ablation device 1600 provided in the sixteenth embodiment is that the second conductive part 2060 is at least part of the second framework 2021 in the anchoring part 2020.

### Embodiment 21

Please refer to Figure 23A and Figure 23B. The ablation system of this embodiment includes a left atrial appendage occlusion and ablation device 2100, which is used for occluding and ablating the left atrial appendage. The left atrial appendage occlusion and ablation device 2100 includes a sealing part 2110 arranged at the proximal end and an anchoring part 2120 arranged at the distal end, and the sealing part 2110 and the anchoring part 2120 are connected to each other. Figure 23B in this embodiment is a cross - sectional view of the left atrial appendage occlusion and ablation device 2100 along the axial direction.

The main difference between the left atrial appendage occlusion and ablation device 2100 provided in this embodiment and the left atrial appendage occlusion and ablation device 1500C provided in the fifteenth embodiment is the specific structures of the sealing part 2110 and the anchoring part 2120.

Specifically, in this embodiment, from the perspective of Figure 23A and Figure 23B, the cross section of the sealing part 2110 along the axis is trapezoidal. The first framework 2111 includes a distal end disk surface facing the anchoring part 2120, a proximal end disk surface facing away from the anchoring part 2120, and a waist part 2113 connected between the distal end disk surface and the proximal end disk surface. The proximal end disk surface and the distal end disk surface are roughly planar, the waist part is connected between the proximal end disk surface and the distal end disk surface, and the waist part is in the shape of a conical cylinder.

In this embodiment, the first conductive part 2150 is at least part of the first framework 2111, and the first conductive part 2150 is at least arranged in the edge area with a larger radial size of the sealing part 2110, such as the waist part 2113, preferably, arranged in the part with the largest radial size in the circumferential direction of the sealing part 2110. As shown in Figure 23A and Figure 23B, the first conductive part 2150 can also be arranged on the waist part 2113 and the proximal end disk surface (the part in the dotted line frame in the figure). In some embodiments, the first conductive part 2150 is arranged on the waist part 2113, at least part of the proximal end disk surface and at least part of the distal end disk surface. The surface of the part of the first framework 2111 not used as the first conductive part 2150 is subjected to insulation treatment, and the insulation treatment can be at least one of the ways of arranging a membrane, sleeving an insulating sleeve, plating an insulating coating, making of an insulating material, etc. Preferably, at least part of the distal end disk surface of the sealing part 2110 is subjected to insulation treatment, so as to avoid short circuit between the first conductive part 2150 and the anchoring part 2120 and improve the insulation performance between the first conductive part 2150 and the second conductive part 2160.

The connecting piece 2130 at the proximal end of the sealing part 2110 can be used for mechanical connection with the delivery device and electrical connection, so that the first conductive part 2150 is electrically connected to an external pulse signal source through the connecting piece 2130.

The second conductive part 2160 is arranged on the anchoring part 2120, specifically a wave electrode. A membrane 2190 is arranged between the second framework 2121 and the second conductive part 2160. In a modified embodiment, an insulating coating can be plated on the surface of the second framework 2121, or an insulating sleeve can be sleeved for insulation. In some embodiments, at least two same or different insulation modes are adopted between the second framework 2121 and the second conductive part 2160 for insulation.

A connecting piece 2191 is connected between the sealing part 2110 and the anchoring part 2120. In some embodiments, at least part of the connecting piece 2191 is made of insulating materials for insulated connection between the second conductive part 2160 and the delivery device. Preferably, the connection piece 2191 is at least partially made of a conductive material and is used for electrically connecting between the second conductive part 2160 and the delivery device.

The main difference between the anchoring part 2120 and the anchoring part 1520 is that the ends of two adjacent fourth anchoring rods 2125 in the anchoring part 2120 far away from the anchoring rods 2124 are connected together, which improves the mechanical performance of the anchoring part 2120, avoids mutual hooking of the fourth anchoring rods 2125 during radial deformation of the left atrial appendage occlusion and ablation device 2100, and improves the reliability of the left atrial appendage occlusion and ablation device 2100.

In the process of ablation using the left atrial appendage occlusion and ablation device 2100 provided in this embodiment, it can be used in cooperation with a mapping catheter. It can be understood that in some embodiments, the mapping catheter can collect electrophysiological signals at the target tissue and also release ablation energy to the target tissue.

### Embodiment 22

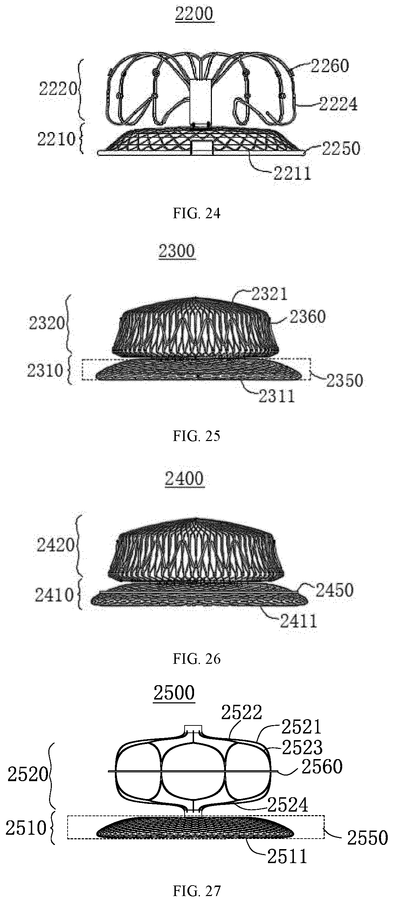

Please refer to Figure 24. The ablation system of this embodiment includes a left atrial appendage occlusion and ablation device 2200, which is used for occluding and ablating the left atrial appendage. The left atrial appendage occlusion and ablation device 2200 includes a sealing part 2210 arranged at the proximal end and an anchoring part 2220 arranged at the distal end, and the sealing part 2210 and the anchoring part 2220 are connected to each other.

The main difference between the left atrial appendage occlusion and ablation device 2200 provided in this embodiment and the left atrial appendage occlusion and ablation device 2100 provided in the twenty - first embodiment is that the first conductive part 2250 is an electrode part arranged at the position with the largest radial size of the sealing part 2110, and the form of the electrode part is a ring electrode. The second conductive part 2260 is an electrode part arranged on the surface of the anchoring part 2220, and the specific form of the electrode part is a plurality of dot electrodes arranged at intervals. In this embodiment, two dot electrodes are arranged at intervals on each third anchoring rod 2224. In other embodiments, the number of dot electrodes arranged on each third anchoring rod 2224 can be set as required, and the surface of some third anchoring rods 2224 may not be provided with dot electrodes. It can be understood that the left atrial appendage occlusion and ablation device 2200 can be provided with a membrane as required.

### Embodiment 23

Please refer to Figure 25. The ablation system of this embodiment includes a left atrial appendage occlusion and ablation device 2300, which is used for occluding and ablating the left atrial appendage. The left atrial appendage occlusion and ablation device 2300 includes a sealing part 2310 arranged at the proximal end and an anchoring part 2320 arranged at the distal end, and the sealing part 2310 and the anchoring part 2320 are connected to each other.

The main difference between the left atrial appendage occlusion and ablation device 2300 provided in this embodiment and the left atrial appendage occlusion and ablation device 2100 provided in the twenty - first embodiment is that the specific structures of the sealing part 2310 and the anchoring part 2320 are different from those in the twenty - first embodiment.

Specifically, in this embodiment, both the sealing part 2310 and the anchoring part 2320 are made by braiding.

The first framework 2311 in the sealing part 2310 has a double - layer disk surface structure, including a distal end disk surface facing the anchoring part 2320 and a proximal end disk surface facing away from the anchoring part 2320. The proximal end disk surface is roughly planar, the distal end disk surface is partially spherical, and the proximal end disk surface and the distal end disk surface are smoothly transitionally connected. The first conductive part 2350 is at least part of the first framework 2311, arranged in the edge area with a larger radial size in the sealing part 2310.

The second framework 2321 in the anchoring part 2320 has a double - layer disk structure, roughly cylindrical, and the radial size of the distal side of the side wall of the second framework 2321 is smaller than that of the proximal side. The second conductive part 2360 is an electrode part arranged on the anchoring part 2320, and the form of the electrode part is a wave electrode.

### Embodiment 24

Please refer to Figure 26. The ablation system of this embodiment includes a left atrial appendage occlusion and ablation device 2400, which is used for occluding and ablating the left atrial appendage. The left atrial appendage occlusion and ablation device 2400 includes a sealing part 2410 arranged at the proximal end and an anchoring part 2420 arranged at the distal end, and the sealing part 2410 and the anchoring part 2420 are connected to each other.

The main difference between the left atrial appendage occlusion and ablation device 2400 provided in this embodiment and the left atrial appendage occlusion and ablation device 2300 provided in the twenty - third embodiment is that the first conductive part 2450 is an electrode part arranged on the surface of the first framework 2411 in the sealing part 2410, and the specific form of the electrode is a ring electrode. For the description of the specific ring electrode, please refer to the above embodiment.

### Embodiment 25

Please refer to Figure 27. The ablation system of this embodiment includes a left atrial appendage occlusion and ablation device 2500, which is used for occluding and ablating the left atrial appendage. The left atrial appendage occlusion and ablation device 2500 includes a sealing part 2510 arranged at the proximal end and an anchoring part 2520 arranged at the distal end, and the sealing part 2510 and the anchoring part 2520 are connected to each other.

The main difference between the left atrial appendage occlusion and ablation device 2500 provided in this embodiment and the left atrial appendage occlusion and ablation device 2300 provided in the twenty - third embodiment is that the structure of the anchoring part 2520 and the structure of the second conductive part 2560 are different from those in the twenty - third embodiment.

Specifically, the anchoring part 2520 is manufactured by a cutting process. The anchoring part 2520 has a double - layer disk structure, and the proximal end and the distal end are closed. The second framework 2521 includes a first anchoring rod 2522, a second anchoring rod 2523 and a third anchoring rod 2524 which are sequentially connected from the distal end to the proximal end.

The first anchoring rod 2522, the second anchoring rod 2523 and the third anchoring rod 2524 all extend between the proximal end and the distal end. The proximal end of each first anchoring rod 2522 is connected with two different second anchoring rods 2523. The two second anchoring rods 2523 connected to the same first anchoring rod 2522 extend in different directions. Among the four second anchoring rods 2523 connected to two adjacent first anchoring rods 2522, the middle sections of two adjacent second anchoring rods 2523 connected to different first anchoring rods 2522 are close to each other and connected. The proximal ends of the two second anchoring rods 2523 connected to the same first anchoring rod 2522 are connected to each other and connected to the distal end of the third anchoring rod 2524. The proximal ends of the plurality of third anchoring rods 2524 are combined together, thus forming a closed structure at both ends of the anchoring part 120.

The plurality of second anchoring rods 2523 form a plurality of mesh holes in the anchoring part 2520, which is convenient for improving the radial deformation ability of the anchoring part 2520 and the friction force with the tissue.

The second conductive part 2560 is an electrode part arranged on the surface of the second framework 2521 in the anchoring part 2520, and the specific form of the electrode is a ring electrode. For the description of the specific ring electrode, please refer to the above embodiment.

### Embodiment 26

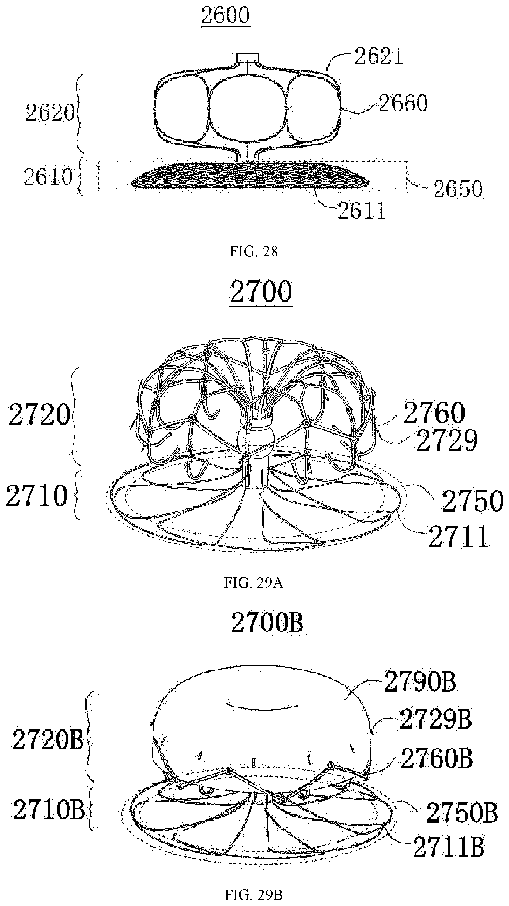

Please refer to Figure 28. The ablation system of this embodiment includes a left atrial appendage occlusion and ablation device 2600, which is used for occluding and ablating the left atrial appendage. The left atrial appendage occlusion and ablation device 2600 includes a sealing part 2610 arranged at the proximal end and an anchoring part 2620 arranged at the distal end, and the sealing part 2610 and the anchoring part 2620 are connected to each other.

The main difference between the left atrial appendage occlusion and ablation device 2600 provided in this embodiment and the left atrial appendage occlusion and ablation device 2500 provided in the twenty - fifth embodiment is that the structure of the second conductive part 2660 is different from that in the twenty - fifth embodiment.

In this embodiment, the second conductive part 2660 is a plurality of dot electrodes arranged at intervals on the surface of the second framework 2621 in the anchoring part 2620. For the description of the specific dot electrodes, please refer to the above embodiment.

### Embodiment 27

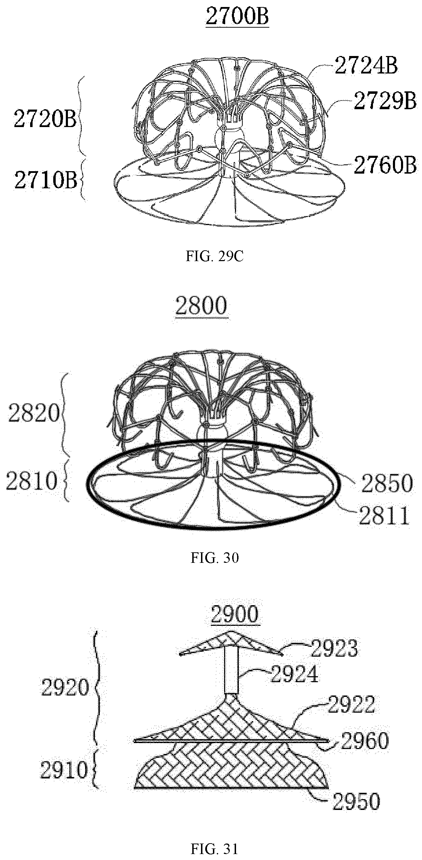

Please refer to Figure 29A. The ablation system of this embodiment includes a left atrial appendage occlusion and ablation device 2700, which is used for occluding and ablating the left atrial appendage. The left atrial appendage occlusion and ablation device 2700 includes a sealing part 2710 arranged at the proximal end and an anchoring part 2720 arranged at the distal end, and the sealing part 2710 and the anchoring part 2720 are connected to each other.

The main difference between the left atrial appendage occlusion and ablation device 2700 provided in this embodiment and the left atrial appendage occlusion and ablation device 1500 provided in the fifteenth embodiment is that the structure of the sealing part 2710 and the structure of the first conductive part 2750 are different from those in the fifteenth embodiment.

Specifically, the first framework 2711 in the sealing part 2710 includes a plurality of first sealing rods, and both ends of each first sealing rod are arranged at the central axis of the sealing part 2710. When projected on a plane perpendicular to the central axis, that is, viewed from the distal end to the proximal end or from the proximal end to the distal end, each first sealing rod forms a roughly annular support ring, which is fan - shaped in this embodiment. In other embodiments, the ring can be circular, elliptical, partial ring, or other irregular rings. A plurality of support rings formed by a plurality of first sealing rods are arranged in the circumferential direction to obtain the first framework 2711.

As shown in Figure 29A, adjacent support rings overlap each other in the circumferential direction. When projected on a plane perpendicular to the central axis, that is, viewed from the distal end to the proximal end or from the proximal end to the distal end, the central angles occupied by adjacent support rings overlap. In other embodiments, the central angles occupied by adjacent support rings do not overlap in this plane. In some embodiments, the central angles of adjacent support rings are also arranged at intervals.

As shown in Figure 29A, the positions of both ends of each first sealing rod in the axial direction are different, that is, both ends of each first sealing rod are staggered in the axial direction. In other embodiments, the positions of both ends of each first sealing rod in the axial direction are the same.

The first conductive part 2750 is all or part of the framework of the first framework 2711 in the sealing part 2710. Specifically, the first conductive part 2750 is the part with a larger radial size in the first framework 2711, including the first framework 2711 at the position with the largest radial size. In this embodiment, the part between the two dotted rings in Figure 29A is the periphery of the sealing part 2710, and the first conductive part 2750 is arranged between the two dotted rings in Figure 29A. In some embodiments, the first conductive part 2750 is arranged at the position with the largest radial size in the first framework 2711, that is, a circle of framework surrounding the circumferential edge of the first framework 2711.

The second conductive part 2760 is a wave electrode arranged on the surface of the anchoring part 2720, and both the wave troughs and wave crests of the second conductive part 2760 are arranged corresponding to the third anchoring rods of the anchoring part 2720.

Preferably, there is an insulation design between the second conductive part 2760 and the second framework of the anchoring part 2720, for example, a membrane is arranged between the second conductive part 2760 and the second framework of the anchoring part 2720, and an insulating coating can be plated on the surface of the second framework, or an insulating sleeve can be sleeved, etc.

Anchor thorns 2729 are arranged on the surface of the anchoring part 2720, and the anchor thorns 2729 are arranged on the proximal side of the second conductive part 2760.

Please refer to Figure 29B to Figure 29C. The left atrial appendage occlusion and ablation device 2700B provided in Figure 29B to Figure 29C is a modified embodiment based on the left atrial appendage occlusion and ablation device 2700 in Figure 29A. A membrane 2790B is arranged between the second conductive part 2760B and the second framework of the anchoring part 2720B in the left atrial appendage occlusion and ablation device 2700B. It can be understood that at least one of the following insulation designs can be adopted between the second conductive part 2760B and the second framework of the anchoring part 2720B, for example, a membrane 2790B is arranged between the second conductive part 2760B and the second framework of the anchoring part 2720B, an insulating coating can be plated on the surface of the second framework, an insulating sleeve can be sleeved, or the anchoring part 2720B can be made of an insulating material, etc.

The difference between this embodiment and the twenty - seventh embodiment is that the second conductive part 2760B is a wave electrode arranged on the surface of the anchoring part 2720B, and both the wave troughs and wave crests of the second conductive part 2760B are arranged staggered from the third anchoring rods 2724B of the anchoring part 2720B, that is, both the wave crests and wave troughs of the second conductive part 2760B are arranged at positions between two adjacent third anchoring rods 2724B. The second conductive part 2760B can be fixed to the membrane 2791 through the wave crests and wave troughs. In some embodiments, the second conductive part 2760B can be fixed to the membrane 2791B through the parts between the wave crests and wave troughs, and can be further fixed to the third anchoring rods 2724B.

Anchor thorns 2129B are arranged on the surface of the anchoring part 2720B, and the anchor thorns 2129B are arranged on the distal side of the second conductive part 2760B, thus being beneficial to reducing the risk of sparking of the anchor thorns 2129B during ablation. Specifically, at least one anchor thorn 2129B is arranged on the outer side of each third anchoring rod 2724B.

### Embodiment 28

Please refer to Figure 30. The ablation system of this embodiment includes a left atrial appendage occlusion and ablation device 2800, which is used for occluding and ablating the left atrial appendage. The left atrial appendage occlusion and ablation device 2800 includes a sealing part 2810 arranged at the proximal end and an anchoring part 2820 arranged at the distal end, and the sealing part 2810 and the anchoring part 2820 are connected to each other.

The main difference between the left atrial appendage occlusion and ablation device 2800 provided in this embodiment and the left atrial appendage occlusion and ablation device 2700 provided in the twenty - seventh embodiment is that the structure of the first conductive part 2850 is different from that in the twenty - seventh embodiment.

In this embodiment, the first conductive part 2850 is an electrode part arranged at the position with the largest radial size in the circumferential direction of the sealing part 2810, and the form of the electrode part is a ring electrode. For the description of the specific ring electrode, please refer to the foregoing embodiment.

### Embodiment 29

Please refer to Figure 31. The ablation system of this embodiment includes a left atrial appendage occlusion and ablation device 2900, which is used for occluding and ablating the left atrial appendage. The left atrial appendage occlusion and ablation device 2900 includes a sealing part 2910 arranged at the proximal end and an anchoring part 2920 arranged at the distal end, and the sealing part 2910 and the anchoring part 2920 are connected to each other.

The main difference between the left atrial appendage occlusion and ablation device 2900 provided in this embodiment and the left atrial appendage occlusion and ablation device provided in the foregoing embodiment is that the anchoring part 2920 has a double - disk structure.

Specifically, both the sealing part 2910 and the anchoring part 2920 can be woven from braided wires.

The sealing part 2910 is cylindrical or conical, and its radial size is larger than the opening of the left atrial appendage. As shown in the figure, the first conductive part 2950 is a ring electrode, arranged at the position with the largest radial size of the sealing part 2910. In some other embodiments, the first conductive part 2950 can also be arranged at other positions in the edge area.

The anchoring part 2920 includes a first anchoring part 2922 and a second anchoring part 2922 which are arranged at intervals in the axial direction. Both the first anchoring part 2922 and the second anchoring part 2922 are woven from braided wires, and are cylindrical or conical. A connecting piece 2924 is arranged between the first anchoring part 2922 and the second anchoring part 2922. In some embodiments, the connecting piece 2924 is flexible and/or elastic.

The second conductive part 2960 is an electrode part arranged at the position with a larger radial size of the first anchoring part 2922, and the specific electrode part is a ring electrode. For the description of the specific ring electrode, please refer to the foregoing embodiment.

In some embodiments, the second conductive part 2960 can be arranged on the second anchoring part 2922, or at the position with a larger radial size of the sealing part 2910, or on the connecting piece 2924.

### Embodiment 30

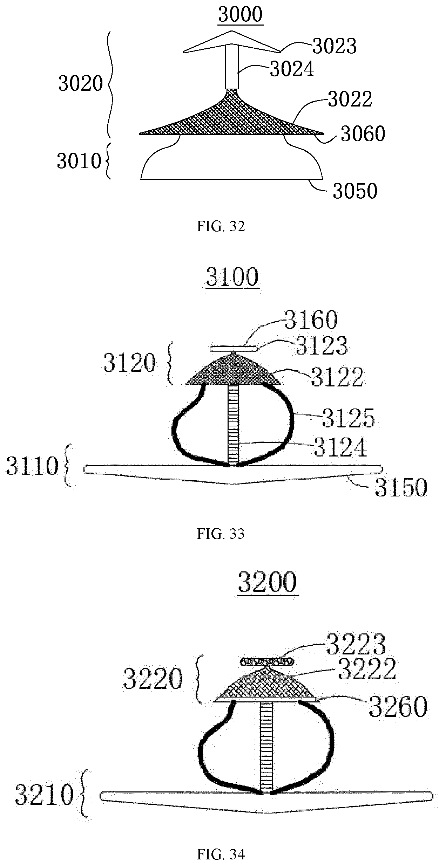

Please refer to Figure 32. The ablation system of this embodiment is a left atrial appendage occlusion and ablation device 3000, which is used for occluding and ablating the left atrial appendage. The left atrial appendage occlusion and ablation device 3000 includes a sealing part 3010 arranged at the proximal end and an anchoring part 3020 arranged at the distal end, and the sealing part 3010 and the anchoring part 3020 are connected to each other.

The main difference between the left atrial appendage occlusion and ablation device 3000 provided in this embodiment and the left atrial appendage occlusion and ablation device provided in the twenty - ninth embodiment is the structures of the first conductive part 3050 and the second conductive part 3060.

In this embodiment, the first conductive part 3050 is at least part of the first framework in the sealing part 3010, preferably the part with a larger radial size, preferably including the part with the largest radial size.

The second conductive part 3060 is at least part of the framework in the anchoring part 3020, preferably the part with a larger radial size of the first anchoring part 3022, preferably including the part with the largest radial size of the first anchoring part 3022. The second conductive part 3060 can also be arranged on the second anchoring part 3023, or on the sealing part 3010, or on the connecting piece 3024.

### Embodiment 31

Please refer to Figure 33. The ablation system of this embodiment is a left atrial appendage occlusion and ablation device 3100, which is used for occluding and ablating the left atrial appendage. The left atrial appendage occlusion and ablation device 3100 includes a sealing part 3110 arranged at the proximal end and an anchoring part 3120 arranged at the distal end, and the sealing part 3110 and the anchoring part 3120 are connected to each other.

The main difference between the left atrial appendage occlusion and ablation device 3100 provided in this embodiment and the left atrial appendage occlusion and ablation device provided in the thirtieth embodiment is the structures of the sealing part 3110 and the anchoring part 3120, and the structures of the first conductive part 3150 and the second conductive part 3160.

Both the sealing part 3110 and the anchoring part 3120 can be made by braiding. The sealing part 3110 has a double - layer disk shape, and the diameter of the sealing part 3110 is larger than that of the orifice of the left atrial appendage.

The anchoring part 3120 includes a first anchoring part 3122 and a second anchoring part 3123. Both the first anchoring part 3122 and the second anchoring part 3123 can be in the shape of a single - layer disk or a multi - layer disk, and the first anchoring part 3122 is arranged on the proximal side of the second anchoring part 3123.

A connecting piece 3124 and a connecting piece 3125 are connected between the sealing part 3110 and the first anchoring part 3122. The connecting piece 3124 and the connecting piece 3125 extend between the proximal end and the distal end. The connecting piece 3124 is arranged along the axial direction of the left atrial appendage occlusion and ablation device 3100. The number of the connecting pieces 3125 is at least one, arranged around the connecting piece 3124 in the circumferential direction.

The connecting piece 3124 can be a spiral structure, and the connecting piece 3124 can have a variable length, which enables the left atrial appendage occlusion and ablation device 3100 to be used for left atrial appendages with different shapes and sizes. The connecting piece 3124 can be bent, thus allowing the left atrial appendage occlusion and ablation device 3100 to have different angles and directions, which also enables the left atrial appendage occlusion and ablation device 3100 to be used for left atrial appendages with different shapes and sizes. The connecting piece 3124 has a substantially constant or unchanged cross section.

The connecting piece 3125 includes a substantially inelastic or completely inelastic material, so that the connecting piece 3125 prevents the connecting piece 3124 from extending beyond a predetermined length. Since the connecting piece 3125 prevents the connecting piece 3124 from extending beyond the predetermined length, the connecting piece 3125 prevents the connecting piece 3124 from being overstretched during loading the left atrial appendage occlusion and ablation device 3100 into the left atrial appendage or removing the left atrial appendage occlusion and ablation device 3100 from the left atrial appendage. Two connecting pieces 3125 or more connecting pieces 3125 can be arranged around the connecting piece 3124.

The first conductive part 3150 is at least part of the first framework in the sealing part 3110, preferably the part with a larger radial size, preferably including the part with the largest radial size.

The second conductive part 3160 is at least part of the second framework in the second anchoring part 3123, preferably the part with a larger radial size of the second anchoring part 3123, preferably including the part with the largest radial size of the second anchoring part 3123.

In some embodiments, the second conductive part 3160 is at least part of the second framework in the first anchoring part 3122, or arranged on the connecting piece 3124 and/or the connecting piece 3125.

The connecting piece 3124 and the connecting piece 3125 can be used as conductive connecting pieces for transmitting electric energy.

### Embodiment 32

Please refer to Figure 34. The ablation system of this embodiment is a left atrial appendage occlusion and ablation device 3200, which is used for occluding and ablating the left atrial appendage. The left atrial appendage occlusion and ablation device 3200 includes a sealing part 3210 arranged at the proximal end and an anchoring part 3220 arranged at the distal end, and the sealing part 3210 and the anchoring part 3220 are connected to each other.

The main difference between the left atrial appendage occlusion and ablation device 3200 provided in this embodiment and the left atrial appendage occlusion and ablation device 3100 provided in the thirty - first embodiment is the structure of the second conductive part 3260.

In this embodiment, the second conductive part 3260 is an electrode part arranged on the surface of the first anchoring part 3222, and the form of the electrode part is a ring electrode. The second conductive part 3260 is preferably arranged at the part with a larger radial size of the first anchoring part 3222, preferably at the part with the largest radial size of the first anchoring part 3222. In some embodiments, the second conductive part 3260 is an electrode part arranged on the surface of the second anchoring part 3222, preferably arranged at the part with a larger radial size of the anchoring part 3223.

### Embodiment 33

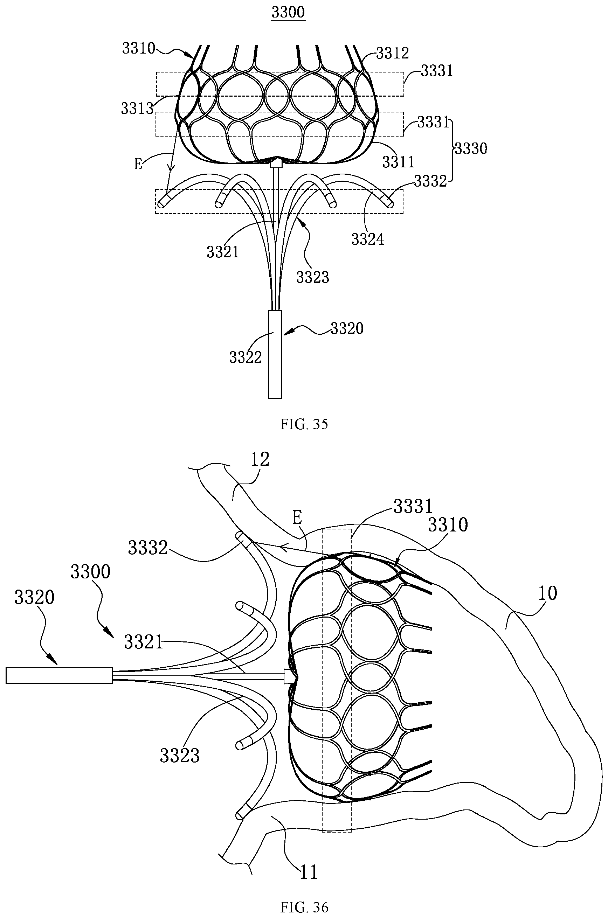

Figure 35 is a schematic structural diagram of the ablation system 3300 provided in the thirty - third embodiment, and Figure 36 is a schematic structural diagram of the ablation system 3300 implanted into the left atrial appendage. With reference to Figure 35 and Figure 36, the ablation system 3300 includes a support body 3310, a delivery device 3320 and an ablation part 3330. The support body 3310 is used for being implanted into target tissues, such as the opening of the left atrial appendage and other positions requiring ablation operations. The delivery device 3320 is used for delivering the support body 3310 to target tissues, such as the opening of the left atrial appendage or other target tissues, and the target tissues include but are not limited to myocardial tissues. The ablation part 3330 is used for electrically ablating the wall of the target tissue. The tissue ablated by the ablation part 3330 can be different from the tissue position where the support body 3310 is released.

The support body 3310 adopts a single - disk structure and is cylindrical as a whole. The support body 3310 is a hollow grid structure made by braiding and/or cutting processes. The support body 3310 includes a sealing part 3311 and an anchoring part 3312 which are connected. After the support body 3310 is released into the left atrial appendage 10, the sealing part 3311 is located on the distal side of the orifice of the left atrial appendage. An opening is formed on the distal side of the anchoring part 3312.

Anchor thorns 3313 are arranged on the outer wall surface of the anchoring part 3312, and the anchor thorns 3313 are used for anchoring on the inner wall of the left atrial appendage. A plurality of anchor thorns 3313 are uniformly arranged along the circumference of the outer wall surface of the anchoring part 3312, and different arrangement modes can be adopted according to the structure of the anchor thorns 3313. The anchor thorns 3313 can be directly fixed on the anchoring part 3312, and specifically can be fixed by welding. The anchor thorns 3313 can also be fixedly connected with the anchoring part 3312 through a steel sleeve. The anchor thorns 3313 and the anchoring part 3312 can also be of an integral structure, that is, the anchor thorns 3313 are directly extended from the anchoring part 3312.

The ablation part 3330 includes a first conductive part 3331 and a second conductive part 3332. The first conductive part 3331 and the second conductive part 3332 are used for transmitting pulse ablation energy with different polarities to the target tissue to realize tissue ablation. In this embodiment, the first conductive part 3331 is arranged on the support body 3310, the first conductive part 3331 is electrically connected to an external pulse ablation source through the delivery device 3320, the second conductive part 3332 is arranged on the delivery device 3320, and the second conductive part 3332 can move relative to the support body 3310 and is used for being released on the proximal side of the support body 3310. Specifically, the side wall of the support body 3310 is arc - shaped, and the position with the largest radial size of the support body 3310 is used for radially pushing against the inner wall tissue of the left atrial appendage, with good wall - apposition. A first conductive part 3331 is arranged at the position with the largest radial size of the support body 3310, which is convenient for the first conductive part 3331 to appose the wall and ensures the ablation effect. The position with the largest radial size of the support body 3310 is located between its proximal end and distal end and close to the proximal end. After the support body 3310 is implanted into the left atrial appendage 10, the first conductive part 3331 is located inside the left atrial appendage 10. Since the support body 3310 adopts a single - disk structure and the position with the largest radial size of the support body 3310 is located between the proximal end and the distal end of the support body 3310, it is convenient for the support body 3310 to be implanted and fixed in the inner cavity of the left atrial appendage. In a modified embodiment, the radial sizes of the side walls of the support body at different axial positions are the same.

In some embodiments, at least part of the first conductive part 3331 and/or the second conductive part 3332 can be used for collecting tissue physiological signals, that is, the ablation part 3330 has ablation and mapping functions. In some embodiments, some components in the first conductive part 3331 can only be used for mapping and have no ablation effect. In some embodiments, some components in the first conductive part 3331 are used for mapping, and some components have ablation effects. In some embodiments, at least part of the components of the first conductive part 3331 are used for mapping and ablation at different times. In some embodiments, some components in the second conductive part 3332 can only be used for mapping and have no ablation effect. In some embodiments, some components of the second conductive part 3332 are used for mapping, and some components have ablation effects. In some embodiments, at least part of the components of the second conductive part 3332 are used for mapping and ablation at different times.

The delivery device 3320 includes an inner sheath tube 3321 and an ablation catheter 3323. The inner sheath tube 3321 is connected with the support body 3310. The delivery device 3320 includes an outer sheath tube 3322 sleeved outside the inner sheath tube 3321. The outer sheath tube 3322 and the inner sheath tube 3321 can move relative to each other. The distal end of the inner sheath tube 3321 is connected with the support body 3310, and the proximal end of the ablation catheter 3323 is connected with the distal end of the outer sheath tube 3322. After the ablation is completed, the ablation catheter 3323 can be recovered into an outer catheter (not shown) sleeved outside the outer sheath tube 3322. The ablation catheter 3323 is used for being released on the proximal side of the sealing part 3311. A second conductive part 3332 is arranged on the ablation catheter 3323. Specifically, the ablation catheter 3323 includes a plurality of support rods 3324 arranged at the distal end of the outer sheath tube. The plurality of support rods 3324 are umbrella - shaped after being released at the proximal end of the support body 3310, forming an umbrella - shaped frame body.

Specifically, the ablation catheter 3323 includes a plurality of support rods 3324 radially arranged around its axis. The proximal ends of the plurality of support rods 3324 are combined at the distal end of the outer sheath tube 3322. After the support rods 3324 are released, each support rod 3324 is in an arc shape protruding towards the support body 3310. One end of each support rod 3324 far away from its proximal end is a free end, and the free end is used for extending to the proximal end. The second conductive part 3332 is arranged on the support rod 3324.

The second conductive part 3332 can be a part or all of the support rod 3324, or an electrode part arranged on the support rod 3324. In this embodiment, an electrode part is arranged at the circumferential end of each support rod 3324, and the radial size of the second conductive part 3332 is larger than that of the first conductive part 3331, which is convenient for the characteristic electric field line E to pass through the orifice tissue. It can be understood that the number of electrode parts on the support rod 3324 can be adjusted as required, and electrode parts may not be arranged on some support rods 3324.

Since the second conductive part 3332 is arranged in the circumferential edge area with a larger radial size of the ablation catheter, it is convenient for the second conductive part 3332 to be easily apposed to the tissue on the proximal side of the orifice of the left atrial appendage after release. The position with the largest radial size of the support body 3310 is located between its proximal end and distal end, so that when the support body 3310 is implanted into the left atrial appendage, the sealing part 3311 of the support body 3310 is used for being implanted on the distal side of the orifice of the left atrial appendage, and the sealing part 3311 of the support body 3310 will not cover the orifice of the left atrial appendage, avoiding mutual interference between the support body 3310 and the umbrella - shaped frame body. In the natural state (not subject to external force) or during ablation, the radial size of the annular structure formed by the second conductive part 3332 is larger than that of the annular structure formed by the first conductive part 3331, that is, among the first conductive part 3331 and the second conductive part 3332, the radial size of the annular structure formed by the conductive part located on the proximal side is larger than that of the annular structure formed by the conductive part located on the distal side, so that the distal side of at least one characteristic electric field line E is inclined towards the axis of the support body, facilitating the characteristic electric field line between the first conductive part 3331 and the second conductive part 3332 to pass through the left atrial appendage tissue outside the accommodating part, and ensuring the ablation effect. In this embodiment, the accommodating part is the part where the support body 3310 and the support rod 3324 face and are close to each other.

It can be understood that the first conductive part 3331 and the second conductive part 3332 can be various forms of electrodes arranged on the support body 3310 or the support rod 3324, such as at least one of dot electrodes, rod electrodes, arc electrodes, ring electrodes and wave electrodes. The first conductive part 3331 can also be at least part of the support body 3310, and the second conductive part 3332 can be at least part of the support rod 3324, for example, the support body 3310 is a hollow grid structure made of conductive metal materials by braiding and/or cutting processes. The first conductive part 3331 is at least part of the support body 3310, or all of the support body 3310. In some embodiments, the support rod 3324 of the ablation catheter 3323 is made of conductive metal materials by braiding and/or cutting processes; The second conductive part 3332 is at least part of the support rod 3324, or all of the support rod 3324.

The number of the first conductive part 3331 and the second conductive part 3332 can be multiple. As shown in Figure 33, a plurality of first conductive parts 3331 are arranged on the support body 3310, specifically two, which are respectively arranged on the sealing part 3311 and the anchoring part 3312. Preferably, the two first conductive parts 3331 are used for transmitting pulse ablation energy with opposite polarities and forming an ablation loop. It can be understood that a plurality of first conductive parts 3331 can be arranged on the sealing part 3311 or the anchoring part 3312. In the embodiment where the first conductive part 3331 includes a plurality of electrodes, the plurality of electrodes in the first conductive part 3331 can be arranged at intervals or in contact with each other. The plurality of electrodes in the second conductive part 3332 can be arranged at intervals or connected to each other. In this embodiment, the second conductive part 3332 includes a plurality of electrodes. In other embodiments, a plurality of second conductive parts 3332 can be arranged, and each second conductive part 3332 includes at least one electrode or at least one section of support rod. In some embodiments, all the second conductive parts 3332 are used for mapping.

Compared with the prior art, the ablation system 3300 provided by this application can realize pulse ablation of target tissues through the cooperation between the first conductive part 3331 and the second conductive part 3332.

The following description takes the ablation system 3300 as a left atrial appendage occlusion and ablation system, that is, it is used for occluding and ablating the left atrial appendage 10.

The ablation system includes a left atrial appendage occlusion and ablation device and a delivery device 3320. The left atrial appendage occlusion and ablation device includes a support body 3310 and a first conductive part 3331 in the ablation part 3330. The support body 3310 is connected to the delivery device 3320, and the first conductive part 3331 is connected to a pulse ablation instrument through the delivery device 3320. In some embodiments, the first conductive part 3331 is connected to the pulse ablation instrument through a cable via the delivery device 3320. In some embodiments, a membrane is arranged on the support body 3310, and the membrane is used for blocking thrombus in the left atrial appendage from flowing out to the left atrium.

In some embodiments, both the first conductive part 3331 and the second conductive part 3332 are arranged on the support body 3310, and the two conductive parts are used for transmitting ablation electric energy with the same or different parameters, such as ablation electric energy with different polarities. In this embodiment, the first conductive part is arranged on the support body 3310, and the second conductive part is arranged on the delivery device 3320, such as the ablation catheter of the delivery device 3320 at the proximal end of the support body 3310. In other embodiments, the ablation catheter provided with the second conductive part is used for release on the distal side of the support body 3310. In some embodiments, the second conductive part is independently arranged with the support body 3310 and the delivery device 3320, and is used for being attached to the surface of the organism during ablation.

In this embodiment, the first conductive part 3331 and the second conductive part 3332 meet the parameters provided in the foregoing embodiment, for example, during ablation, the electric field intensity generated at a position 3mm away from the surfaces of the first conductive part 3331 and the second conductive part 3332 is greater than 400V/cm. In some embodiments, the ablation system 3300 is used to ablate cardiac tissues other than the left atrial appendage 10, such as the pulmonary vein with a thickness of generally 2mm. It is ensured that during ablation, the electric field intensity generated at a position 2mm away from the surface of the ablation part 3330 is greater than the myocardial threshold field intensity, such as greater than 400V/cm, so as to facilitate transmural ablation.

In the embodiment where the ablation part 3330 is arranged on the support body 3310 and/or the delivery device, the pulse voltage range adopted by the ablation part 3330 (the first conductive part and the second conductive part) is 500V ~ 4000V, the discharge area of the first conductive part 3331 is 15mm² ~ 4700mm², and the discharge area of the second conductive part 3332 is 15mm² ~ 4700mm². When the conductive part has multiple electrode parts, the value range of the discharge area refers to the sum of all discharge areas (electrode parts or frameworks) of the conductive part. The distance between the nearest two points on the surfaces of the first conductive part 3331 and the second conductive part 3332 ranges from 1mm to 45mm. Preferably, the distance between the first conductive part 3331 and the second conductive part 3332 can be further limited to 3mm ~ 22mm. Within this range, it is convenient for the ablation areas of the two conductive parts to partially overlap. The ablation system is used for ablating myocardial tissue. The area around the first conductive part 3331 where the electric field intensity is greater than the myocardial threshold intensity is the first ablation area, and the area around the second conductive part 3332 where the electric field intensity is greater than the myocardial threshold intensity is the second ablation area. The size of the ablation area formed after the partial overlap of the two ablation areas in the axial direction is large.

Two first conductive parts 3331 are arranged on the support body 3310 in Figure 35. In some embodiments, one of them can be selected for use according to actual application requirements.

Under the conditions that the frequency range is 500Hz ~ 3MHz and the voltage range is 500V ~ 4000V, when the ablation area corresponds to the electric field distribution area with an electric field intensity of 400V/cm or more, the ablation area is arranged around the surface of the conductive part. The ablation area formed after the partial overlap of the two ablation areas in the axial direction has a larger size in the axial direction than any single ablation area, which is convenient to concentrate the energy of the two ablation areas to ablate a part, and improve the success rate of ablation in the area to be ablated. That is, even after the device is implanted into the orifice 11 of the left atrial appendage, even if there are some positions in the circumferential edge area of the sealing part 3311 with poor apposition, due to the ablation part 3330 having an ablation area with a larger size in the axial direction, compared with two separated ablation areas with smaller axial sizes, it is easier to form transmural ablation around the sealing part 3311.

By adopting the way of partial overlapping of ablation areas, there are proximal and distal parts in the axial direction. Even if the proximal part is difficult to cover the outer wall of the left atrial appendage due to poor apposition, the distal part may cover the outer wall of the left atrial appendage, thus reducing the impact of the apposition condition of the sealing part 3311 after release on the ablation effect of the occlusion and ablation system 3300 and improving the success rate of system ablation.

In this embodiment, the first conductive part 3331 is arranged on the sealing part 3311, and the ablation area formed after the partial overlapping of the first ablation area and the second ablation area is distributed at the orifice 11 of the left atrial appendage, so as to ablate the orifice 11 of the left atrial appendage. Compared with the internal tissue of the left atrial appendage, the tissue at the orifice of the left atrial appendage has a smooth surface and regular shape, the first conductive part 3331 is easier to appose the wall, and the thickness is relatively uniform, so the ablation area is convenient to form continuous transmural ablation at the orifice.

Since the wall thickness of the left atrial appendage is generally 3mm, the distance between the boundary of the two overlapping ablation areas and the surface of the conductive part is large, exceeding the wall thickness of the left atrial appendage, so transmural ablation can be realized.

With reference to Figure 35 and Figure 36, the operation method of the ablation system 3300 of this embodiment includes the following steps:
Step A: Use the delivery device 3320 to deliver the left atrial appendage occlusion and ablation device to the opening of the left atrial appendage for release to occlude the opening of the left atrial appendage. Specifically, after the left atrial appendage occlusion and ablation device is released into the left atrial appendage 10, the inner sheath tube 3321 remains connected with the occlusion and ablation device, and the second conductive part 3332 (ablation catheter 3323) is released; When the second conductive part 3332 is close to the tissue on the proximal side of the orifice of the left atrial appendage, the ablation catheter 3323 remains stationary, and the pulse ablation source is turned on at the same time; Under the action of the pulse energy source, at least one characteristic electric field line E formed by the first conductive part 3331 and the second conductive part 3332 passes through the orifice tissue, which is convenient for transmural ablation at the orifice. It destroys the intracellular and extracellular environment homeostasis, achieves the effect of electrical isolation, ensures that electrical physiological signals cannot be transmitted between the left atrial appendage 10 and the left atrium 12 on both sides of the ablated tissue, and achieves the purpose of treating atrial fibrillation. After the ablation is completed, the connecting steel cable is released.
Step B: Withdraw the ablation catheter 3323 to complete the operation.
Step C: The ablation catheter 3323 performs ablation alone. Specifically, under DSA angiography or ultrasound guidance, the ablation catheter 3323 is positioned at the orifice 11 of the left atrial appendage, the second conductive part 3332 is close to the tissue on the proximal side of the orifice of the left atrial appendage, the ablation catheter 3323 remains stationary, and the pulse ablation source is turned on at the same time. The electrodes of the second conductive part 3332 are used for transmitting pulse ablation energy with different polarities. The plurality of electrodes of the second conductive part 3332 form a ring, which is convenient for forming a transmural annular ablation zone in the left atrial appendage 10, the orifice 11 of the left atrial appendage, the proximal side of the orifice of the left atrial appendage, the pulmonary vein and the atrium.

Step C can be implemented before step A and/or between step A and step B. The release sequence of the ablation catheter 3323 and the occlusion and ablation device can be adjusted as required, and their release sequences are not mutually restrictive. In some embodiments, step C can be omitted.

### Embodiment 34

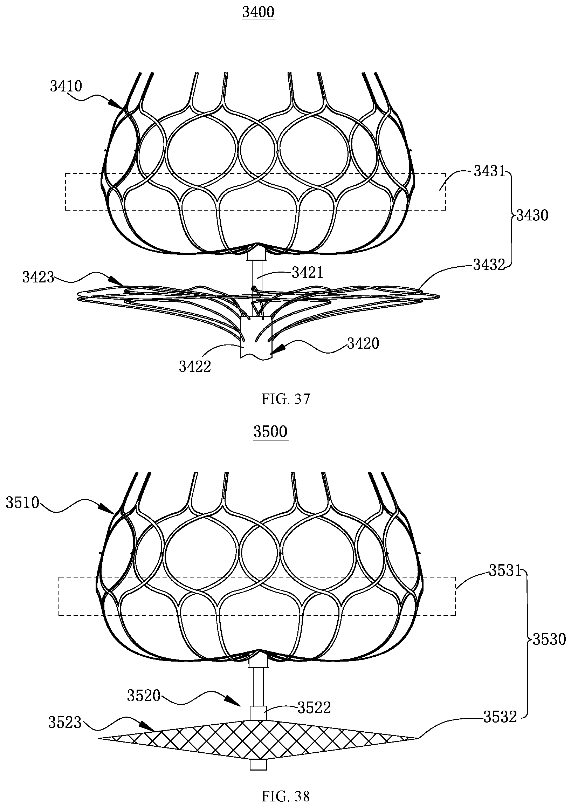

Figure 37 is a schematic structural diagram of the ablation system 3400 according to the thirty - fourth embodiment of the present application.

The ablation system 3400 of this embodiment is similar to the ablation system 3300 of the thirty - third embodiment, and the same parts can refer to the description of the thirty - third embodiment above, which will not be repeated here. The main difference between this embodiment and the thirty - third embodiment is that the structure of the ablation catheter is different.

In this embodiment, the ablation catheter 3423 includes an outer sheath tube 3422 and a plurality of support rods arranged at the distal end of the outer sheath tube 3422. The first conductive part 3431 is arranged on the outer peripheral wall of the proximal side of the support body 3410. The second conductive part 3432 is arranged on the plurality of support rods, and the second conductive part 3432 can be a part or all of the plurality of support rods or an electrode part arranged on the plurality of support rods.

Each support rod in the plurality of support rods forms a support ring, and the plurality of support rings are sequentially arranged in the circumferential direction. The projection of each support ring on the plane perpendicular to the axis of the support body is ring - shaped, which is fan - shaped in this embodiment. In other embodiments, the ring can be circular, elliptical, partial ring, or other regular or irregular rings. The projections of adjacent support rings on the plane perpendicular to the axis of the support body have overlapping parts. In other embodiments, the projections of adjacent support rings on this plane do not overlap, or the overlapping parts are smaller or larger.

The positions of the two ends of the support rod in the axial direction do not coincide, that is, they are staggered in the axial direction, so as to increase the support performance of each support ring. In some embodiments, the positions of the two ends of the support ring in the axial direction are the same, and can be connected together or arranged at intervals.

The edge part of the support ring can be better apposed to the tissue wall, ensuring the effects of ablation and mapping.

In some embodiments, the support body 3410 adopts a single - disk or multi - disk structure, and the second conductive part 3432 is arranged on the delivery device 3420. The second conductive part 3432 can move relative to the support body 3410, and the second conductive part 3432 is used for being released on the proximal side of the support body 3410, and the first conductive part 3431 is adjacently arranged on the proximal end of the support body 3410. The distance between the first conductive part 3431 and the second conductive part 3432 can be adjusted to be relatively close, so that the ablation catheter at the distal end of the delivery device 3420 has a good cooperation effect with the first conductive part 3431 on the sealing part 3410.

### Embodiment 35

Figure 38 is a schematic structural diagram of the ablation system 3500 according to the thirty - fifth embodiment of the present application.

The ablation system 3500 of this embodiment is similar to the ablation system 200 of the thirty - fourth embodiment, and the same parts can refer to the description of the thirty - fourth embodiment above, which will not be repeated here. The main difference between this embodiment and the thirty - fourth embodiment is that the structure of the ablation catheter is different.

In this embodiment, the ablation catheter 3523 is a disk - shaped grid structure arranged at the distal end of the outer sheath tube 3522, and the disk - shaped grid structure can be made by braiding or cutting processes. As shown in Figure 38, the disk - shaped grid structure is a double - layer disk, that is, the disk - shaped grid structure includes two layers of braided nets in the axial direction (up and down direction in the figure) of the disk - shaped grid structure, and the circumferential edges of the two layers of braided nets are connected to each other. In other embodiments, the disk - shaped grid structure is a single - layer disk, that is, the disk - shaped grid structure only includes a single layer of braided net in the axial direction (up and down direction in the figure) of the disk - shaped grid structure.

The second conductive part 3532 is arranged on the braided disk. The first conductive part 3531 is arranged on the outer peripheral wall of the proximal side of the support body 3510. The second conductive part 3532 can be a part or all of the disk - shaped grid structure, so that the entire braided disk can be used as an electrode to transmit ablation energy to the tissue. In some embodiments, the second conductive part 3532 can be an electrode arranged on the disk - shaped grid structure.

### Embodiment 36

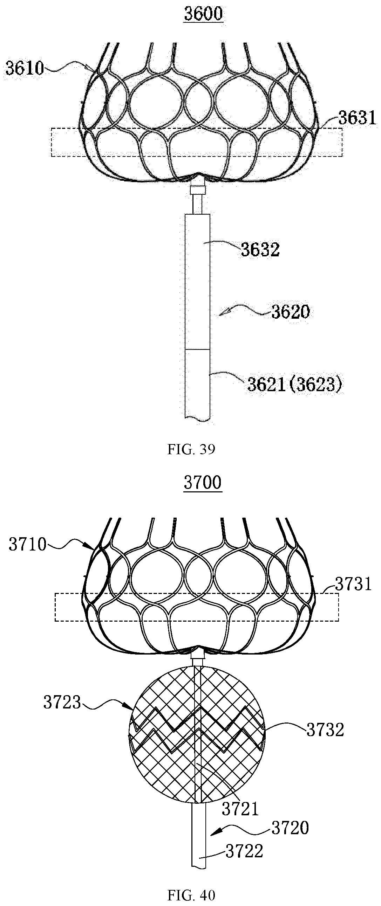

Figure 39 is a schematic structural diagram of the ablation system 3600 according to the thirty - sixth embodiment of the present application.

The ablation system 3600 of this embodiment is similar to the ablation system 3300 of the thirty - third embodiment shown in Figure 35, and the same parts can refer to the description of the thirty - third embodiment above, which will not be repeated here. The main difference between this embodiment and the thirty - third embodiment is that the structure of the ablation catheter is different.

In this embodiment, the ablation catheter 3623 is an inner sheath tube 3621, and the distal end of the inner sheath tube 3621 is detachably connected with the support body 3610. The inner sheath tube 3621 is rod - shaped or tubular, and the second conductive part 3632 is arranged on the outer peripheral side wall of the inner sheath tube 3621. The second conductive part 3632 can be an electrode or an exposed part of a conductive piece in the inner sheath tube 3621. The first conductive part 3631 is arranged on the outer peripheral wall of the proximal side of the support body 3610.

### Embodiment 37

Figure 40 is a schematic structural diagram of the ablation system 3700 according to the thirty - seventh embodiment of the present application.

The ablation system 3700 of this embodiment is similar to the ablation system 100 of the thirty - third embodiment, and the same parts can refer to the description of the thirty - third embodiment above, which will not be repeated here. The main difference between this embodiment and the thirty - third embodiment is that the structure of the ablation catheter is different.

In this embodiment, the delivery device 3720 includes an outer sheath tube 3722 and an inner sheath tube 3721 which is inserted into the inner cavity of the outer sheath tube 3722. The ablation catheter 4023 in the delivery device 3720 is a spherical grid structure arranged at the distal end of the outer sheath tube 3722. The second conductive part 3732 is at least one wave electrode arranged in the circumferential edge area of the spherical grid structure, so that the second conductive part 3732 can be closely apposed to the tissue during ablation. The spherical grid structure can be made by braiding or cutting processes, and the spherical grid structure can be radially retractable and expandable. In the natural state without external force, the spherical grid structure can be a regular or irregular sphere such as a sphere or an ellipsoid. The first conductive part 3731 is arranged on the outer peripheral wall of the proximal side of the support body 3710.

The delivery device 3720 adopts a double - layer sheath tube, and the inner sheath tube 3721 is inserted into the outer sheath tube 3722, and can be used as a delivery catheter to connect with the support body 3710. The distal end of the spherical grid structure is connected with the distal end of the inner sheath tube 3721. Before the spherical grid structure performs the ablation function, the diameters of the spherical grid structure and the second conductive part 3732 can be adjusted through the inner and outer sheath tubes. Specifically, it can be adjusted by fixing the inner sheath tube 3721, pushing the outer sheath tube 3722 to the distal end, and/or pulling the inner sheath tube 3721. The spherical grid structure is compressed, thereby increasing the radial size of the second conductive part 3732 and making its edge appose to the tissue outside the orifice of the left atrial appendage. After the ablation is completed, the axial length of the spherical grid structure is increased by adjusting the inner and outer sheath tubes until the spherical grid structure can be converged for delivery.

It can be understood that in the natural state without external force, the spherical grid structure can be a regular or irregular closed structure such as a sphere, an ellipsoid, or a cylinder.

### Embodiment 38

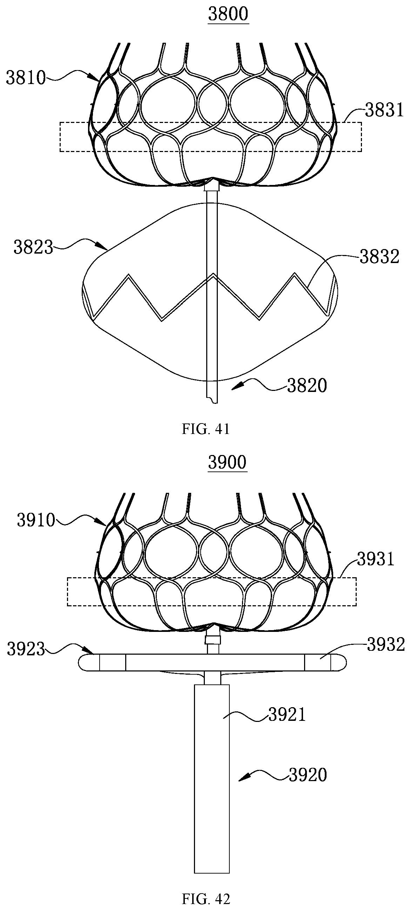

Figure 41 is a schematic structural diagram of the ablation system 3800 according to the thirty - eighth embodiment of the present application.

The ablation system 3800 of this embodiment is similar to the ablation system 3700 of the thirty - seventh embodiment, and the same parts can refer to the description of the thirty - seventh embodiment above, which will not be repeated here. The main difference between this embodiment and the thirty - seventh embodiment is that the structure of the ablation catheter is different.

In this embodiment, the ablation catheter 3823 is a balloon arranged on the delivery device 3820. After the interior of the balloon is filled with a medium, it expands outside the orifice of the left atrial appendage. The shape of the balloon after inflation is disk - shaped. The second conductive part 3832 is arranged on the outer peripheral edge of the inflated balloon. After the ablation is completed, the balloon is recovered into the delivery device. The first conductive part 3831 is arranged on the circumferential outer side surface of the support body 3810 close to the proximal side.

### Embodiment 39

Figure 42 is a schematic structural diagram of the ablation system 3900 according to the thirty - ninth embodiment of the present application.

The ablation system 3900 of this embodiment is similar to the ablation system 3300 of the thirty - third embodiment, and the same parts can refer to the description of the thirty - third embodiment above, which will not be repeated here. The main difference between this embodiment and the thirty - third embodiment is that the structure of the ablation catheter is different.

In this embodiment, the delivery device 3920 includes an outer sheath tube 3921 and an ablation catheter 3923 arranged at the distal end of the outer sheath tube 3921. In the natural state, the ablation catheter 3923 is ring - shaped, and the second conductive part 3932 is arranged on the ablation catheter 3923. The ablation catheter 3923 is coiled around the axis of the outer sheath tube 3921 into a planar ring or a three - dimensional ring. The ablation catheter 3923 is wound around the axis of the outer sheath tube 3921 for at least one circle, and can be multiple circles. The second conductive part 3932 includes a plurality of parts arranged at intervals along the length direction of the ablation catheter 3923, such as a plurality of electrode parts arranged at intervals in sequence along the axial direction of the ablation catheter 3923. The second conductive part 3932 can be a part or all of the ablation catheter 3923, and can be arranged at intervals or continuously. The first conductive part 3931 is arranged on the circumferential outer side surface of the support body 3910 close to the proximal side.

### Embodiment 40

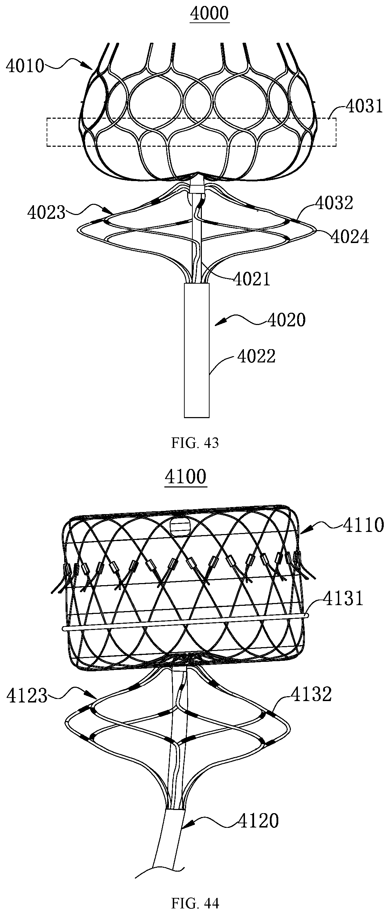

Figure 43 is a schematic structural diagram of the ablation system 4000 according to the fortieth embodiment of the present application.

The ablation system 4000 of this embodiment is similar to the ablation system 3300 of the thirty - third embodiment shown in Figure 35, and the same parts can refer to the description of the thirty - third embodiment above, which will not be repeated here. The main difference between this embodiment and the thirty - third embodiment is that the structure of the ablation catheter is different.

In this embodiment, the delivery device 4020 includes an inner sheath tube 4021, an outer sheath tube 4022 and an ablation catheter 4023. The ablation catheter 4023 includes a plurality of support rods 4024. The inner sheath tube 4021 is inserted into the outer sheath tube 4022. The distal ends of the support rods 4024 are combined together and connected to the distal end of the inner sheath tube 4021. The proximal ends of the support rods 4024 are combined together and connected to the distal end of the outer sheath tube 4022. The support rod 4024 is spiral, which is convenient for deformation during loading and release. In addition, during ablation, the radial size of the support rod 4024 is changed by pulling the inner sheath tube 4021 of the delivery device 4020 and/or pushing the outer sheath tube 4022 to the distal end. That is, the support rod 4024 is compressed in the axial direction, the radial size of the support rod 4024 is expanded, and the spiral support rod 4024 can easily adapt to the tissue shape at the orifice, appose to the tissue after deformation, and will not produce large pressure on the tissue. The second conductive part 4032 arranged on the spiral support rod 4024 is used for ablating the tissue. The first conductive part 4031 is arranged on the circumferential outer side surface of the support body 4010 close to the proximal end.

After the radial size of each support rod 4024 is compressed to a certain extent, the spiral support rods 4024 are easy to be stacked on each other in the axial direction, forming a structure for apposing the tissue on the outer side (proximal side) of the orifice, that is, forming a stable disk - shaped structure. A plurality of electrode parts in the second conductive part 4032 are arranged at intervals on each support rod 4024. Preferably, the second conductive part 4032 is at least arranged in the area with a larger radial size when the support rod 4024 is in the axially compressed state, so as to ablate the tissue in the edge area on the outer side of the orifice. After the ablation is completed, each support rod 4024 can be recovered into the outer sheath tube 4022. In some embodiments, the support rod 4024 can also have no spiral angle.

### Embodiment 41

Figure 44 is a schematic structural diagram of the ablation system 4100 according to the forty - first embodiment of the present application.

The ablation system 4100 of this embodiment is similar to the ablation system 4000 of the fortieth embodiment, and the same parts can refer to the description of the fortieth embodiment above, which will not be repeated here. The main difference between this embodiment and the fortieth embodiment is that the structure of the support body is different.

In this embodiment, the support body 4110 is a single - disk cylindrical structure, which can be made by braiding or cutting processes. A first conductive part 4131 is arranged on the outer periphery of the proximal side of the support body 4110, and the first conductive part 4131 is a ring electrode. The second conductive part 4132 is arranged on the delivery device 4120, and the first conductive part 4131 and the second conductive part 4132 cooperate with each other for ablation.

### Embodiment 42

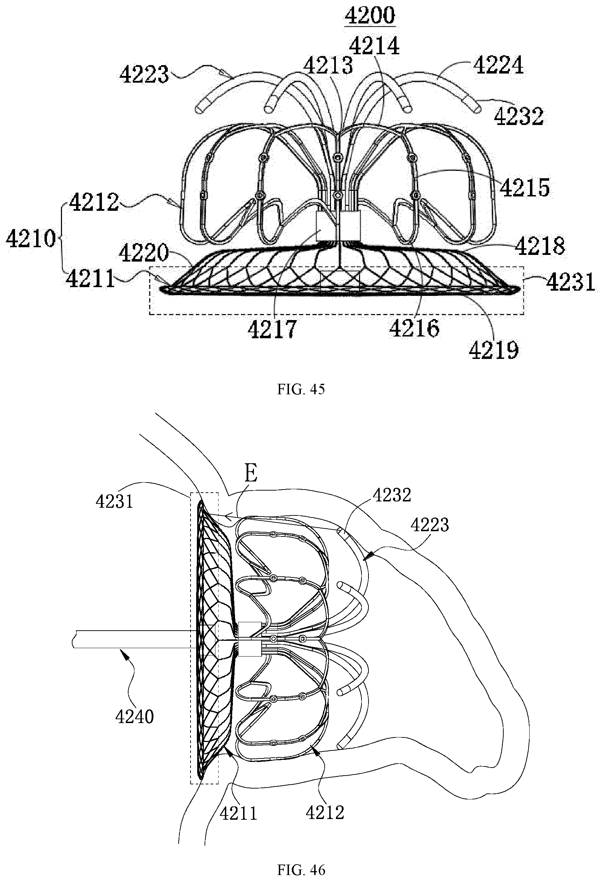

Figure 45 is a schematic structural diagram of the ablation system 4200 according to the forty - second embodiment of the present application, and Figure 46 is a schematic diagram of the principle structure of the ablation system for ablating the left atrial appendage tissue.

The ablation system 4200 of this embodiment is similar to the ablation system 3300 of the thirty - third embodiment shown in Figure 35, and the same parts can refer to the description of the thirty - third embodiment above, which will not be repeated here. The main difference between this embodiment and the thirty - third embodiment is that the structure of the support body and the arrangement mode of the delivery device are different.

The structures of the support body 4210 and the first conductive part 4231 provided in this embodiment are consistent with those of the support body and the first conductive part 2150 provided in the left atrial appendage occlusion and ablation device 2100 shown in Figure 23A and Figure 23B.

With reference to Figure 45 and Figure 46, in this embodiment, the second conductive part 4232 is arranged on the delivery device, and the second conductive part 4323 can move relative to the support body 4210 and is used for being released on the distal side of the support body 4210. The support body 4210 has a double - disk structure. The structure of the support body 4210 is similar to that shown in Figure 21 and Figure 22. The support body 4210 includes a sealing part 4211 and an anchoring part 4212, which are connected through a connecting piece 4217 and realize electrical isolation. A channel is formed along the axial direction in the space enclosed by the support body 4210. The distal end of the ablation catheter 4223 passes through the channel and can be released on the distal side of the anchoring part 4212.

The sealing part 4211 is trapezoidal, including a distal end disk surface 4218 facing the anchoring part 4212, a proximal end disk surface 4219 facing away from the anchoring part 4212, and a waist part 4220 between the distal end disk surface 4218 and the proximal end disk surface 4219. The proximal end disk surface 4219 and the distal end disk surface 4218 are roughly planar, the waist part 4220 is connected between the proximal end disk surface 4219 and the distal end disk surface 4218, and the waist part 4220 is in the shape of a conical cylinder.

The first conductive part 4231 is at least arranged in the edge area with a larger radial size of the sealing part 4211, such as the waist part 4220. Preferably, the first conductive part 4231 is arranged at the part with the largest radial size in the circumferential direction of the sealing part 4211. In this embodiment, as shown in Figure 45, the first conductive part 4231 is arranged on the waist part 4220 and the proximal end disk surface 4219 (the part in the dotted line frame in the figure), or on the waist part 4220, the proximal end disk surface 4219 and at least a part of the distal end disk surface 4218. The first conductive part 4231 is a part of the first framework in the sealing part 4211. The surface of the remaining part of the sealing part 4211 that is not used as the first conductive part 4231 can be insulated.

Specifically, the structure of the sealing part 4211 is consistent with that of the sealing part 2110 in Figure 23A and Figure 23B. The main difference from the sealing parts provided in Figure 21 and Figure 22 is the grid form. The mesh sizes of the proximal part and the distal part of the sealing part 4211 are different. Specifically, the mesh of the proximal part of the sealing part 4211 is smaller and the number is larger, and the mesh of the distal part of the sealing part 4211 is larger and the number is smaller. The first framework of the small mesh part in the sealing part 4211 is used as the first conductive part, and the surface of the first framework of the large mesh part is insulated. Specifically, the small mesh part is formed by dense mesh braiding, and the large mesh part is obtained by extending multiple strands of braided wires in the small mesh part in a bundle, and the multiple strands of braided wires in each bundle are fixed to each other. There are no cross points that can rub against each other between the first frameworks in the large mesh part, or the density of cross points that can rub against each other is lower than that in the small mesh area. For example, friction may occur between two bundles of braided wires, which is convenient for insulation treatment on the first framework in the large mesh area. It can be understood that the sealing part 4211 can also adopt other insulation modes or add other insulation modes, such as arranging an insulating sleeve, arranging a membrane, etc.

The structure of the anchoring part 4212 is the same as that in Figure 21 and Figure 22, showing a disk shape. The anchoring part 4212 includes a plurality of anchoring rods, and the anchoring rods include a first anchoring rod 4213, a second anchoring rod 4214, a third anchoring rod 4215 and an fourth anchoring rod 4216 which are connected in sequence. The proximal end of the first anchoring rod 4213 is connected with the insulating connecting piece 4217, and the first anchoring rod 4213 extends between the proximal end and the distal end. One ends of two adjacent second anchoring rods 4214 far away from the third anchoring rod 4215 are connected to each other and connected to the corresponding first anchoring rod 4213. The third anchoring rod 4215 extends between the proximal end and the distal end, and is located on the outer peripheral side of the anchoring part 4212, used for leaning against the inner wall of the left atrial appendage. The proximal end of the third anchoring rod 4215 is connected with the corresponding two second anchoring rods 4214. The second anchoring rods 4214 are connected end to end in the circumferential direction to form a sawtooth structure. One end of the fourth anchoring rod 4216 far away from the third anchoring rod 4215 extends towards the axis and to the distal end. One ends of two adjacent fourth anchoring rods 4216 far away from the third anchoring rod 4215 are connected together, which can improve the mechanical performance of the anchoring part 4212, avoid mutual hooking of adjacent fourth anchoring rods 4216 during radial deformation of the anchoring part 4212, and improve the reliability of the support body 4210. Two dot electrodes are arranged at intervals on each third anchoring rod 4215.

The structure of the ablation catheter 4223 is the same as that of the ablation catheter provided in Figure 35, and also adopts an umbrella - shaped frame body. The ablation catheter 4223 includes a plurality of support rods 4224 radially arranged around its axis. An electrode part is arranged at the circumferential end of each support rod 4224, so that the second conductive part 4232 is formed at the circumferential end of the umbrella - shaped frame body.

The difference between the ablation catheter 4223 in this embodiment and the ablation catheter in Figure 35 is that the ablation catheter 4223 is used for release on the distal side of the support body 4210.

Specifically, with reference to Figure 45 and Figure 46, the support body 4210 is delivered to the opening of the left atrial appendage through the delivery device 4240. The waist part 4220 of the sealing part 4211 leans against the opening of the left atrial appendage. The first conductive part 4231 on the waist part 4220 leans against the tissue on the proximal side of the left atrial appendage. The third anchoring rod 4215 leans against the inner wall of the left atrial appendage. The pulse ablation source is turned on. The characteristic electric field line between the first conductive part 4231 and the second conductive part 4232 passes through the orifice tissue, which is convenient for transmural ablation at the orifice. After the ablation is completed, the support body 4210 is released, and the ablation catheter 4223 is withdrawn. The distal end of the ablation catheter 4223 in this embodiment passes through the axial channel of the support body 4210 and can be released on the distal side of the anchoring part 4212.

In other embodiments, the support body adopts the structure provided in other embodiments, and the release position of the second conductive part on the delivery device can be adaptively changed according to the specific structure of the support body. For example, the anchoring part of the support body is cup - shaped, and the distal end is open, and the second conductive part can be released on the distal side of the support body. Specifically, the second conductive part can be released in the anchoring part, on the distal end of the support body or beyond the distal end of the support body.

### Embodiment 43

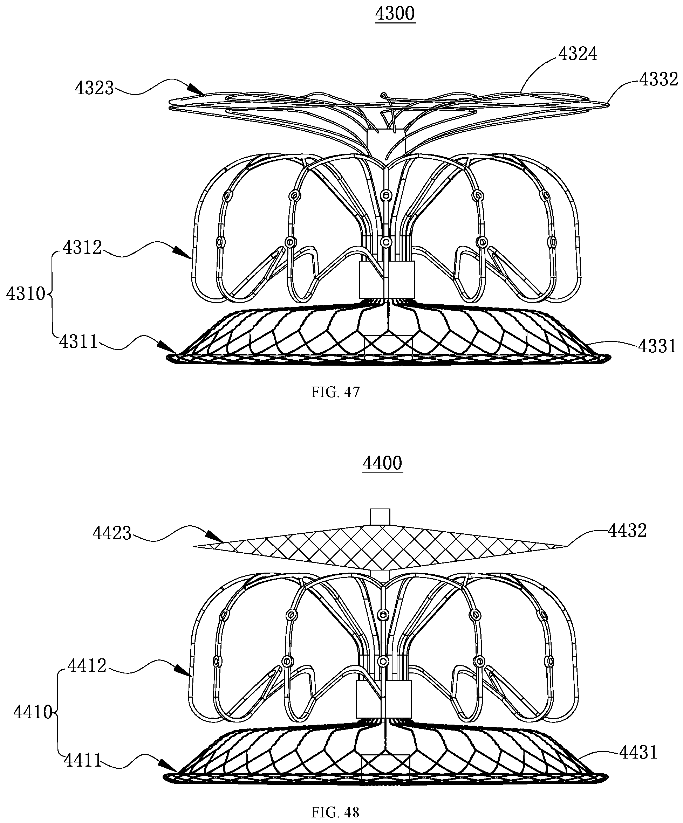

Figure 47 is a schematic structural diagram of the ablation system 4300 according to the forty - third embodiment of the present application.

The ablation system 4300 of this embodiment is similar to the ablation system 4200 of the forty - second embodiment, and the structures of the support body and the arrangement mode of the ablation catheter are the same. The same parts can refer to the description of the forty - second embodiment above, which will not be repeated here. The main difference between this embodiment and the forty - second embodiment is that the structure of the ablation catheter is different.

In this embodiment, the structure of the ablation catheter 4323 is the same as that of the ablation catheter of the thirty - fourth embodiment corresponding to Figure 37, and the second conductive part 4332 is arranged on the support ring. The description of the structure of the support ring will not be repeated. The difference from the ablation system 200 of the thirty - fourth embodiment is that the distal end of the ablation catheter 4323 in this embodiment passes through the axial channel of the support body 4310 and can be released on the distal side of the anchoring part 4312. The ablation catheter of the thirty - fourth embodiment is released on the proximal side of the sealing part.

### Embodiment 44

Figure 48 is a schematic structural diagram of the ablation system 4400 according to the forty - fourth embodiment of the present application.

The ablation system 4400 of this embodiment is similar to the ablation system 4200 of the forty - second embodiment corresponding to Figure 45, and the same parts can refer to the description of the forty - second embodiment above, which will not be repeated here. The main difference between this embodiment and the forty - second embodiment is that the structure of the ablation catheter is different.

In this embodiment, the structure of the ablation catheter 4423 is the same as that of the ablation catheter 3523 of the thirty - fifth embodiment corresponding to Figure 38, and the ablation catheter 4423 also adopts a disk - shaped grid structure. The second conductive part 4432 is arranged on the disk - shaped grid structure. The first conductive part 4431 is arranged on the sealing part 4411. The difference from the ablation system of the thirty •fifth embodiment is that the distal end of the ablation catheter 4423 in this embodiment passes through the axial channel of the support body 4410 and can be released on the distal side of the anchoring part 4412. The ablation catheter of the thirty - fifth embodiment is released on the proximal side of the sealing part.

### Embodiment 45

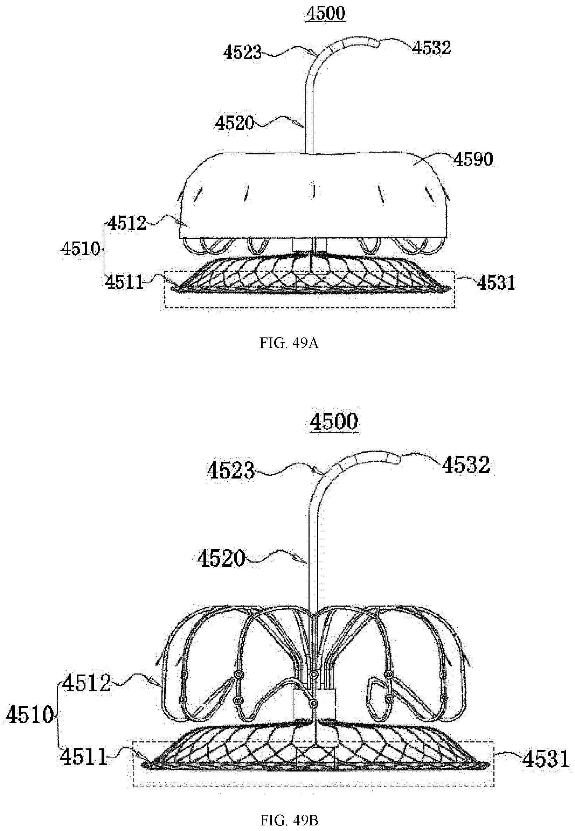

Figure 49A and Figure 49B are schematic structural diagrams of the ablation system 4500 according to the forty - fifth embodiment of the present application.

A membrane 4590 is arranged on the surface of the anchoring part 4512 of the ablation system 4500 of this embodiment, and the membrane 4590 is used for blocking flow and insulating between the second conductive part 4532 and the anchoring part 4512.

The ablation system 4500 of this embodiment is similar to the ablation system 4000 of the forty - second embodiment corresponding to Figure 45, and the same parts can refer to the description of the forty - second embodiment above, which will not be repeated here. The main difference between this embodiment and the forty - second embodiment is that the structure of the ablation catheter is different.

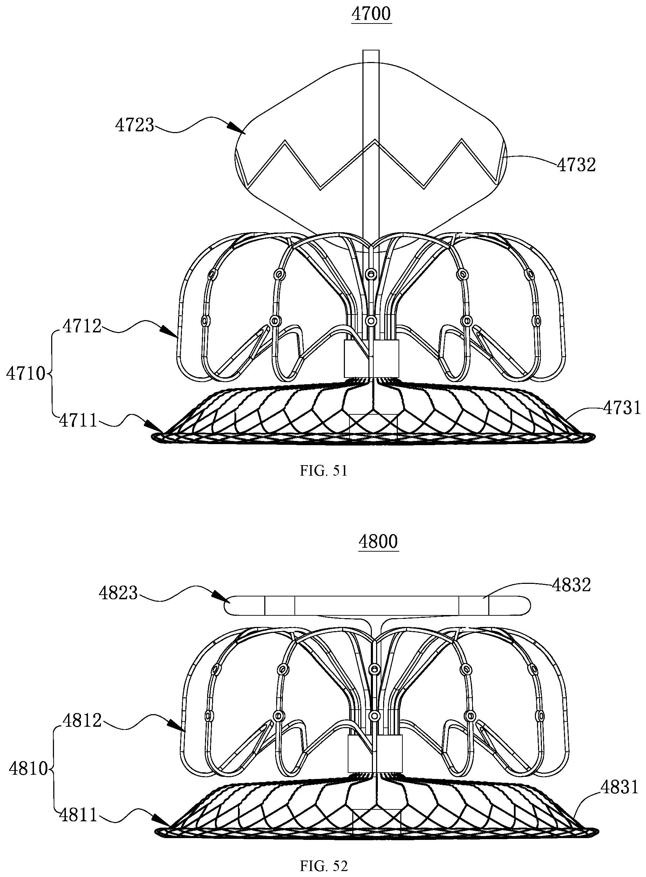

In this embodiment, in the delivery state, the ablation catheter 4523 is linearly accommodated in the outer catheter of the delivery device, and after the ablation catheter 4523 is released from the outer catheter on the distal side of the anchoring part 4512, one end of the ablation catheter 4523 far away from the support body 4510 bends and extends radially outward, and can be coiled into a ring shape (the shape of the ablation catheter 4823 in Figure 52). In some embodiments, the ablation catheter 4523 is also linear after being released from the outer catheter, and the second conductive part 4532 is arranged on the side wall of the ablation catheter 4523. The first conductive part 4531 is arranged on the sealing part 4511. The second conductive part 4532 can be an electrode or an exposed part of a conductive piece in the ablation catheter 4523.

In some embodiments, the anchoring part 4512 is provided with a third conductive part for releasing pulse ablation energy to the tissue. In Figure 49A to Figure 49B, the third conductive part can be an electrode part arranged on the surface of the anchoring part 4512, or a part of the second framework in the anchoring part 4512.

At least one of the first conductive part 4531, the second conductive part 4532 and the third conductive part is used for releasing pulse ablation energy to the tissue; In some embodiments, all of the first conductive part 4531, the second conductive part 4532 and the third conductive part are used for releasing pulse ablation energy; In some embodiments, the first conductive part 4531 and the third conductive part are used for releasing pulse ablation energy, that is, the first conductive part 4531 and the third conductive part are used for transmitting pulse ablation energy with opposite polarities and forming an ablation loop. The second conductive part 4532 is used for collecting electrophysiological signals of the target tissue; In some embodiments, at least one of the first conductive part 4531, the second conductive part 4532 and the third conductive part is used for ablation and mapping at different times.

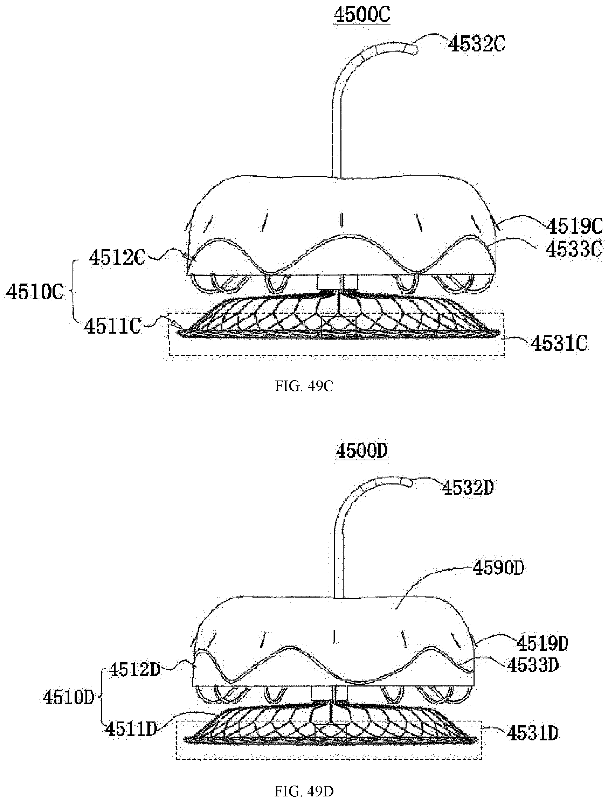

Please refer to Figure 49C. Figure 49C is a modified embodiment based on the forty - fifth embodiment in Figure 49A. The difference between the ablation system 4500C provided in this embodiment and the ablation system 4500 in the forty - fifth embodiment is that a third conductive part 4533C is arranged on the surface of the anchoring part 4512C, and the third conductive part 4533C is specifically a wave electrode. The wave crests and wave troughs of the third conductive part 4533C are arranged corresponding to the anchoring rods in the anchoring part 4512C.

Anchor thorns 4519C are arranged on the surface of the anchoring part 4512C, and the anchor thorns 4519C are arranged on the distal side of the third conductive part 4533C, so as to avoid sparks generated by the anchor thorns 4519C when the first conductive part 4531C and the third conductive part 4533C discharge for ablation at the same time.

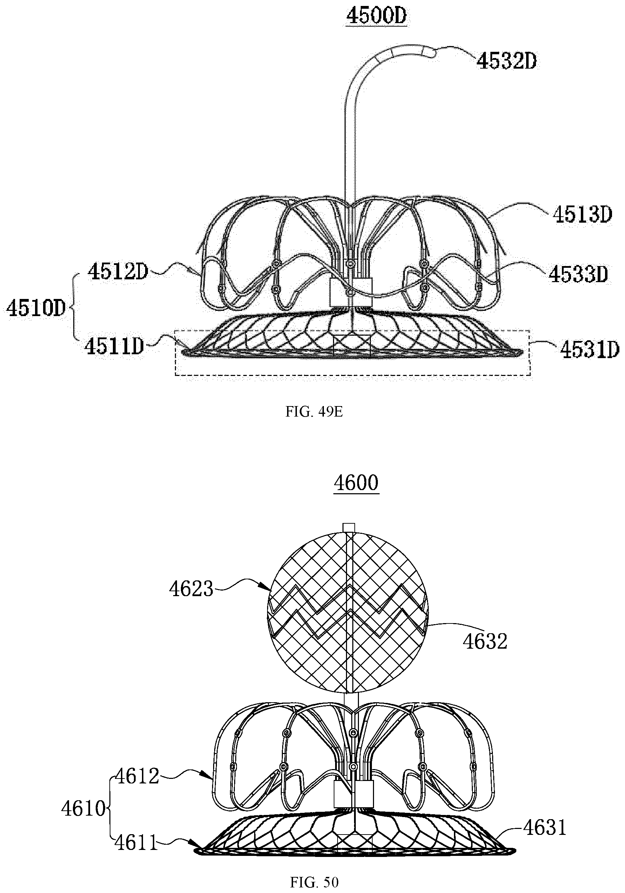

Please refer to Figure 49D and Figure 49E. The ablation system 4500D provided in Figure 49D and Figure 49D is a modified embodiment based on the ablation system 4500C in Figure 49C. The difference between the ablation system 4500D in this embodiment and the ablation system 4500C is that the wave crests and wave troughs of the third conductive part 4533D are arranged staggered from the anchoring rods 4513D of the anchoring part 4512D. The third conductive part 4533D can be connected to the membrane 4590D through the wave crests and wave troughs, so as to avoid perforation at the position where the wave crests and wave troughs of the third conductive part 4533D are connected to the membrane 4590D, and short circuit between the third conductive part 4533D and the anchoring part 4512 through the perforation, thus improving the insulation performance between the third conductive part 4533D and the anchoring part 4512D.

### Embodiment 46

Figure 50 is a schematic structural diagram of the ablation system 4600 according to the forty - sixth embodiment of the present application.

The ablation system 4600 of this embodiment is similar to the ablation system 4200 of the forty - second embodiment corresponding to Figure 45, and the same parts can refer to the description of the forty - second embodiment above, which will not be repeated here. The main difference between this embodiment and the forty - second embodiment is that the structure of the ablation catheter is different.

In this embodiment, the first conductive part 4631 is arranged on the sealing part 4611. The ablation catheter 4623 is a spherical grid structure, and the second conductive part 4632 is arranged in the circumferential edge area of the spherical grid structure. The spherical grid structure is the same as that of the thirty - seventh embodiment corresponding to Figure 40, which will not be repeated here. The difference from the ablation system of the thirty - seventh embodiment is that the distal end of the ablation catheter 4623 in this embodiment passes through the axial channel of the support body 4610 and can be released on the distal side of the anchoring part. The ablation catheter of the thirty - seventh embodiment is released on the proximal side of the sealing part.

### Embodiment 47

Figure 51 is a schematic structural diagram of the ablation system 4700 according to the forty - seventh embodiment of the present application.

The ablation system 4700 of this embodiment is similar to the ablation system 1000 of the forty - second embodiment corresponding to Figure 45, and the same parts can refer to the description of the forty - second embodiment above, which will not be repeated here. The main difference between this embodiment and the forty - second embodiment is that the structure of the ablation catheter is different.

In this embodiment, the ablation catheter 4723 is a balloon, which is similar to the structure of the ablation catheter provided in the thirty - eighth embodiment corresponding to Figure 41. After the interior of the balloon is filled with a medium, it expands outside the orifice of the left atrial appendage. The shape of the balloon after inflation is disk - shaped. The second conductive part 4732 is arranged on the outer peripheral edge of the inflated balloon. After the ablation is completed, the balloon is recovered into the outer sheath tube. The first conductive part 4731 is arranged on the sealing part 4711. The distal end of the ablation catheter 4723 in this embodiment passes through the axial channel of the support body 4710 and can be released on the distal side of the anchoring part 4712. The ablation catheter of the thirty - eighth embodiment is released on the proximal side of the sealing part.

### Embodiment 48

Figure 52 is a schematic structural diagram of the ablation system 4800 according to the forty - eighth embodiment of the present application.

The ablation system 4800 of this embodiment is similar to the ablation system 4200 of the forty - second embodiment corresponding to Figure 45, and the same parts can refer to the description of the forty - second embodiment above, which will not be repeated here. The main difference between this embodiment and the forty - second embodiment is that the structure of the ablation catheter is different.

In this embodiment, the ablation catheter 4823 is a ring body, which is similar to the structure of the ablation catheter provided in the thirty - ninth embodiment corresponding to Figure 42. The second conductive part 4832 is arranged on the ring body, and the first conductive part 4831 is arranged on the sealing part 4811. The second conductive part 4832 includes a plurality of parts arranged at intervals along the axial direction of the ring body, such as a plurality of electrode parts arranged at intervals in sequence along the axial direction of the ring body. The second conductive part 4832 can be a part or all of the ring body, and can be arranged at intervals or continuously. The distal end of the ablation catheter 4823 in this embodiment passes through the axial channel of the support body 4810 and can be released on the distal side of the anchoring part 4812. The ablation catheter of the thirty - ninth embodiment is released on the proximal side of the sealing part.

### Embodiment 49

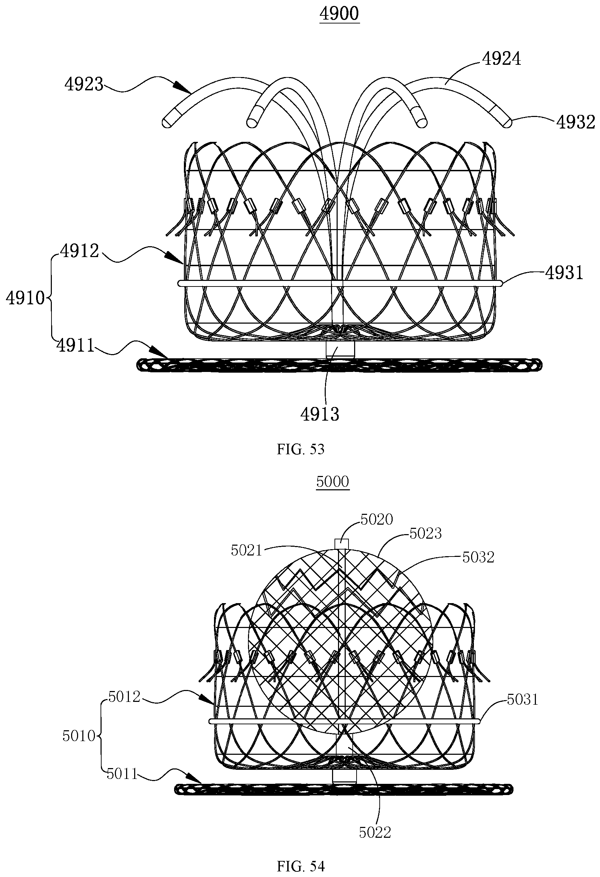

Figure 53 is a schematic structural diagram of the ablation system 4900 according to the forty - ninth embodiment of the present application.

The ablation system 4900 of this embodiment is similar to the ablation system 4200 of the forty - second embodiment corresponding to Figure 45, and the same parts can refer to the description of the forty - second embodiment above, which will not be repeated here. The main difference between this embodiment and the forty - second embodiment is that the structure of the support body and the arrangement mode of the first conductive part are different.

In this embodiment, the support body 4910 has a double - disk structure. The second conductive part 4932 is arranged on the delivery device, and the second conductive part 4932 can move relative to the support body 4910 and is used for being released on the distal side of the support body 4910. The first conductive part 4931 is adjacently arranged on the proximal end of the support body 4910. The support body 4910 includes a sealing part 4911 and an anchoring part 4912, which are connected through a connecting piece 4913 and realize insulated connection. The sealing part 4911 adopts a disk - shaped structure with a short axial length. The anchoring part 4912 is cup - shaped, and its distal end is open. The anchoring part 4912 can be made of braided metal wires or by cutting a pipe. The distal ends of the metal wires of the anchoring part 4912 are converged radially inward at the connecting piece 4913. The first conductive part 4931 is arranged on the surface of the outer peripheral side of the anchoring part 4912. The first conductive part 4931 is a ring electrode.

The ablation catheter 4923 of this embodiment has the same structure as the ablation catheter 4223 of the forty - second embodiment, and the ablation catheter 4923 also adopts an umbrella - shaped frame body. The ablation catheter 4923 includes a plurality of support rods 4924 radially arranged around its axis. An electrode part is arranged at the circumferential end of each support rod 4924, so that the second conductive part 4932 is formed at the circumferential end of the umbrella - shaped frame body. Since the distal side of the anchoring part 4912 is open, the ablation catheter 4923 can be released inside the anchoring part 4912.

### Embodiment 50

Figure 54 is a schematic structural diagram of the ablation system 5000 according to the fiftieth embodiment of the present application.

The structure of the ablation system of this embodiment is similar to that of the ablation system 4900 of the forty - ninth embodiment corresponding to Figure 53, and the same parts can refer to the description of the forty - ninth embodiment above, which will not be repeated here. The main difference between this embodiment and the forty - ninth embodiment is that the structure of the ablation catheter is different.

In this embodiment, the second conductive part 5032 is arranged on the delivery device, and the second conductive part 5032 can move relative to the support body 5010 and is used for being released inside the support body 5010. The anchoring part 5012 of the support body 5010 is cup - shaped, and its distal end is open, and the second conductive part 5032 can be released inside the anchoring part 5012. The ablation catheter 5023 is a balloon. After the interior of the balloon is filled with a medium, it expands outside the orifice of the left atrial appendage. The shape of the balloon after inflation is disk - shaped. The support body 5010 includes a sealing part 5011 and an anchoring part 5012 which are connected. The first conductive part 5031 is a circular electrode fixed on the outer peripheral wall of the anchoring part 5012. The second conductive part 5032 is arranged on the outer peripheral edge of the inflated balloon. After the ablation is completed, the balloon is recovered into the outer sheath tube 5022. Since the distal side of the anchoring part 5012 is open, the balloon can be released inside the anchoring part 5012.

It can be understood that for the support body 5010 with different structures, when the distal end of the anchoring part 5012 is open, such as the anchoring part 5012 is cylindrical or cup - shaped, the corresponding ablation catheter 5023 can be released inside the anchoring part 5012. In the forty - ninth embodiment and the fiftieth embodiment, the corresponding ablation catheter 5023 can be released inside the anchoring part 5012. The second conductive part 5032 can expand its radial size, contact the tissue through the grid of the anchoring part 5012, or ablate the left atrial appendage tissue by contacting the blood without deforming to expand its radial size.

### Embodiment 51

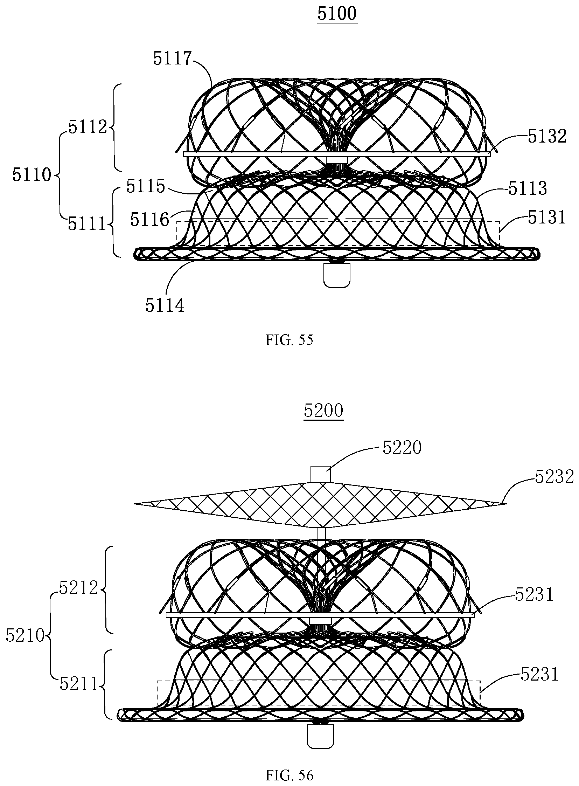

Figure 55 is a schematic structural diagram of the ablation system 5100 according to the fifty - first embodiment of the present application.

In this embodiment, the support body 5110 has a double - disk structure, and both the first conductive part 5131 and the second conductive part 5132 are arranged on the support body 5110.

The support body 5110 includes a sealing part 5111 and an anchoring part 5112. The sealing part 5111 includes a first framework 5113, and the anchoring part 5112 includes a second framework 5117. The second framework 5117 of the anchoring part 5112 is made of braided metal wires, and the braided second framework 5117 has an inner - outer double - layer structure. Specifically, the metal wires extend from the proximal end to the distal end of the anchoring part 5112 to form the inner layer of the second framework 5117, and then fold back from the distal end to the proximal end to form the outer layer of the second framework 5117.

The second conductive part is arranged on the second framework 5117 of the anchoring part 5112. In this embodiment, the second conductive part is a second electrode part arranged on the second framework 5117, and the second electrode part serves as the second conductive part 5132. The surface of the remaining part of the second framework 5117 that is not connected with the second electrode part is insulated, that is, the anchoring part 5112 can be set such that only the surface of the part of the second framework 5117 that does not correspond to the second electrode part is insulated.

Preferably, the second conductive part 5132 adopts a ring electrode, that is, the second electrode part is a ring electrode, and the ring electrode is fixed on the outer wall surface of the anchoring part 5112 in the circumferential direction. The anchoring part 5112 is set such that the surface of the entire second framework 5117 is insulated, and the ring electrode is an additionally arranged electrode. Specifically, the surface insulation of the second framework 5117 can be realized by arranging a membrane on the second framework 5117. The part of the second framework 5117 corresponding to the second electrode part is the part of the second framework 5117 in contact with the second electrode part. Correspondingly, the part of the second framework 5117 not corresponding to the second electrode part is the part of the second framework 5117 not in contact with the second electrode part. It can be understood that in other embodiments, the second electrode part can also be set to a structure bent and extended in the circumferential direction, such as a sawtooth shape, a wave shape, etc.

Specifically, the first framework 5113 is a structure formed by braiding metal wires. The sealing part 5111 includes a disk surface 5114 facing away from the anchoring part 5112, a disk bottom 5115 facing the anchoring part 5112, and a waist part 5116 connected between the disk surface 5114 and the disk bottom 5115. The diameter of the disk surface 5114 is larger than that of the disk bottom 5115, and the diameter of the disk surface 5114 is slightly larger than the inner diameter of the left atrial appendage. After the support body 5110 is implanted into the left atrial appendage, the disk surface 5114 presses against the opening of the left atrial appendage, and the disk bottom 5115 is inserted into the left atrial appendage. The diameter of the disk bottom 5115 is approximately the same as the inner diameter of the left atrial appendage. The first electrode part is arranged at the waist part 5116 of the sealing part 5111, serving as the first conductive part 5131, so as to fit the orifice of the left atrial appendage.

Meanwhile, in the support body 5110 shown in this embodiment, the first conductive part 5131 on the sealing part 5111 is a part of the first framework 5113, and the second conductive part 5132 on the anchoring part 5112 is a second electrode part arranged on the second framework. It can be understood that the framework or additional electrode parts can also be selected according to requirements.

### Embodiment 52

Figure 56 is a schematic structural diagram of the ablation system 5200 according to the fifty - second embodiment of the present application.

The structure of the support body 5210 of the ablation system 5200 in this embodiment is similar to that of the support body 5110 of the fifty - first embodiment corresponding to Figure 55. For the same parts, please refer to the description of the fifty - first embodiment above, which will not be repeated here. The main difference between this embodiment and the fifty - first embodiment is that the arrangement position of the second conductive part is different.

In this embodiment, two first conductive parts 5231 are arranged on the support body 5210 at intervals along the axis, which are respectively arranged on the sealing part 5211 and the anchoring part 5212. The first conductive part 5231 arranged on the sealing part 5211 is at least part of the first framework, and the first conductive part 5231 on the anchoring part 5212 is a ring electrode fixed on the outer wall surface. The second conductive part 5232 is arranged on the delivery device 5220.

The delivery device 5220 includes an ablation catheter, and the second conductive part 5232 is arranged on the ablation catheter. In this embodiment, the ablation catheter is a disk - shaped grid structure, which is similar to the structure of the ablation catheter provided in the thirty - fifth embodiment corresponding to Figure 38. Preferably, the second conductive part 5232 can be the entire disk, so that the entire braided disk can be used as an electrode to transmit ablation energy to the tissue. The distal end of the delivery catheter in this embodiment passes through the axial channel of the support body 5210 and can be released on the distal side of the anchoring part 5212.

In some embodiments, the anchoring part can be insulated. For example, an insulating coating is arranged on the second framework of the anchoring part, or an insulating sleeve is sleeved, or the anchoring part is made of an insulating material. By insulating the anchoring part, it can avoid electrical contact between the anchoring part 5212 and the second conductive part 5232, prevent the problem of increasing the discharge area of the second conductive part 5232, improve the stability of the system, and improve the success rate of ablation.

### Embodiment 53

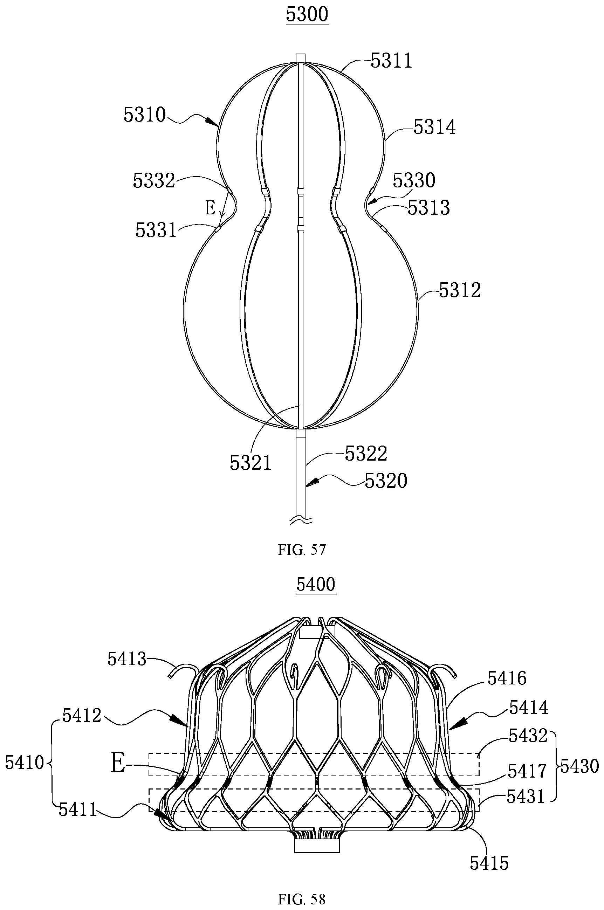

Figure 57 is a schematic structural diagram of the ablation system 5300 according to the fifty - third embodiment of the present application.

This embodiment of the ablation system 5300 is used for ablating the pulmonary veins, atria, left atrial appendage in the heart, or tissues to be ablated outside the heart. The support body is a radially retractable and expandable framework. The ablation system 5300 includes a support body 5310 arranged at the distal end and a delivery device 5320 arranged at the proximal end. The support body 5310 is connected to the distal end of the delivery device 5320, and is used for delivering the support body 5310 to the vicinity of the tissue to be ablated along the delivery path, releasing it, and realizing tissue ablation. The support body 5310 can adjust its radial size through the delivery device 5320, thereby realizing radial contraction and expansion. Specifically, the delivery device 5320 includes an outer sheath tube 5322 and an inner sheath tube 5321, and the inner sheath tube 5321 is inserted into the inner cavity of the outer sheath tube 5322. The proximal end of the support body 5310 is connected to the distal end of the outer sheath tube 5322, and the distal end of the support body 5310 is connected to the distal end of the inner sheath tube 5321. The radial size of the support body 5310 can be adjusted by adjusting the outer sheath tube 5322 and the inner sheath tube 5321 of the delivery device 5320 to move relative to each other, such as pulling the inner sheath tube 5321 proximally and/or pushing the outer sheath tube 5322 distally to make the outer sheath tube 5322 and the inner sheath tube 5321 move relative to each other.

In this embodiment, the support body adopts a support body 5310 with a variable diameter structure, that is, when the support body 5310 expands, it forms a large - diameter end and a small - diameter end. The maximum radial size of the large - diameter end is larger than that of the small - diameter end, the large - diameter end is arranged near the proximal end, and the small - diameter end is arranged near the distal end. The axial length of the large - diameter end is larger than that of the small - diameter end.

The support body 5310 forms an accommodating part 5330 at the connection between the large - diameter end and the small - diameter end. The accommodating part 5330 is arranged adjacent to the axis of the support body 5310 relative to the first conductive part 5331 and/or the second conductive part 5332. In this embodiment, the accommodating part 5330 is arranged adjacent to the axis of the support body 5310 relative to the first conductive part 5331 and the second conductive part 5332, that is, a concave area is formed on the support body 5310, and an accommodating space for accommodating the tissue to be ablated is formed on the outer side of the outer wall of the accommodating part 5330, so that the characteristic electric field line E can pass through the tissue in the accommodating space. Preferably, at least one characteristic electric field line E passing through the accommodating space is inclined relative to the axis of the support body 5310, so that the accommodating part 5330 of the support body 5310 can accommodate more tissues, and the difficulty of the characteristic electric field line E passing through the tissues in the accommodating space can be reduced. In this embodiment, the first conductive part 5331 is arranged on the distal side of the large - diameter end, and the second conductive part 5332 is arranged on the proximal side of the small - diameter end. The radial size of the first conductive part 5331 is larger than that of the second conductive part 5332, that is, the radial size of the annular structure formed by the conductive part on the proximal side is larger than that of the annular structure formed by the conductive part on the distal side, so that at least one characteristic electric field line E passing through the accommodating space is inclined relative to the axis of the support body 5310, facilitating the characteristic electric field line to pass through the tissues in the accommodating space.

It can be understood that the support body 5310 can also adopt other variable diameter structures, such as the maximum radial sizes of the proximal end and the distal end are equal, an accommodating part is formed between the proximal end and the distal end, and the radial size of the first conductive part 5331 is different from that of the second conductive part 5332, so that at least one characteristic electric field line E passing through the accommodating space is also inclined relative to the axis of the support body 5310, facilitating the characteristic electric field line to pass through the tissues in the accommodating space.

As shown in Figure 57, the accommodating part 5330 is arranged adjacent to the axis of the support body 5310 relative to the first conductive part 5331 and the second conductive part 5332. The characteristic electric field line E passes through the accommodating space, and the electric field lines passing through the accommodating space are relatively dense, with greater electric field intensity, so that the tissues in the accommodating space are more likely to be ablated; The first conductive part 5331 and the second conductive part 5332 are arranged on opposite sides of the accommodating space, the radial size of the first conductive part 5331 is larger than that of the second conductive part 5332, and at least one characteristic electric field line E passing through the accommodating space is inclined relative to the axis of the support body 5310.

Specifically, the support body 5310 includes a plurality of support rods 5311 arranged at intervals along the circumferential direction of the axis of the support body 5310. The number of support rods 5311 can be 4, 6, 8 or more. The distal ends of the support rods 5311 are connected together, and the proximal ends are connected together. Each support rod 5311 includes a first arc segment 5312, a second arc segment 5313 and a third arc segment 5314 which are connected in sequence. When the support body 5310 expands, both the first arc segment 5312 and the third arc segment 5314 bend in the direction away from the central axis of the framework, and the second arc segment 5313 bends in the direction close to the central axis of the framework, so that the support body 5310 forms an accommodating part 5330 at the second arc segment 5313; The radial size of the first arc segment 5312 is larger than that of the third arc segment 5314, and the axial size of the first arc segment 5312 is larger than that of the third arc segment 5314. **In** other embodiments, the axial size of the first arc segment 5312 is not larger than that of the third arc segment 5314.

The first conductive part 5331 is arranged at the connection between the first arc segment 5312 and the second arc segment 5313, and the second conductive part 5332 is arranged at the connection between the second arc segment 5313 and the third arc segment 5314. That is, the first conductive part 5331 is located on the proximal side of the accommodating part 5330, and the second conductive part 5332 is located on the distal side of the accommodating part 5330. When the support body 5310 expands, the radial size of the first conductive part 5331 is larger than that of the second conductive part 5332. The delivery device 5320 delivers the support body 5310 to the target area, the accommodating part 5330 is attached to the pulmonary vein or the orifice of the left atrial appendage, and the characteristic electric field line E can pass through the tissue.

As shown in Figure 57, each support rod 5311 is sleeved with two ring electrodes at different positions in the axial direction, so that two circles of spaced electrodes are formed around the axial direction on the support body 5310. The first conductive part 5313 includes a plurality of electrodes spaced around the axial direction, and the second conductive part includes another circle of spaced electrodes around the axial direction.

In this embodiment, the conductive parts on both sides of the accommodating part 5330 are arranged at intervals in the axial direction. The extending direction of the accommodating part 5330 can also be between the circumferential direction and the axial direction, or be irregular. The conductive parts on both sides are arranged on opposite sides of the accommodating part 5330, so that the characteristic electric field line E passes through the area outside the support body 5310, and thus easily passes through the tissue during ablation to form transmural ablation.

In other embodiments, the support body 5310 can also be replaced with a balloon, such as a balloon made by cutting or braiding processes, which can contract and expand radially. The balloon is filled with a medium, and the radial size of the balloon is adjusted by controlling the volume of the internal medium.

### Embodiment 54

Figure 58 is a schematic structural diagram of the ablation system 5400 according to the fifty - fourth embodiment of the present application.

In this embodiment, both the first conductive part 5431 and the second conductive part 5432 are arranged on the support body 5410, that is, the first conductive part 5431 and the second conductive part 5432 are arranged on the support body 5410 at intervals in the axial direction. The support body 5410 has a structure with a large proximal end and a small distal end, that is, the radial size of the proximal end of the support body 5410 is larger than that of the distal end. The framework is provided with an accommodating part at the position between the first conductive part 5431 and the second conductive part 5432. It can be understood that for support bodies with different shapes and structures, the formed accommodating parts and the corresponding accommodating spaces on the outer side are different. In this embodiment, the accommodating part is formed between the sealing part 5411 and the anchoring part 5412.

Specifically, the first conductive part 5431 is arranged on the proximal side of the support body 5410, specifically on the sealing part 5411, and the second conductive part 5432 is located on the distal side of the first conductive part 5431, specifically on the anchoring part 5412. The radial sizes at their positions of the two conductive parts on the support body 5410 are different, and the radial size of the first conductive part 5431 is larger than that of the second conductive part 5432. When the support body 5410 is delivered to the opening of the left atrial appendage, the characteristic electric field line E between the first conductive part 5431 and the second conductive part 5432 easily passes through the area outside the accommodating part in the support body 5410, and the support body 5410 between the two conductive parts is used for attaching to the tissue, so that the characteristic electric field line can easily pass through the tissue during ablation.

The support body 5410 includes a sealing part 5411 and an anchoring part 5412 which are connected, and anchor thorns 5413 are arranged on the anchoring part 5412. When the anchor thorns 5413 are removed, it can be used as an ablation catheter to ablate areas such as pulmonary veins and left atrial appendages. It can be understood that some components in the first conductive part 5431 can only be used for mapping and have no ablation effect. In some embodiments, some components in the first conductive part 5431 are used for mapping, and some components have ablation effects. In some embodiments, at least part of the components of the first conductive part 5431 are used for mapping and ablation at different times. It can be understood that some components in the second conductive part 5432 can only be used for mapping and have no ablation effect. In some embodiments, some components of the second conductive part 5432 are used for mapping, and some components have ablation effects. In some embodiments, at least part of the components of the second conductive part 5432 are used for mapping and ablation at different times.

Specifically, the support body 5410 includes a framework 5414. A barrier (such as a membrane) for blocking thrombus can be arranged on the surface of the framework 5414, and the number and arrangement position of the barriers are not limited. For the sake of clarity, the barrier is not shown in Figure 58. The framework 5414 includes a first framework 5415, a second framework 5416 and an insulating part 5417. Both the first framework 5415 and the second framework 5416 are made of conductive materials, and the first framework 5415 and the second framework 5416 are connected through the insulating part 5417. The first framework 5415 and the second framework 5416 are respectively used for transmitting ablation energy with different polarities to the target tissue area.

Both the first framework 5415 and the second framework 5416 can be made of metal materials or polymer materials with good biocompatibility. The metal materials can be nickel - titanium alloy, cobalt - chromium alloy, stainless steel and degradable metal materials, preferably superelastic shape memory alloy nickel - titanium wires. The insulating part 5417 is made of an insulating material with good biocompatibility. In some embodiments, both the first framework 5415 and the second framework 5416 are made by braiding. In some embodiments, one of the first framework 5415 and the second framework 5416 is made by braiding, and the other is formed by cutting.

The manufacturing process of the framework 5414 is as follows: the first framework 5415 and the second framework 5416 are respectively made by cutting conductive pipes, and the insulating part 5417 is connected between the first framework 5415 and the second framework 5416 by one or more of fusion connection, plug connection and clamping connection.

As shown in Figure 58, the first framework 5415 and the second framework 5416 are arranged at intervals in the axial direction, and the insulating part 5417 forms an annular insulating area around the axis of the support body 5410. The ablation part 5430 includes a first conductive part 5431 arranged on the first framework 5415 and a second conductive part 5432 arranged on the second framework 5416. In this embodiment, the first conductive part 5431 is at least part of the first framework 5415, and the second conductive part 5432 is at least part of the second framework 5416. The surface of the area outside the first conductive part 5431 on the first framework 5415 is insulated, and the surface of the area outside the second conductive part 5432 on the second framework 5416 is insulated, that is, both the first framework 5415 and the second framework 5416 ablate the target tissue area through the surface of part of the support rods.

In this embodiment, both the first framework 5415 and the second framework 5416 are made by cutting, forming a plurality of mesh holes, and the insulating part 5417 connected between the first framework 5415 and the second framework 5416 is in a straight rod shape. In a modified embodiment, each support rod in the insulating part 5417 is arc - shaped, fold - line shaped, annular, or the size of the insulating part 5417 in the axial direction is larger than that in Figure 58. The plurality of support rods in the insulating part 5417 are connected to each other to form mesh holes, that is, the specific form of the insulating part 5417 is not limited. In a modified embodiment, at least one of the first framework 5415 and the second framework 5416 can be obtained by braiding.

In this embodiment, both the first conductive part 5431 and the second conductive part 5432 are part of the framework 5414. In a modified embodiment, at least one of the first conductive part 5431 and the second conductive part 5432 is an ablation electrode additionally arranged on the framework 5414 (the first framework 5415 or the second framework 5416). The ablation electrode can maintain electrical connection with the corresponding framework, or be insulated from the framework and connected to an external ablation signal source through a wire.

As shown in Figure 58,In this embodiment, the first framework 5415 and the second framework 5416 are arranged at intervals in the axial direction. In a modified embodiment, the first framework 5415 and the second framework 5416 are arranged at intervals in the circumferential direction, for example, the first framework 5415 is arranged within the first angular range in the circumferential direction of the support body 5410, the second framework 5416 is arranged within the second angular range in the circumferential direction of the support body 5410, and the first framework 5415 and the second framework 5416 are connected through the insulating part 5417.

In a modified embodiment, the first framework 5415 and the second framework 5416 are arranged at intervals in both the circumferential direction and the axial direction.

Since the sealing part 5411 is used to cover the opening of the left atrial appendage, it is easier to cover a circle in the circumferential direction at the opening of the left atrial appendage. Therefore, arranging the first conductive part 5431 on the sealing part 5411 is beneficial to forming a complete annular ablation area at the opening of the left atrial appendage and improving the ablation effect. Preferably, the first conductive part 5431 is arranged in the circumferential edge area of the sealing part 5411. The first conductive part 5431 can be arranged at least one of the proximal edge, the middle section edge and the distal edge of the sealing part 5411, and the first conductive part 5431 is preferably arranged in the area of the sealing part 5411 that can be attached to a circle at the opening of the left atrial appendage. At each position on the sealing part 5411, the distance from the axis of the sealing part 5411 is constant. In some embodiments, the circumferential edge area refers to the area on the sealing part 5411 where the distance from the axis of the sealing part 5411 is 20% of the maximum distance. In some embodiments, the circumferential edge area refers to the area on the sealing part 5411 where the distance from the axis of the sealing part 5411 is 10% of the maximum distance. In some embodiments, the circumferential edge area refers to the area on the sealing part 5411 where the distance from the axis of the sealing part 5411 is 5% of the maximum distance. In some embodiments, the circumferential edge area refers to the area on the sealing part 5411 where the distance from the axis of the sealing part 5411 is 3% of the maximum distance. It can be understood that the specific identification of the edge area can be set according to actual needs, which is not limited here.

In a modified embodiment, at least one of the first conductive part 5431 and the second conductive part 5432 is an ablation electrode. In the first conductive part 5431 and the second conductive part 5432, more ablation electrodes can be arranged, and different ablation electrodes are arranged at intervals, and the respective arrangement positions of different ablation electrodes are not limited.

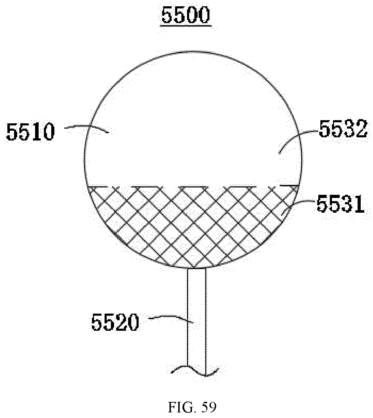

Please refer to Figure 59. Figure 59 is a schematic structural diagram of the ablation system 5500 provided in the fifty - fifth embodiment of the present application. The ablation system 5500 is specifically an ablation catheter, which can be used for ablating intracardiac tissues or tissues to be ablated outside the heart. The ablation system 5500 includes a support body 5510 and a delivery device 5520. The support body 5510 is connected to the distal end of the delivery device 5520. Only the catheter part of the delivery device 5520 connected to the support body 5510 is shown in Figure 59.

The difference between the ablation system 5500 provided in this embodiment and the ablation system provided in Figure 58 is the structures of the support body 5510, the first conductive part 5531 and the second conductive part 5532.

Specifically, the support body 5510 provided in this embodiment is roughly spherical, and the support body 5510 can be spherical, ellipsoidal or nearly spherical. The support body 5510 includes a proximal part and a distal part, the proximal part of which is used for connecting the delivery device 5520, and the distal part of which is arranged away from the delivery device 5520 relative to the proximal part. The second conductive part 5532 is arranged on the surface of the distal part of the support body 5510, and the first conductive part 5531 is arranged on the surface of the proximal part of the support body 5510.

At least one of the first conductive part 5531 and the second conductive part 5532 can be realized by any one or more of the electrode parts provided in other embodiments of this application. It can be understood that at least one of the first conductive part 5531 and the second conductive part 5532 can also be the conductive part of the framework of the support body.

In some embodiments, the proximal part of the support body 5510 forms a grid structure by braiding or cutting, at least part of which is made of conductive metal materials, and at least part of the conductive grid is used as the first conductive part 5531.

In some embodiments, the second conductive part 5532 arranged on the distal part of the support body 5510 includes one or more electrode parts, and the electrode parts can be in the form of dot electrodes, rod electrodes, arc electrodes, wave electrodes, etc.

The first conductive part 5531 and the second conductive part 5532 are used to form an ablation loop for transmitting pulse signals with opposite polarities, and they are insulated from each other. The structure of the distal part of the support body 5510 is the same as that of the proximal part, both of which are the same grid structure. Alternatively, the proximal part of the support body 5510 is provided with the grid structure shown in Figure 59, and the distal part of the support body 5510 is provided with other forms of support structures, such as other grid structures with different densities and different grid shapes, or in a basket shape.

In some embodiments, the second conductive part 5532 and the support body 5510 are insulated from each other, and for the specific insulation mode, please refer to the foregoing embodiment; In some embodiments, the proximal part and the distal part of the support body 5510 are connected through an insulating part.

In the process of ablation using the ablation system 5500 provided in this embodiment, the distal part of the support body 5510 is used to appose the target tissue wall, and the second conductive part 5532 needs to appose the target tissue wall for ablation. The wall - apposition condition of the second conductive part 5532 is better than that of the first conductive part 5531, and the expected ablation depth of the second conductive part 5532 is larger than that of the first conductive part 5531. Therefore, the discharge area of the second conductive part 5532 is set to be smaller than that of the first conductive part 5531, so that the average current density of the second conductive part 5532 is larger, thereby expanding the ablation range and ablation depth of the second conductive part 5532.

The first conductive part 5531 and the second conductive part 5532 in this embodiment can adopt the specific parameters in the other foregoing embodiments, which will not be repeated here.

It should be noted that the specific technical solutions in the above embodiments can be applied to each other without violating the technical principle of the present invention. For example, on the basis of the above embodiments, changing the specific forms of the first conductive part and the second conductive part among using framework conduction, or additionally setting dot electrodes, rod electrodes, arc electrodes, ring electrodes, wave electrodes, etc., or changing the specific setting position of the second conductive part to the sealing part, anchoring part, connecting piece, delivery device, or the position outside the support body and the delivery device, or setting more of the above - mentioned first conductive parts and second conductive parts on the support body, or combining the sealing parts, anchoring parts, and ablation catheters in the above embodiments to obtain a new ablation system, all belong to the content of the present application. The first conductive part and the second conductive part in each of the above embodiments can adopt the electric field intensity, voltage, average current density, distance, area and other parameters provided in the embodiments of the present application, and can adopt the technical solution of partial overlapping of the first ablation area and the second ablation area. The support body in each of the above embodiments can be provided with an accommodating part arranged between two conductive parts for accommodating tissues, at least one characteristic electric field line passes through the accommodating space outside the accommodating part, and the distal end of at least one characteristic electric field line passing through the accommodating space is inclined towards the axis of the support body. Inclining towards the axis of the support body means that the at least one characteristic electric field line passing through the accommodating space is neither parallel nor perpendicular to the axis of the support body. As shown in the drawings corresponding to the above embodiments, the projection of the at least one characteristic electric field line passing through the accommodating space on the characteristic plane can form two angles with the axis of the support body, and the sum of the two angles is 180 degrees, wherein the smaller angle is an acute angle.

The above descriptions are only preferred embodiments of the present application and are not intended to limit the present application. Any modification, equivalent replacement and improvement made within the spirit and principle of the present application shall be included in the protection scope of the present application.

## Claims

1. An ablation system, **characterized in that** it comprises a support body and an ablation part, at least part of the ablation part is arranged on the support body, the ablation part comprises a first conductive part and a second conductive part, the first conductive part and the second conductive part are used to form a loop to transmit pulse ablation energy, their polarities are opposite, and the average current densities of the first conductive part and the second conductive part are not equal.

2. The ablation system according to claim 1, **characterized in that** in the natural state, the first conductive part and the second conductive part are arranged at intervals in the axial direction, the first conductive part is arranged on the proximal side of the second conductive part, and during ablation, the average current density on the surface of the second conductive part is greater than that on the surface of the first conductive part.

3. The ablation system according to claim 2, **characterized in that** both the first conductive part and the second conductive part are used to ablate the target tissue, and the wall - apposition condition of the first conductive part is better than that of the second conductive part.

4. The ablation system according to claim 2, **characterized in that** the discharge area of the second conductive part is smaller than that of the first conductive part.

5. The ablation system according to claim 4, **characterized in that** the discharge area of the first conductive part is 15mm² ~ 4700mm², and the discharge area of the second conductive part is 15mm² ~ 500mm².

6. The ablation system according to claim 5, **characterized in that** the support body comprises a metal framework, the first conductive part and the second conductive part are arranged on the support body, the first conductive part is at least part of the metal framework, and the second conductive part is at least one electrode part arranged on the support body.

7. The ablation system according to claim 6, **characterized in that** the electrode part is a rod electrode, a dot electrode, an arc electrode, or a strip - shaped or filament - shaped electrode extending around the outer periphery of the support body for at least one circle.

8. The ablation system according to claim 4, **characterized in that** in the natural state, the proximal end of the second conductive part is arranged on the distal side of the distal end of the first conductive part.

9. The ablation system according to any one of claims 1 - 8, **characterized in that** the ablation area of the first conductive part is the area around the first conductive part where the electric field intensity is greater than the threshold intensity of the target tissue; The ablation area of the second conductive part is the area around the second conductive part where the electric field intensity is greater than the threshold intensity of the target tissue; The ablation area of the first conductive part and the ablation area of the second conductive part partially overlap.

10. The ablation system according to claim 9, **characterized in that** the distance between the first conductive part and the second conductive part is 3mm ~ 22mm.

11. The ablation system according to claim 10, **characterized in that** the distance between the nearest two points on the surfaces of the first conductive part and the second conductive part ranges from 3mm to 18mm.

12. The ablation system according to claim 8, **characterized in that** the straight line connecting any point on the first conductive part and any point on the second conductive part is a characteristic electric field line, and at least one characteristic electric field line passes through the area outside the outer wall of the support body.

13. The ablation system according to claim 12, **characterized in that** the first conductive part and the second conductive part are arranged on the support body, the support body comprises an accommodating part, in the radial direction, the accommodating part is arranged adjacent to the axis of the support body relative to the first conductive part and/or the second conductive part, an accommodating space for accommodating the tissue to be ablated is formed on the outer side of the circumferential outer wall of the accommodating part, at least part of the outer wall of the accommodating part is used for apposing the tissue to be ablated, the accommodating part extends along the circumferential direction of the support body, the first conductive part and the second conductive part are arranged on opposite sides of the accommodating space, and during ablation, at least one characteristic electric field line passes through the accommodating space.

14. The ablation system according to claim 13, **characterized in that** the radial size of the first conductive part is larger than that of the second conductive part.

15. The ablation system according to claim 14, **characterized in that** in the natural state, the projection of the shortest one of the characteristic electric field lines between the selected first characteristic point and at least one second characteristic point on the characteristic plane forms an angle of 0° - 70° with the axis of the support body,
the first characteristic point is the point with the largest radial size on the periphery of the first conductive part;
the second characteristic point is the point with the largest radial size on the periphery of the second conductive part;
the characteristic plane is the plane passing through the axis of the support body and the first characteristic point.

16. The ablation system according to any one of claims 1 - 5, 8, 12 - 15, **characterized in that** the ablation system is used for occluding and ablating the left atrial appendage, the first conductive part is arranged in the circumferential edge area of the proximal part of the support body, and the second conductive part is arranged in the circumferential edge area of the distal part of the support body.

17. The ablation system according to any one of claims 1 - 5, 8, 12 - 15, **characterized in that** the ablation system is used for occluding and ablating the left atrial appendage, the support body comprises a sealing part and an anchoring part which are connected, the sealing part is arranged on the proximal side of the anchoring part, the first conductive part is arranged in the circumferential edge area of the sealing part, the second conductive part is arranged in the circumferential edge area of the anchoring part, the first conductive part is used for ablating the tissue at the orifice of the left atrial appendage, the second conductive part is used for ablating the tissue on the inner side of the orifice of the left atrial appendage, and the sealing part and the anchoring part are connected in an insulated manner through a connecting piece.

18. The ablation system according to claim 17, **characterized in that** the anchoring part comprises a second framework and a membrane arranged on the outer surface of the second framework, an insulating coating is plated on the surface of the second framework, and the second conductive part is arranged on the surface of the membrane facing away from the second framework.

19. The ablation system according to any one of claims 1 - 5, 8, 12 - 15, **characterized in that** the ablation system comprises a delivery device for delivering the support body, the distal end of the delivery device is connected with the support body, and the first conductive part is arranged on the support body;
the second conductive part is arranged on the delivery device, and the second conductive part is movable relative to the support body and is used for being released on the proximal side or the distal side of the support body; or

20. The ablation system according to any one of claims 1 - 19, **characterized in that** at least part of the first conductive part and/or the second conductive part can be used for collecting tissue physiological signals.
the second conductive part is independently arranged relative to the support body and the delivery device.
